(19) Europäisches Patentamt
European Patent Office
Office européen des brevets

(11) EP 4 083 009 A1

(12) **EUROPEAN PATENT APPLICATION**
published in accordance with Art. 153(4) EPC

(43) Date of publication:
**02.11.2022 Bulletin 2022/44**

(21) Application number: **20905860.1**

(22) Date of filing: **23.12.2020**

(51) International Patent Classification (IPC):
***C07C 31/20*** *(2006.01)* ***A61K 8/34*** *(2006.01)*
***A61Q 19/00*** *(2006.01)* ***C07C 29/80*** *(2006.01)*

(52) Cooperative Patent Classification (CPC):
**A61K 8/34; A61Q 19/00; C07C 29/80; C07C 31/20; C07C 37/74**

(86) International application number:
**PCT/JP2020/048232**

(87) International publication number:
**WO 2021/132369 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**28.12.2019 JP 2019239974**
**28.12.2019 JP 2019239975**
**28.12.2019 JP 2019239976**
**28.12.2019 JP 2019239977**
**28.12.2019 JP 2019239978**
**28.12.2019 JP 2019239979**
**20.01.2020 JP 2020006660**
**06.02.2020 JP 2020018910**

(71) Applicant: **Daicel Corporation**
**Osaka 530-0011 (JP)**

(72) Inventors:
- **SHIMIZU, Masahiko**
**Tokyo 108-8230 (JP)**
- **KAJIKAWA, Yasuteru**
**Tokyo 108-8230 (JP)**

(74) Representative: **Grünecker Patent- und Rechtsanwälte**
**PartG mbB**
**Leopoldstraße 4**
**80802 München (DE)**

# (54) 1,3-BUTYLENE GLYCOL PRODUCT

(57) A high-purity 1,3-butylene glycol product that is colorless and odorless (or almost colorless and odorless), unlikely to cause coloration and odor over time, and/or unlikely to cause an acid concentration increase over time when the product is left in a state containing water is provided. A 1,3-butylene glycol product containing 1,3-butylene glycol, wherein, after the 1,3-butylene glycol product has been kept at 180°C for 3 hours in air atmosphere, at least one of contents of compounds represented by the following Formula (A) or (B) is less than 8 ppm. In the following formula, $R^1$ to $R^4$ are the same as or different from each other, and each of $R^1$ to $R^4$ is a hydrogen atom, an alkyl group which has from 1 to 4 carbon atoms and may be substituted with a hydroxy group, or an alkenyl group which has from 2 to 4 carbon atoms and may be substituted with a hydroxy group.

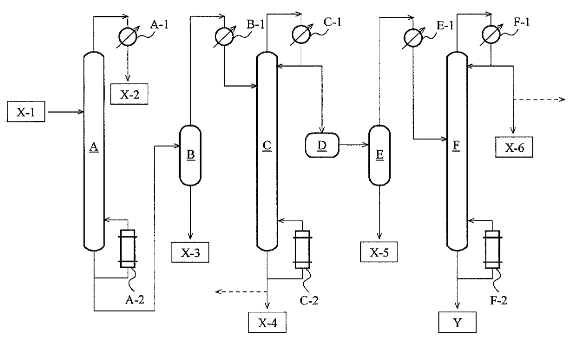


FIG. 1

EP 4 083 009 A1

## Description

### Technical Field

The present disclosure relates to a 1,3-butylene glycol product. The present application claims priority from the Japanese Patent Application No. 2019-239974, Japanese Patent Application No. 2019-239975, Japanese Patent Application No. 2019-239976, Japanese Patent Application No. 2019-239977, Japanese Patent Application No. 2019-239978, and Japanese Patent Application No. 2019-239979, all filed in Japan on December 28, 2019, the Japanese Patent Application No. 2020-006660 filed in Japan on January 20, 2020, and the Japanese Patent Application No. 2020-018910 filed in Japan on February 6, 2020, the entire contents of which are incorporated herein by reference.

### Background Art

1,3-Butylene glycol is a colorless, transparent, and odorless liquid and has properties, such as low volatility, low toxicity, and high hygroscopicity, and has excellent chemical stability. 1,3-butylene glycol has a wide range of applications, including raw materials for various synthetic resins and surfactants, as well as materials for cosmetics, hygroscopic agents, high boiling point solvents, and antifreezes, etc. Particularly in recent years, 1,3-butylene glycol has been attracting attention for having excellent properties as a moisturizer, and demand is growing in the cosmetic industry.

1,3-Butylene glycol produced by a known production method has had a problem of acid concentration (acidity) increase when the 1,3-butylene glycol is left for a long period of time in a state containing water. The cause of the acid concentration increase has been uncertain but thought to be related to by-products contained in crude 1,3-butylene glycol. Cosmetics typically contain water and require a long period of time from production to actual use by general consumers. In addition, from the viewpoint, such as storage stability of cosmetics, liquidity is strictly controlled. When 1,3-butylene glycol produced by a known method is used in cosmetics, an acid concentration increase can disrupt the liquidity balance of the cosmetics, and this can lead to a loss of the intended effect. In addition, the acid concentration increase of cosmetics can cause rough skin or the like of the users. Furthermore, there was a case where cosmetics containing no water also have absorbed water during use or storage, and this has also increased the acid concentration accordingly. Thus, removing by-products from crude 1,3-butylene glycol to yield high-purity 1,3-butylene glycol has been required.

Also, 1,3-butylene glycol produced by a known production method have sometimes had an odor due to effects from by-products. In addition, 1,3-butylene glycol transparent immediately after production may be colored over time, which is a problem in storing for a long period of time. For example, during use and/or storage after use of a cosmetic, the cosmetic is exposed to air. In addition, in production of cosmetics, the production is usually performed in air atmosphere, and the product may be heated for the purposes of, for example, sterilization. When 1,3-butylene glycol produced by a known method is used in cosmetics, sometimes coloration occurs due to the presence of air or effects of heating. To solve such problems, removing by-products from crude 1,3-butylene glycol to yield high-purity 1,3-butylene glycol has been required.

For a method to produce high-purity 1,3-butylene glycol, a method including adding sodium hydroxide to crude 1,3-butylene glycol produced by hydrogen reduction of acetaldols and performing distillation has been proposed. In addition, other methods have been proposed, including a method in which an alkali metal base is added to crude 1,3-butylene glycol from which a high boiling point substance has been removed, the mixture is heat-treated, then 1,3-butylene glycol is distilled, and the alkali metal compound and a high boiling point substance are separated as residues, and subsequently a low boiling point substance is distilled off from the 1,3-butylene glycol distillate (Patent Documents 1 to 6). Various methods for purifying 1,3-butylene glycol have been thus proposed to produce high-purity 1,3-butylene glycol.

### Citation List

#### Patent Document

Patent Document 1: JP 07-258129 A

Patent Document 2: WO 00/07969

Patent Document 3: JP 2001-213822 A

Patent Document 4: JP 2001-213824 A

Patent Document 5: JP 2001-213825 A

Patent Document 6: JP 2001-213828 A

Summary of Invention

Technical Problem

However, 1,3-butylene glycol products produced from these purification methods still contain by-products and have a problem of having an odor, a problem of increasing acid concentration over time when containing water, and a problem that coloration occurs over time.

1,3-Butylene glycol is produced, for example, by a method of (1) reduction (hydrogenation) of acetaldols, (2) hydrolysis of 1,3-butylene oxide, (3) selective hydrocracking of erythritol, (4) selective hydration of butadiene, (5) hydrogen addition to n-butanol-3-one, (6) hydrogen addition to 1-butanol-3-one, (7) hydrogen addition to 3-hydroxy-1-butanoic acid, (8) hydrogen addition to β-butyrolactone, or (9) hydrogen addition to diketene.

Of the production methods, the method of (1) reduction (hydrogenation) of acetaldols to yield 1,3 butylene glycol is preferable. Among them, the method of reduction of acetaldols in a liquid phase is preferable in terms of yield. The reasons for this are that acetaldols have a high boiling point, that acetaldols are unstable to heat and readily undergo a dehydration reaction at high temperatures to form crotonaldehyde or the like, and furthermore, that a reaction rate of the dehydration reaction is greater than that of the reduction reaction (hydrogenation reaction) at high temperatures. That is, when acetaldols are subjected to vapor-phase reduction, the temperature inside the reaction system needs to be increased to high temperature, but subjecting the acetaldols to high temperature causes a dehydration reaction to produce crotonaldehyde or the like, and the subsequent reduction reaction produces by-products, such as butanol. Thus, this results in relatively reduced yield of the target 1,3-butylene glycol. Thus, to produce a high-purity 1,3-butylene glycol product, a liquid phase reduction rather than a gas phase reduction is preferably performed.

In production of 1,3-butylene glycol, by-products are generally produced in the production process. For example, in production of 1,3-butylene glycol by hydrogen reduction of an acetaldol, a low boiling point substance (low boiling point compound) having an unsaturated bond, such as acetaldehyde, butylaldehyde, crotonaldehyde, acetone, and methyl vinyl ketone; a condensate of these (e.g., a trimer of acetaldehyde); a hydride of the above condensate; a condensate of 1,3-butylene glycol and the above low boiling point substance (e.g., an acetal of 1,3-butylene glycol and acetaldol); or the like is produced as a by-product. In addition, an acetal of crotonaldehyde and 1,3-butylene glycol; an acetal of acetaldehyde and 1,3-butylene glycol; an acetal of a hydride of a trimer of acetaldol, acetaldehyde, and acetaldehyde; or the like is produced as a by-product. Furthermore, in addition to the above, acetic acid may be present as an impurity in an acetaldol used as a raw material or is used to neutralize sodium hydroxide used in producing an acetaldol, and a condensate of the acetic acid and 1,3-butylene glycol (ester of acetic acid and 1,3-butylene glycol) is formed as a by-product. Moreover, these by-products may have properties as a substance that causes coloration, a substance that causes odor or a substance that causes acidity.

Whether the acetal is a substance that causes coloration, a substance that causes odor or a substance that causes acidity is uncertain, and the acetal is also considered to have all the properties but is considered to have strong properties as a substance that causes odor. Specifically, the acetal itself is unlikely to be a substance that causes odor but can form a substance that causes odor by a change over time or heating. Furthermore, the acetal may be hydrolyzed to form acetaldol, which is a substance that causes odor and has an oxidation (coloration) promoting effect. Thus, the acetal can also be said to be a substance that causes coloration. Here, the substance that causes coloration is defined as including not only a substance actually having a hue itself but also a substance changing over time into a substance having a hue. The substance that causes odor is defined as including not only a substance actually emitting an odor itself but also a substance changing over time into a substance emitting an odor. The substance that causes acidity is defined as a substance increasing acid concentration over time when containing water.

Whether the ester is a substance that causes coloration, a substance that causes odor or a substance that causes acidity is uncertain, and the ester is also considered to have all the properties but is considered to have strong properties as both a substance that causes odor and a substance that causes acidity. This is because when the ester is hydrolyzed by water, acetic acid is produced.

In production of 1,3-butylene glycol, it is considered that by-products in the production process may include, in addition to the acetal and ester, a wide variety of by-products corresponding to a substance that causes coloration, a substance that causes odor, or a substance that causes acidity. For example, the hydride of a trimer of acetaldehyde described above is considered to possibly correspond to any of a substance that causes coloration, a substance that causes odor or a substance that causes acidity.

The by-products described above are difficult to completely remove even using a purification means, such as distillation known in the art. This is considered to be because crude 1,3-butylene glycol is subjected to high temperature conditions and alkali treatment in the purification stage of the crude 1,3-butylene glycol, and new by-products are produced accordingly. For these reasons, as described above, the 1,3-butylene glycol products of Patent Documents 1 to 6 contain a wide variety of by-products, thus have an odor and are colored over time, and furthermore, increase acid concentration over time in a state containing water.

Thus, an object of the present disclosure is to provide a high-purity 1,3-butylene glycol product that is colorless and odorless (or almost colorless and odorless), unlikely to cause or increase coloration and odor over time, and/or unlikely to cause an acid concentration increase over time even in a state containing water.

Furthermore, another object of the present disclosure is to provide a moisturizer and a cosmetic product that have excellent moisturizing performance and can retain high quality for a long period of time.

Solution to Problem

As a result of diligent research to achieve the purpose described above, the present inventors have found that storage of the 1,3-butylene glycol product for a long period of time causes deteriorations in qualities such as increase in coloration, increase in odor, decrease in initial boiling point over time, rise in dry point, and decrease in potassium permanganate test value over time, and that a content of a specific chemical substance increases over time in the 1,3-butylene glycol product correspondingly. Therefore, as a result of subjecting the 1,3-butylene glycol product to a heating test (accelerated test) under specific conditions, and reviewing correlation between the content of the specific chemical substance in a sample after the test and the deteriorations in qualities (coloration, odor, initial boiling point, dry point, potassium permanganate test value, acid concentration, etc.) of the product over time, the present inventors have found a clear correlation therebetween. Having studied a pathway of the specific chemical substance formation at the same time, the present inventors have found a method of suppressing a specific chemical substance and its precursor from being mixed into the 1,3-butylene glycol product. More specifically, the present inventors have found that, by adjusting distillation conditions (content of specific impurities in a charged liquid, reflux ratio, etc.) of a product column, a high boiling point component removal column, a dehydration column, etc., and further, by adjusting reaction conditions (particularly, increasing a partial pressure of hydrogen in a reactor) in a reaction step (e.g., hydrogenation of an acetaldol), it is possible to suppress the specific chemical substance and its precursor from being mixed into the 1,3-butylene glycol product, and to produce 1,3-butylene glycol that is characteristically unlikely to cause or increase coloration and odor over time, and unlikely to cause an acid concentration increase over time even in a state containing water. The invention according to the present disclosure has been completed by further studying based on these findings.

That is, the present disclosure provides a 1,3-butylene glycol product containing 1,3-butylene glycol, in which, after the 1,3-butylene glycol product has been kept at 180°C for 3 hours in air atmosphere, at least one of contents of compounds represented by the following Formula (A) or (B) is less than 8 ppm:

[Chemical Formula 1]

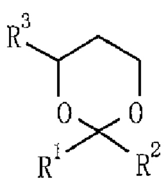

(A)

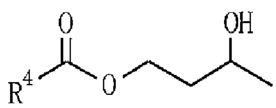

(B)

where $R^1$ to $R^4$ are the same as or different from each other, and each of $R^1$ to $R^4$ is a hydrogen atom, an alkyl group with from 1 to 4 carbon atoms which may be substituted with a hydroxy group, or an alkenyl group with 2 to 4 carbon atoms which may be substituted with a hydroxy group.

In the 1,3-butylene glycol product, after the 1,3-butylene glycol product has been kept at 180°C for 3 hours in air atmosphere, a sum of the contents of the compounds represented by Formula (A) is preferably less than 66 ppm.

Also, after the 1,3-butylene glycol product has been kept at 180°C for 3 hours in air atmosphere, a sum of the contents of the compounds represented by Formula (B) is preferably less than 31 ppm.

The compound represented by Formula (A) is, for example, any of compounds represented by the following Formulas (1) to (7), and the compound represented by Formula (B) is any of compounds represented by the following Formulas (8) to (10).

[Chem. 2]

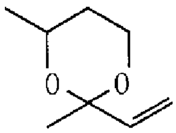

(1)

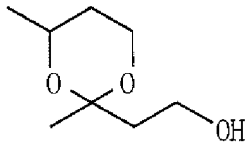

(2)

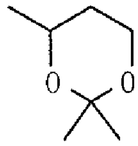

(3)

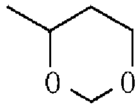

(4)

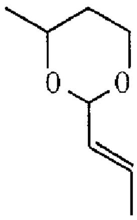

(5)

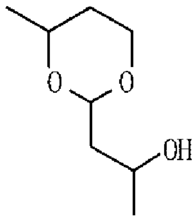

(6)

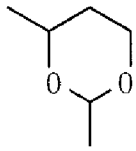

(7)

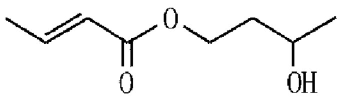

(8)

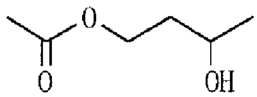

(9)

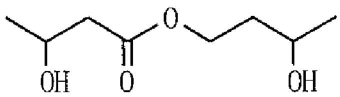

(10)

Also, in this case, after the 1,3-butylene glycol product has been kept at 180°C for 3 hours in air atmosphere, a total content of the compounds represented by Formulas (1) to (4) is preferably less than 34 ppm.

Also, after the 1,3-butylene glycol product has been kept at 180°C for 3 hours in air atmosphere, a total content of the compounds represented by Formulas (5) to (10) is preferably less than 63 ppm.

Further, after the 1,3-butylene glycol product has been kept at 180°C for 3 hours in air atmosphere, a total content of the compounds represented by Formulas (1) to (10) is preferably less than 97 ppm.

After the 1,3-butylene glycol product has been kept at 180°C for 3 hours in air atmosphere, in the 1,3-butylene glycol product, a content of methyl vinyl ketone is preferably less than 8 ppm, and/or a content of 1-hydroxy-3-butanone is preferably less than 1 ppm, and/or a content of acetone is preferably less than 6 ppm, and/or a content of formaldehyde is preferably less than 2 ppm, and/or a content of crotonaldehyde is preferably less than 1 ppm, and/or a content of acetaldol is preferably less than 1 ppm, and/or a content of acetaldehyde is preferably less than 5 ppm, and/or a content of butylaldehyde is preferably less than 4 ppm.

After the 1,3-butylene glycol product has been kept at 180°C for 3 hours in air atmosphere, a total content of methyl vinyl ketone, 1-hydroxy-3-butanone, acetone, formaldehyde, crotonaldehyde, acetaldol and acetaldehyde is preferably less than 24 ppm.

Furthermore, after the 1,3-butylene glycol product has been kept at 180°C for 3 hours in air atmosphere, a total content of methyl vinyl ketone, acetone, and butylaldehyde is preferably less than 18 ppm.

Furthermore, after the 1,3-butylene glycol product has been kept at 180°C for 3 hours in air atmosphere, an APHA is preferably less than 79.

In addition, after the 1,3-butylene glycol product has been kept at 180°C for 3 hours in air atmosphere, an initial boiling point is higher than 203°C and/or a dry point is 209°C or lower.

Furthermore, after the 1,3-butylene glycol product has been kept at 180°C for 3 hours in air atmosphere, a potassium permanganate test value is 30 minutes or longer.

Further, after the 1,3-butylene glycol product has been kept at 180°C for 3 hours in air atmosphere, an acid concentration (in terms of acetic acid) is less than 16 ppm.

In a state where the 1,3-butylene glycol product has not undergone a test in which 1,3-butylene glycol product is kept at 180°C for 3 hours in air atmosphere, a total content of 1-hydroxy-3-butanone and acetaldol is less than 19 ppm.

In a state where the 1,3-butylene glycol product has not undergone a test in which 1,3-butylene glycol product is kept at 180°C for 3 hours in air atmosphere, a content of methyl vinyl ketone is preferably less than 10 ppm, and/or a content of 1-hydroxy-3-butanone is preferably less than 9 ppm, and/or a content of acetone is preferably less than 9 ppm, and/or a content of formaldehyde is preferably less than 3 ppm, and/or a content of crotonaldehyde is preferably less than 2 ppm, and/or a content of acetaldol is preferably less than 10 ppm, and/or a content of acetaldehyde is preferably less than 4 ppm, and/or a content of butylaldehyde is preferably less than 8 ppm.

Further, in a state where the 1,3-butylene glycol product has not undergone a test in which 1,3-butylene glycol product is kept at 180°C for 3 hours in air atmosphere, a total content of methyl vinyl ketone, 1-hydroxy-3-butanone, acetone, formaldehyde, crotonaldehyde, acetaldol and acetaldehyde is preferably less than 47 ppm.

In addition, the present disclosure also provides a moisturizer containing the 1,3-butylene glycol product.

Furthermore, the present disclosure also provides a cosmetic product containing the moisturizer.

In the present disclosure, "1,3-butylene glycol product" means a composition in which 1,3-butylene glycol occupies a majority of the components (e.g., a 1,3-butylene glycol content is 95 wt.% or greater, preferably 98 wt.% or greater).

## Advantageous Effects of Invention

The present disclosure provides a high-purity 1,3-butylene glycol product that is colorless and odorless (or almost colorless and odorless), unlikely to cause or increase coloration and odor over time, and/or unlikely to cause acid concentration increase over time even in a state containing water.

Furthermore, the present disclosure provides a moisturizer and a cosmetic product that have excellent moisturizing performance and can retain high quality for a long period of time.

## Brief Description of Drawings

FIG. 1 is a flowchart illustrating a method (purification method) for producing a 1,3-butylene glycol product of the present disclosure.
FIG. 2 is a chromatogram showing a gas chromatographic analysis for a 1,3-butylene glycol product in Example 1.
FIG. 3 is a chromatogram showing a gas chromatographic analysis for a 1,3-butylene glycol product in Example 12.
FIG. 4 is a chromatogram showing a gas chromatographic analysis for a 1,3-butylene glycol product in Comparative Example 2.

## Description of Embodiments

### 1,3-Butylene glycol product

The 1,3-butylene glycol product according to the present disclosure is a 1,3-butylene glycol product containing 1,3-butylene glycol, in which, after the 1,3-butylene glycol product has been kept at 180°C for 3 hours in air atmosphere, at least one of contents of compounds represented by the following Formula (A) or (B) is less than 8 ppm:

[Chemical Formula 3]

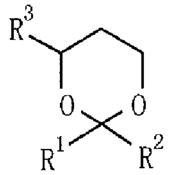
(A)

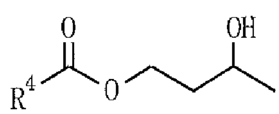
(B)

where $R^1$ to $R^4$ are the same as or different from each other, and each of $R^1$ to $R^4$ is a hydrogen atom, an alkyl group

with from 1 to 4 carbon atoms which may be substituted with a hydroxy group, or an alkenyl group with 2 to 4 carbon atoms which may be substituted with a hydroxy group. The compound represented by Formula (A) is a cyclic acetal compound, and the compound represented by Formula (B) is a 3-hydroxybutyl carboxylate compound. These compounds are also present as impurities during production of 1,3-butylene glycol, but are impurities whose content increases over time, when the 1,3-butylene glycol product is stored for a long period of time. The compound represented by Formula (A) is hydrolyzed in the presence of water to produce a corresponding carbonyl compound. The thus-produced carbonyl compound is itself a reducing substance, not only diminishes the potassium permanganate test value, but also is highly reactive and likely to produce a variety of complex compounds by heat or oxygen, and may become a substance that causes coloration, a substance that causes odor or a substance that causes acidity. Also, the compound represented by Formula (B) is hydrolyzed in the presence of water to produce a corresponding carboxylic acid. The thus-produced carboxylic acid may be a substance that causes odor or a substance that causes acidity.

In Formulas (A) and (B), $R^1$ to $R^4$ are the same as or different from each other, and each of $R^1$ to $R^4$ is a hydrogen atom, an alkyl group with from 1 to 4 carbon atoms which may be substituted with a hydroxy group, or an alkenyl group with 2 to 4 carbon atoms which may be substituted with a hydroxy group. Examples of the alkyl group with from 1 to 4 carbons which may be substituted with a hydroxy group include methyl, ethyl, propyl, butyl, 2-hydroxyethyl, and 2-hydroxypropyl groups. Examples of the alkenyl group with 2 to 4 carbon atoms which may be substituted with a hydroxy group include vinyl and 1-propenyl groups. Examples of $R^1$ include a hydrogen atom and methyl group. Examples of $R^2$ include a hydrogen atom, methyl, vinyl, 2-hydroxyethyl, 2-hydroxypropyl, 1-propenyl, and butyl group. Examples of $R^3$ include a methyl group. Examples of $R^4$ include a hydrogen atom, methyl, 2-hydroxypropyl, and 1-propenyl group.

Representative examples of the compound represented by Formula (A) include the compounds represented by the following formulas (1) to (7). Representative examples of the compound represented by Formula (B) include the compounds represented by the following formulas (8) to (10).

[Chem. 4]

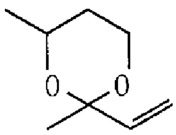

(1)

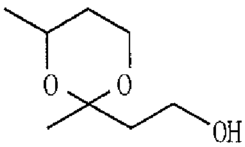

(2)

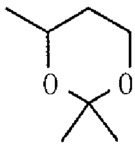

(3)

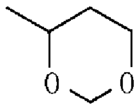

(4)

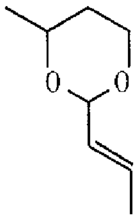

(5)

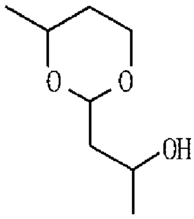

(6)

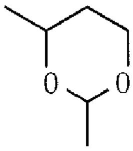

(7)

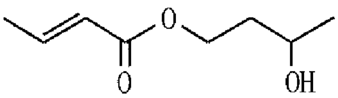

(8)

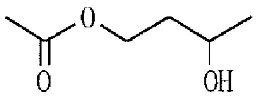

(9)

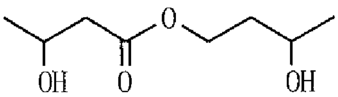

(10)

A content of each of the compounds represented by Formula (A) or (B) [e.g., compounds represented by Formulas (1) to (10) above] can be quantified by GC-MS analysis under the following conditions. In GC-MS analysis, even very small peaks are all subjected to mass spectrometry, and each component is quantified. Since the analysis is performed for a specific mass, a substance different in mass is not detected even when another impurity overlaps the peak. Therefore, the analysis is more sensitive than GC analysis which will be described below. In the present specification, the unit "ppm" of the content of each component determined by GC-MS analysis [content of each compound or the like represented by Formula (A) or (B)] means "ppm by weight".

Conditions for GC-MS analysis

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Ion source temperature: EI 230°C, CI 250°C
Q Pole temperature: 150°C
Sample: subjected to analysis as it is

In the above analytical conditions, the retention time of a peak of 1,3-butylene glycol is usually from 5.5 minutes to 7 minutes. Under the analysis conditions described above, when the relative retention time of the peak of 1,3-butylene

glycol is defined as 1.0, usually, a relative retention time of a peak of the compound represented by Formula (1) is from 1.3 to 1.7, and a relative retention time of a peak of the compound represented by Formula (2) is from 1.6 to 2.0, a relative retention time of a peak of the compound represented by Formula (3) is between 0.7 and 1.0, a relative retention time of a peak of the compound represented by Formula (4) is between 0.4 to 0.6, a relative retention time of a peak of the compound represented by Formula (5) is from 1.3 to 1.7, a relative retention time of a peak of the compound represented by Formula (6) is from 1.6 to 2.0, a relative retention time of a peak of the compound represented by Formula (7) is from 0.6 to 0.8, a relative retention time of a peak of the compound represented by Formula (8) is from 1.6 to 2.0, a relative retention time of a peak of the compound represented by Formula (9) is from 1.0 to 1.2, and a relative retention time of a peak of the compound represented by Formula (10) is from 1.6 to 2.0.

Note that the compound represented by Formula (1) is a compound produced by a reaction (an acetalization reaction) of methyl vinyl ketone with 1,3-butylene glycol. Methyl vinyl ketone is produced by a dehydration reaction of 1-hydroxy-3-butanone. 1-hydroxy-3-butanone is produced by oxidation of 1,3-butylene glycol. The compound represented by Formula (2) is a compound produced by a reaction (an acetalization reaction) of 1-hydroxy-3-butanone with 1,3-butylene glycol. The compound represented by Formula (3) is a compound produced by a reaction (an acetalization reaction) of acetone with 1,3-butylene glycol. The compound represented by Formula (4) is a compound produced by a reaction (an acetalization reaction) of formaldehyde with 1,3-butylene glycol. Acetone and formaldehyde are produced by decomposition of 1-hydroxy-3-butanone. The compound represented by Formula (5) is a compound produced by a reaction (an acetalization reaction) of crotonaldehyde with 1,3-butylene glycol. Crotonaldehyde is produced by a dehydration reaction of acetaldol. Acetaldol is produced by oxidation of 1,3-butylene glycol. Acetaldol is also produced by dimerization of acetaldehyde. The compound represented by Formula (6) is produced by a reaction (an acetalization reaction) of acetaldol with 1,3-butylene glycol. The compound represented by Formula (7) is produced by a reaction (an acetalization reaction) of acetaldehyde with 1,3-butylene glycol. Acetaldehyde is produced by decomposition of acetaldol. The compound represented by Formula (8) is produced by a reaction (an esterification reaction) of crotonic acid with 1,3-butylene glycol. Crotonic acid is produced by oxidation of crotonaldehyde. The compound represented by Formula (9) is produced by a reaction (an esterification reaction) of acetic acid with 1,3-butylene glycol. Acetic acid is produced by oxidation of acetaldehyde. The compound represented by Formula (10) is produced by a reaction (an esterification reaction) of 3-hydroxybutanoic acid with 1,3-butylene glycol. 3-hydroxybutanoic acid is produced by oxidation of acetaldol. In addition, the oxidation of formaldehyde produces formic acid, and a reaction between the formic acid and 1,3-butylene glycol (an esterification reaction) produces 2-hydroxypropyl formate. Decarbonation of the 2-hydroxypropyl formate produces 2-butanol. An estimated formation pathway for each compound (impurity) (sometimes referred to as "impurity formation pathway diagram") is illustrated below. In the following formula, "1,3 BG" represents 1,3-butylene glycol.

[Chem. 5]

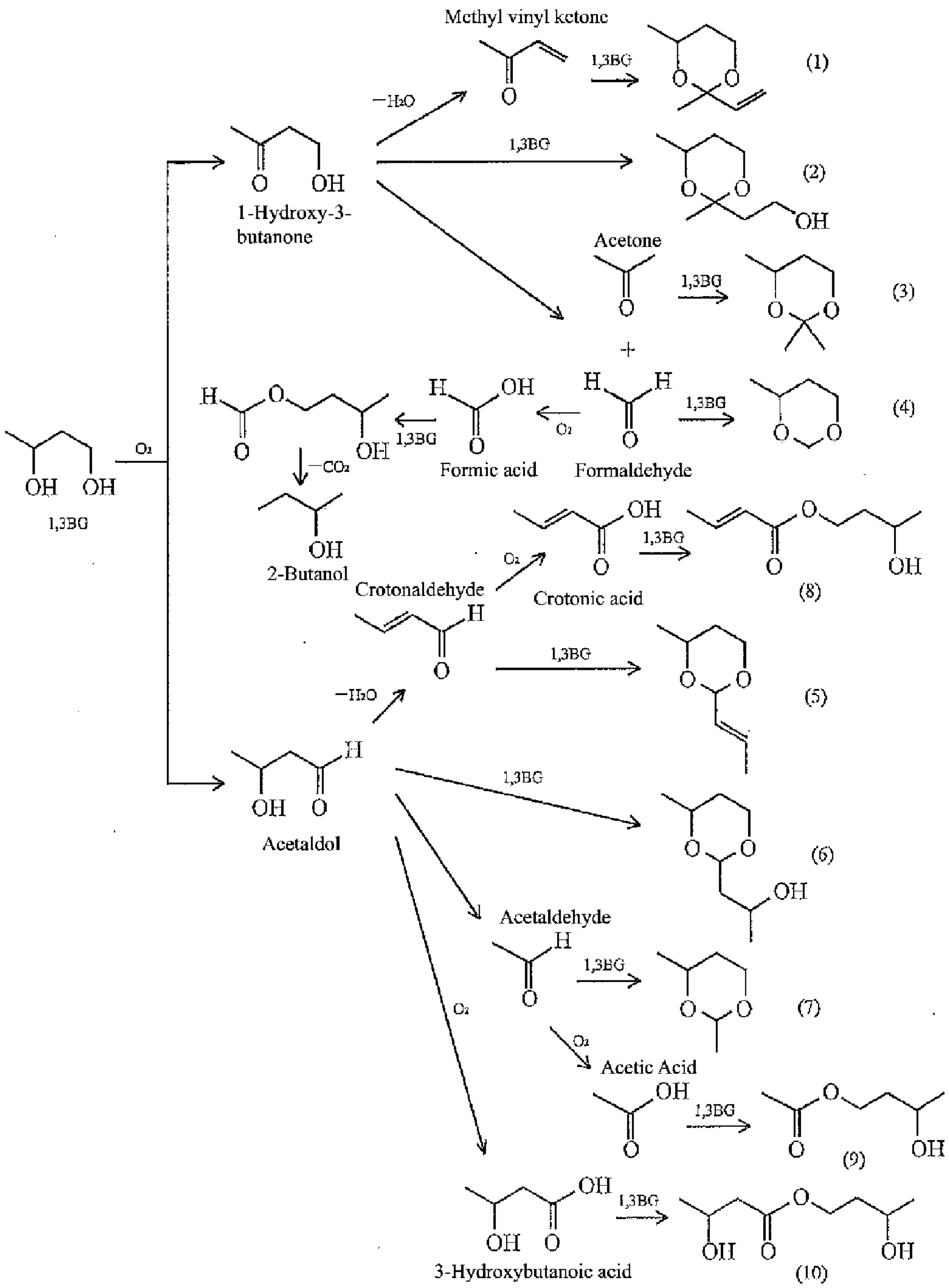


In the 1,3-butylene glycol product according to the present disclosure, after the 1,3-butylene glycol product has been kept at 180°C for 3 hours in air atmosphere, at least one of contents of compounds represented by Formula (A) or (B) [for example, compounds represented by Formulas (1) to (10)] is less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm). Thus, of the contents of the compounds represented by Formula (A) or (B) [for example, the compounds represented by Formulas (1) to (10)], a content of one compound may be less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm), and contents of two compounds (or three compounds, four compounds, five compounds, six compounds, seven compounds, eight compounds, nine compounds, ten compounds, etc.) may be each less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm).

In the 1,3-butylene glycol product according to the present disclosure, after the 1,3-butylene glycol product has

been kept at 180°C for 3 hours in air atmosphere, contents of at least four (four, five, six, seven, eight, nine, or ten) compounds, of the contents of the compounds represented by Formula (A) or (B) [for example, the compounds represented by Formulas (1) to (10)], are each less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm). Particularly, all of the contents of the compounds represented by Formulas (1) to (10) are preferably less than 8 ppm (e.g., 7 ppm or less, preferably 6 ppm or less, more preferably 5 ppm or less, and even more preferably 4 ppm or less, 3 ppm or less, 2 ppm or less, 1 ppm or less, 0.5 ppm or less, 0.4 ppm or less, 0.3 ppm or less, or less than 0.2 ppm).

In the 1,3-butylene glycol product according to the present disclosure, after the 1,3-butylene glycol product has been kept at 180°C for 3 hours in air atmosphere, at least one (e.g., one, two, three, four, ... or ten) of sums of contents of two compounds, of the contents of the compounds represented by Formula (A) or (B) [for example, the compounds represented by Formulas (1) to (10)], is less than 16 ppm (e.g., 15 ppm or less, preferably 14 ppm or less, more preferably 13 ppm or less, even more preferably 12 ppm or less, and particularly preferably 11 ppm or less, 10 ppm or less, 9 ppm or less, 8 ppm or less, 7 ppm or less, 6 ppm or less, 5 ppm or less, 4 ppm or less, 3 ppm 2 ppm or less, 1 ppm or less, 0.5 ppm or less, 0.4 ppm or less, 0.3 ppm or less, or less than 0.2 ppm).

In the 1,3-butylene glycol product according to the present disclosure, after the 1,3-butylene glycol product has been kept at 180°C for 3 hours in air atmosphere, at least one (e.g., one, two, three, four, ...) of sums of contents of three compounds, of the contents of the compounds represented by Formula (A) or (B) [for example, the compounds represented by Formulas (1) to (10)], is less than 24 ppm (e.g., 20 ppm or less, preferably 18 ppm or less, more preferably 16 ppm or less, even more preferably 14 ppm or less, and particularly preferably 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

In the 1,3-butylene glycol product according to the present disclosure, after the 1,3-butylene glycol product has been kept at 180°C for 3 hours in air atmosphere, at least one (e.g., one, two, ...) of sums of contents of four compounds, of the contents of the compounds represented by Formula (A) or (B) [for example, the compounds represented by Formulas (1) to (10)], is less than 32 ppm (e.g., 30 ppm or less, preferably 25 ppm or less, more preferably 20 ppm or less, even more preferably 18 ppm or less, and particularly preferably 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Also, in the 1,3-butylene glycol product according to the present disclosure, after the 1,3-butylene glycol product has been kept at 180°C for 3 hours in air atmosphere, a sum of the contents of the compounds represented by Formula (A), in particular, a sum of contents of compounds represented by Formulas (1) to (7), is less than 66 ppm (e.g., 60 ppm or less, preferably 50 ppm or less, more preferably 40 ppm or less, even more preferably 30 ppm or less, and particularly preferably 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Also, in the 1,3-butylene glycol product according to the present disclosure, after the 1,3-butylene glycol product has been kept at 180°C for 3 hours in air atmosphere, a sum of the contents of the compounds represented by Formula (B), in particular, a sum of contents of compounds represented by Formulas (8) to (10), is less than 31 ppm (e.g., 25 ppm or less, preferably 20 ppm or less, more preferably 18 ppm or less, even more preferably 16 ppm or less, and particularly preferably 14 ppm or less, 13 ppm or less, 12 ppm or less, 11 pm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Further, in the 1,3-butylene glycol product according to the present disclosure, after the 1,3-butylene glycol product has been kept at 180°C for 3 hours in air atmosphere, a total content of the compounds represented by Formulas (1) to (4) is less than 34 ppm (e.g., 30 ppm or less, preferably 25 ppm or less, more preferably 20 ppm or less, even more preferably 18 ppm or less, and particularly preferably 16 ppm or less, 15 ppm or less, 14 ppm or less, 13 ppm or less, 12 ppm or less, 11 pm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Also, in the 1,3-butylene glycol product according to the present disclosure, after the 1,3-butylene glycol product has been kept at 180°C for 3 hours in air atmosphere, a total content of the compounds represented by Formulas (5) to (10) is less than 63 ppm (e.g., 60 ppm or less, preferably 50 ppm or less, more preferably 40 ppm or less, even more preferably 30 ppm or less, and particularly preferably 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Further, in the 1,3-butylene glycol product according to the present disclosure, after the 1,3-butylene glycol product has been kept at 180°C for 3 hours in air atmosphere, a total content of the compounds represented by Formulas

(1) to (10) is less than 97 ppm (e.g., 80 ppm or less, preferably 70 ppm or less, more preferably 60 ppm or less, even more preferably 50 ppm or less, and particularly preferably 40 ppm or less, 30 ppm or less, 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

Methyl vinyl ketone, 1-hydroxy-3-butanone, acetone, formaldehyde, crotonaldehyde, acetaldol, and acetaldehyde, as shown in the impurity formation pathway diagram, are precursors of the compounds represented by Formula (A) or (B). Butylaldehyde can also be a precursor of the compound represented by Formula (A) due to its structure. In addition, these compounds are carbonyl compounds, and are prone to produce various complex compounds by heat or oxygen, and may be a substance that causes coloration, a substance that causes odor or a substance that causes acidity. Therefore, the 1,3-butylene glycol product desirably has low contents of these compounds after being kept at 180°C for 3 hours in air atmosphere. For example, the 1,3-butylene glycol product of the present disclosure preferably has a content of methyl vinyl ketone of less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm) and/or a content of 1-hydroxy-3-butanone of less than 1 ppm (preferably 0.5 ppm or less), and/or a content of acetone of less than 6 ppm (e.g., 5 ppm or less, preferably 4 ppm or less, more preferably 3 ppm or less, even more preferably 2 ppm or less, 1 ppm or less, 0.5 ppm or less, 0.4 ppm or less, 0.3 ppm or less, or less than 0.2 ppm), and/or a content of formaldehyde of less than 2 ppm (preferably 1 ppm or less, more preferably 0.5 ppm or less, 0.4 ppm or less, 0.3 ppm or less, or less than 0.2 ppm), and/or a content of crotonaldehyde of less than 1 ppm (preferably 0.5 ppm or less), and/or a content of acetaldol of less than 1 ppm (preferably 0.5 ppm or less, 0.4 ppm or less, 0.3 ppm or less, or less than 0.2 ppm), and/or a content of acetaldehyde of less than 5 ppm (e.g., 4 ppm or less, preferably 3 ppm or less, more preferably 2 ppm or less, even more preferably 1 ppm or less, 0.5 ppm or less, 0.4 ppm or less, 0.3 ppm or less, or less than 0.2 ppm), and/or a content of butylaldehyde of less than 4 ppm (e.g., 3 ppm or less, preferably 2 ppm or less, more preferably 1 ppm or less, even more preferably 0.5 ppm or less, 0.4 ppm or less, 0.3 ppm or less, or less than 0.2 ppm), after being kept at 180°C for 3 hours in air atmosphere.

Each of the contents of methyl vinyl ketone, 1-hydroxy-3-butanone, acetone, formaldehyde, crotonaldehyde, acetaldol, acetaldehyde, and butylaldehyde in the 1,3-butylene glycol product can be quantified by GC-MS analysis (gas mass analysis) under the conditions described above. When the relative retention time of the peak of 1,3-butylene glycol is defined as 1.0 under the above analysis conditions, usually, a relative retention time of a peak of methyl vinyl ketone is from 0.3 to 0.5, a relative retention time of a peak of 1-hydroxy-3-butanone is from 0.4 to 0.6, a relative retention time of a peak of acetone is from 0.3 to 0.5, a relative retention time of a peak of formaldehyde is from 0.3 to 0.5, a relative retention time of a peak of crotonaldehyde is from 0.3 to 0.5, a relative retention time of a peak of acetaldol is from 0.4 to 0.6, a relative retention time of a peak of acetaldehyde is from 0.3 to 0.5, and a relative retention time of a peak of butylaldehyde is from 0.3 to 0.5.

Also, the 1,3-butylene glycol product according to the present disclosure preferably has a total content of methyl vinyl ketone, 1-hydroxy-3-butanone, acetone, formaldehyde, crotonaldehyde, acetaldol, and acetaldehyde of less than 24 ppm (e.g., 20 ppm or less, preferably 18 ppm or less, more preferably 16 ppm or less, even more preferably 14 ppm or less, and particularly preferably 12 ppm or less, 11 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), after being kept at 180°C for 3 hours in air atmosphere.

Further, the 1,3-butylene glycol product according to the present disclosure preferably has a total content of methyl vinyl ketone, acetone, and butylaldehyde of less than 18 ppm (e.g., 16 ppm or less, preferably 15 ppm or less, more preferably 14 ppm or less, even more preferably 13 ppm or less, and particularly preferably 12 ppm or less, 11 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), after being kept at 180°C for 3 hours in air atmosphere. Methyl vinyl ketone, acetone and butylaldehyde are impurities present in relatively large amounts in the 1,3-butylene glycol product after the heating test.

Furthermore, in the 1,3-butylene glycol product according to the present disclosure, after the 1,3-butylene glycol product has been kept at 180°C for 3 hours in air atmosphere, an APHA is less than 79 (preferably 65 or less, more preferably 60 or less, even more preferably 55 or less, 50 or less, 45 or less, 40 or less, 35 or less, 30 or less, 25 or less, 20 or less, and particularly preferably 18 or less, 15 or less, 14 or less, 13 or less, 12 or less, 11 or less, 10 or less, 9 or less, 8 or less, or 7 or less).

Also, the 1,3-butylene glycol product according to the present disclosure preferably has an initial boiling point of 203°C or higher and preferably has a dry point of 209°C or lower, after the 1,3-butylene glycol product has been kept at 180°C for 3 hours in air atmosphere. The initial boiling point is preferably 204°C or higher, more preferably 205°C or higher, even more preferably 206°C or higher or 207°C, and particularly preferably 208°C or higher.

In the 1,3-butylene glycol product according to the present disclosure, after the 1,3-butylene glycol product has been kept at 180°C for 3 hours in air atmosphere, a potassium permanganate test value (PMT) is 30 minutes or longer.

The potassium permanganate test value is more preferably longer than 30 minutes (e.g., 32 minutes or longer), even more preferably 35 minutes or longer (e.g., 40 minutes or longer), and particularly preferably 50 minutes or longer (especially, 60 minutes or longer).

In the 1,3-butylene glycol product according to the present disclosure, after the 1,3-butylene glycol product has been kept at 180°C for 3 hours in air atmosphere, an acid concentration (in terms of acetic acid) is less than 16 ppm. The acid concentration (in terms of acetic acid) is more preferably 14 ppm or less, even more preferably 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm.

As described above, methyl vinyl ketone, 1-hydroxy-3-butanone, acetone, formaldehyde, crotonaldehyde, acetaldol, acetaldehyde, and butylaldehyde are precursors of the compounds represented by Formula (A) or (B). In particular, 1-hydroxy-3-butanone and acetaldol are precursors of various impurities. Therefore, these impurities are desirably as less as possible in the 1,3-butylene glycol product before the heating test. From such a viewpoint, the 1,3-butylene glycol product according to the present disclosure preferably has a total content of 1-hydroxy-3-butanone and acetaldol of less than 19 ppm (e.g., 18 ppm or less, preferably 16 ppm or less, more preferably 14 ppm or less, even more preferably 12 ppm or less, and particularly preferably 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm),, in a state where the 1,3-butylene glycol product has not undergone a test in which 1,3-butylene glycol product is kept at 180°C for 3 hours in air atmosphere (before the heating test).

Further, the 1,3-butylene glycol product according to the present disclosure preferably has a content of methyl vinyl ketone of less than 10 ppm (e.g., 8 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm), and/or a content of 1-hydroxy-3-butanone of less than 9 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm), and/or a content of acetone of less than 9 ppm (e.g., 7 ppm or less, preferably 6 ppm or less, more preferably 5 ppm or less, even more preferably. 4 ppm or less, 3 ppm or less, 2 ppm or less, 1 ppm or less, 0.5 ppm or less, 0.4 ppm or less, 0.3 ppm or less, or less than 0.2 ppm), and/or a content of formaldehyde of less than 3 ppm (e.g., 2 ppm or less, preferably 1 ppm or less, more preferably 0.5 ppm or less, 0.4 ppm or less, 0.3 ppm or less, or less than 0.2 ppm), and/or a content of crotonaldehyde of less than 2 ppm (e.g., 1.5 ppm or less, preferably 1 ppm or less, more preferably 0.5 ppm or less, 0.4 ppm or less, 0.3 ppm or less, or less than 0.2 ppm), and/or a content of acetaldol of less than 10 ppm (e.g., 8 ppm or less, preferably 6 ppm or less, more preferably 5 ppm or less, more preferably 4 ppm or less, 3 ppm or less, 2 ppm or less, 1 ppm or less, 0.5 ppm or less, 0.4 ppm or less, 0.3 ppm or less, or less than 0.2 ppm), and/or a content of acetaldehyde of or less 4 ppm (e.g., 3 ppm or less, preferably 2 ppm or less, more preferably 1 ppm or less, even more preferably 0.5 ppm or less, 0.4 ppm or less, 0.3 ppm or less, or less than 0.2 ppm) and/or a content of butylaldehyde of less than 8 ppm (e.g., 7 ppm or less, preferably 6 ppm or less, more preferably 5 ppm or less, even more preferably 4 ppm or less, 3 ppm or less, 2 ppm or less, 1 ppm or less, 0.5 ppm or less, 0.4 ppm or less, 0.3 ppm or less, or less than 0.2 ppm), in a state where the 1,3-butylene glycol product has not undergone a test in which 1,3-butylene glycol product is kept at 180°C for 3 hours in air atmosphere (before the heating test).

Furthermore, the 1,3-butylene glycol product according to the present disclosure preferably has a total content of methyl vinyl ketone, 1-hydroxy-3-butanone, acetone, formaldehyde, crotonaldehyde, acetaldol, and acetaldehyde of less than 47 ppm (e.g., 40 ppm or less, preferably 30 ppm or less, more preferably 25 ppm or less, even more preferably 20 ppm or less, and particularly preferably 18 ppm or less, 16 ppm or less, 14 ppm or less, 13 ppm or less, 12 ppm or less, 11 pm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), in a state where the 1,3-butylene glycol product has not undergone a test in which 1,3-butylene glycol product is kept at 180°C for 3 hours in air atmosphere (before the heating test).

Furthermore, the 1,3-butylene glycol product according to the present disclosure preferably has an acid concentration (in terms of acetic acid) of less than 11 ppm (e.g., 10 ppm or less, preferably 9 ppm or less, more preferably 8 ppm or less, even more preferably 7 ppm or less, particularly preferably 6 ppm or less, and most preferably 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), in a state where the 1,3-butylene glycol product has not undergone a test in which 1,3-butylene glycol product is kept at 180°C for 3 hours in air atmosphere (before the heating test). Reduction of the acid concentration in the 1,3-butylene glycol product can suppress an increase of impurities represented by Formula (B) over time.

With the 1,3-butylene glycol product according to the present disclosure, an acid concentration (in terms of acetic acid) of a 90 wt.% aqueous solution of the 1,3-butylene glycol product kept at 100°C for 1 week in air atmosphere is less than 23 ppm (e.g., 20 ppm or less, preferably 19 ppm or less, 18 ppm or less, 17 ppm or less, or 16 ppm or less, more preferably 15 ppm or less, even more preferably 14 ppm or less, 13 ppm or less, 12 ppm or less, 11 ppm or less, and particularly preferably 10 ppm or less, 9 ppm or less, 8 ppm or less, 7 ppm or less, 6 ppm or less, 5 ppm or less, 4

ppm or less, 3 ppm or less, 2 ppm or less, 1 ppm or less, 0.5 ppm or less, 0.4 ppm or less, 0.3 ppm or less, or less than 0.2 ppm), in a state where the 1,3-butylene glycol product has not undergone a test in which 1,3-butylene glycol product is kept at 180°C for 3 hours in air atmosphere (before the heating test). The 90 wt.% aqueous solution means an aqueous solution prepared by mixing the 1,3-butylene glycol product and water (e.g., pure water) to adjust the 1,3-butylene glycol product to 90 wt.%. Reduction of a concentration of an acid precursor in the 1,3-butylene glycol product can suppress an increase of impurities represented by Formula (B) over time.

For the acid concentration (in terms of acetic acid) of the 90 wt.% aqueous solution of the 1,3-butylene glycol product of the present disclosure, a ratio of the acid concentration after keeping the aqueous solution at 100°C for 1 week to the acid concentration before keeping the solution at 100°C for 1 week, [(acid concentration after keeping the solution at 100°C for 1 week)/(acid concentration before keeping the solution at 100°C for 1 week) × 100 (%)], is preferably 200% or less, more preferably 150% or less, and even more preferably 120% or less, in a state where the 1,3-butylene glycol product has not undergone a test in which 1,3-butylene glycol product is kept at 180°C for 3 hours in air atmosphere (before the heating test).

The 1,3-butylene glycol product according to the present disclosure has an APHA (Hazen color number) of, for example, 6 or less (preferably 5 or less, more preferably 4 or less, even more preferably 3 or less, and particularly preferably 2 or less), in a state where the 1,3-butylene glycol product has not undergone a test in which 1,3-butylene glycol product is kept at 180°C for 3 hours in air atmosphere (before the heating test). Furthermore, an APHA, after the 1,3-butylene glycol product has been kept at 100°C for 75 days in air atmosphere, is, for example, 42 or less (preferably 35 or less, 30 or less, 25 or less, 20 or less, 18 or less, 16 or less, 15 or less, 14 or less, or 13 or less, more preferably 12 or less, 11 or less, 10 or less, 9 or less, 8 or less, 7 or less, or 6 or less, and more preferably 5 or less, 4 or less, 3 or less, or 2 or less.

For the APHA of the 1,3-butylene glycol product according to the present disclosure, a ratio of the APHA after the 1,3-butylene glycol product has been kept at 100°C for 75 days to the APHA before the 1,3-butylene glycol product has been kept at 100°C for 75 days, [(APHA after the 1,3-butylene glycol product has been kept at 100°C for 75 days)/(APHA before the 1,3-butylene glycol product has been kept at 100°C for 75 days)], is not particularly limited, but is preferably 10 or less, more preferably 8 or less, even more preferably 7 or less, and particularly preferably 6 or less (for example, 5 or less, 4 or less, or 3 or less). In addition, the ratio is 1 or greater, or may be 2 or greater.

Additionally, the 1,3-butylene glycol product according to the present disclosure preferably has an initial boiling point of higher than 203°C, in a state where the 1,3-butylene glycol product has not undergone a test in which 1,3-butylene glycol product is kept at 180°C for 3 hours in air atmosphere (before the heating test). The initial boiling point is preferably 204°C or higher, more preferably 205°C or higher, even more preferably 206°C or higher or 207°C, and particularly preferably 208°C or higher.

Additionally, the 1,3-butylene glycol product according to the present disclosure preferably has a dry point of 209°C or lower, in a state where the 1,3-butylene glycol product has not undergone a test in which 1,3-butylene glycol product is kept at 180°C for 3 hours in air atmosphere (before the heating test).

Furthermore, the 1,3-butylene glycol product according to the present disclosure preferably has a potassium permanganate test value (PMT) of 30 minutes or longer, in a state where the 1,3-butylene glycol product has not undergone a test in which 1,3-butylene glycol product is kept at 180°C for 3 hours in air atmosphere (before the heating test). The potassium permanganate test value (PMT) is more preferably longer than 30 minutes (e.g., 32 minutes or longer), even more preferably 35 minutes or longer (e.g., 40 minutes or longer), and particularly preferably 50 minutes or longer (especially, 60 minutes or longer).

In the 1,3-butylene glycol product according to the present disclosure, a content of 1,3-butylene glycol is, for example, 98.6% or greater. The content of the 1,3-butylene glycol corresponds to an area ratio (GC area ratio) of the peak of 1,3-butylene glycol, in the gas chromatographic analysis (GC analysis) performed under the following conditions.

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

In the present disclosure, the "(peak) area ratio" means a ratio of the area of a specific peak relative to the sum of the areas of all peaks appearing in the chromatogram. In addition, all peaks mean, for example, all of the peaks appearing in the analysis continued until and discontinued at a relative retention time of 7.8, provided that the relative

retention time of 1,3-butylene glycol is 1.0. The content of 1,3-butylene glycol in the 1,3-butylene glycol product is, for example, 98.6% or greater, and thus basic properties inherent in 1,3-butylene glycol are guaranteed.

The content (GC area ratio) of the 1,3-butylene glycol is preferably 98.7% or greater, more preferably 98.8% or greater, even more preferably 98.9% or greater, and particularly preferably 99.0% or greater. Furthermore, the content (GC area ratio) of 1,3-butylene glycol is especially 99.1% or greater, 99.2% or greater, 99.3% or greater, 99.4% or greater, 99.5% or greater, 99.6% or greater, 99.7% or greater, or 99.8% or greater.

In the GC analysis, a total area ratio of peaks shorter in retention time than the peak of 1,3-butylene glycol is preferably 0.28% or less, more preferably 0.25% or less, even more preferably 0.23% or less, 0.2% or less, 0.17% or less, 0.15% or less, 0.12% or less, 0.1% or less, 0.07% or less, 0.04% or less, 0.03% or less, 0.02% or less, 0.01% or less, or 0.007% or less, and particularly preferably 0.005% or less (for example, 0.002% or less).

In the GC analysis, the total area ratio of peaks longer in retention time than the peak of 1,3-butylene glycol is preferably 1% or less, more preferably 0.9% or less, more preferably 0.8% or less, 0.7% or less, 0.6% or less, 0.5% or less, 0.4% or less, 0.3% or less, or 0.2% or less, and particularly preferably 0.1% or less.

In the 1,3-butylene glycol product according to the present disclosure, a content of water is preferably less than 0.4 wt.%. The content of water is preferably 0.3 wt.% or less, more preferably 0.2 wt.% or less, even more preferably 0.1 wt.% or less, 0.07 wt.% or less, 0.05 wt.% or less, 0.03 wt.% or less, 0.02 wt.% or less, or 0.01 wt.% or less, and particularly preferably 0.005 wt.% or less. Note that the content of water can be quantified by a Karl Fischer water content measurement instrument.

A 1,3-butylene glycol product having a content of 1,3-butylene glycol of a specific value or greater and a content of a specific substance after the heating test under the specific conditions has high purity and high quality, and is hardly deteriorated in quality over time. Therefore, it can be suitably used as a raw material for moisturizers or cosmetic products.

Moisturizer and cosmetic product

A moisturizer of the present disclosure contains the 1,3-butylene glycol product described above. Therefore, the moisturizer has excellent moisturizing performance. The moisturizer of the present disclosure may contain a component besides the 1,3-butylene glycol product described above, such as a moisturizer component besides the 1,3-butylene glycol product described above. In the moisturizer of the present disclosure, the content of the 1,3-butylene glycol product described above is, for example, 10 wt.% or greater, preferably 30 wt.% or greater, more preferably 50 wt.% or greater, even more preferably 80 wt.% or greater, and particularly preferably 90 wt.% or greater, and the moisturizer may be composed of only the 1,3-butylene glycol product described above.

A cosmetic of the present disclosure contains the moisturizer described above. The blending amount of the 1,3-butylene glycol product in the cosmetic product of the present disclosure is any amount in which the moisturizing performance can be exhibited according to the type and form of cosmetic. The blending amount of the 1,3-butylene glycol product in the cosmetic product of the present disclosure is, for example, from 0.01 to 40 wt.%, preferably from 0.1 to 30 wt.%, more preferably from 0.2 to 20 wt.%, even more preferably from 0.5 to 15 wt.%, and particularly preferably from 1 to 10 wt.%.

The cosmetic product of the present disclosure may contain, in addition to the 1,3-butylene glycol product, for example, another moisturizer; an oil, such as a vegetable oil, a hydrocarbon oil, a greater fatty acid, a greater alcohol, or a silicone; a surfactant, such as an anionic surfactant, a cationic surfactant, an amphoteric surfactant, or a nonionic surfactant; a preservative, a sequestrant, a thickener, a powder, an ultraviolet absorber, an ultraviolet blocker, a fragrance, or a pH adjuster; or a medicinal ingredient or bioactive component, such as a vitamin preparation, a skin activator, a blood circulation promoter, a skin-lightening preparation, an antibacterial agent, or an anti-inflammatory agent.

The cosmetic product of the present disclosure can be a skin cosmetic product, such as a lotion, an emulsion, a cream, a gel, a pack, or a mask; or a hair cosmetic product, such as a shampoo, a rinse, or a hair restorer. In addition, the cosmetic product may be a sunscreen cosmetic product, a make-up cosmetic product or the like. Furthermore, the cosmetic product can be a pharmaceutical product or quasi drug containing a medical component.

The cosmetic product of the present disclosure can be produced by utilizing a method known per se.

Method for producing 1,3-butylene glycol

The 1,3-butylene glycol product of the present disclosure can be produced, for example, by a method 1 of producing 1,3-butylene glycol which will be described below, or a method 2 of producing 1,3-butylene glycol which will be described below.

Method 1 of producing 1,3-butylene glycol:

A method for producing 1,3-butylene glycol to yield purified 1,3-butylene glycol from a reaction crude liquid

containing 1,3-butylene glycol,

the method including: performing dehydration including removing water by distillation, removing a high boiling point component including removing a high boiling point component by distillation, and performing product distillation to yield purified 1,3-butylene glycol,
wherein, in a product column for use in the performing product distillation, a 1,3-butylene glycol charged liquid having a content of acetaldehyde of 500 ppm or less, a content of crotonaldehyde of 200 ppm or less, a content of water of 0.7 wt.% or less, and a concentration of 1,3-butylene glycol of 97.6 area % by gas chromatographic analysis under the following conditions is subjected to distillation under a condition that a reflux ratio is 0.3 or greater.

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

Method 2 of producing 1,3-butylene glycol:

A method for producing 1,3-butylene glycol to yield purified 1,3-butylene glycol from a reaction crude liquid containing 1,3-butylene glycol,

the method including: performing dehydration including removing water by distillation and removing a high boiling point component including removing a high boiling point component by distillation,
wherein, in a high boiling point component removal column for use in the removing a high boiling point component, a charged liquid containing 1,3-butylene glycol, which has a content of acetaldehyde of 500 ppm or less, a content of crotonaldehyde of 200 ppm or less, a content of water of 3 wt.% or less, and a concentration of 1,3-butylene glycol of 96.7 area % or greater by gas chromatographic analysis under the following conditions is subjected to distillation under a condition that a reflux ratio is 0.03 or greater.

The conditions for the gas chromatographic analysis are as follows:
same as in the method 1 of producing 1,3-Butylene glycol.

The method 1 for producing 1,3-butylene glycol described above (hereinafter, sometimes simply referred to as "production method 1") is a method for producing 1,3-butylene glycol to yield purified 1,3-butylene glycol from a reaction crude liquid containing 1,3-butylene glycol (1,3 BG) (hereinafter, sometimes referred to as "crude 1,3-butylene glycol"), the method including: performing dehydration including removing water by distillation, removing a high boiling point component including removing a high boiling point component by distillation, and performing product distillation to yield purified 1,3-butylene glycol. Then, in the product column for use in the performing product distillation, a 1,3-butylene glycol charged liquid having a content of acetaldehyde of 500 ppm or less, a content of crotonaldehyde of 200 ppm or less, a content of water of 0.7 wt.% or less, and a concentration of 1,3-butylene glycol of 97.6 area % by gas chromatographic analysis under the above conditions is subjected to distillation under a condition that a reflux ratio is 0.3 or greater, a liquid with a concentrated low boiling point component is distilled from above a charging plate, and 1,3-butylene glycol is extracted from below the charging plate. The thus-produced 1,3-butylene glycol is colorless and odorless (or almost colorless and odorless), unlikely to cause or increase coloration and odor over time, and, besides, unlikely to cause an acid concentration increase over time even in a state that the 1,3-butylene glycol contains water. Thus, such 1,3-butylene glycol can constitute a 1,3-butylene glycol product.

The method 2 for producing 1,3-butylene glycol (hereinafter, sometimes simply referred to as "production method 2") is a method for producing 1,3-butylene glycol to yield purified 1,3-butylene glycol from a reaction crude liquid containing 1,3-butylene glycol, the method including: performing dehydration including removing water by distillation and removing a high boiling point component including removing a high boiling point component by distillation. In a high boiling point component removal column for use in the removing a high boiling point component, a charged liquid containing 1,3-butylene glycol, which has a content of acetaldehyde of 500 ppm or less, a content of crotonaldehyde of 200 ppm or less, a content of water of 3 wt.% or less, and a concentration of 1,3-butylene glycol of 96.7 area % or greater by gas chromatographic analysis under the conditions above is subjected to distillation under a condition that a reflux

ratio is 0.03 or greater, 1,3-butylene glycol having improved purity is distilled from above the charging plate, and a liquid in which a high boiling point component is concentrated is extracted from below the charging plate. The thus-produced 1,3-butylene glycol is colorless and odorless (or almost colorless and odorless), hardly causes or increases coloration over time, and, besides, is unlikely to cause an acid concentration increase over time even in a state that the 1,3-butylene glycol contains water. Thus, such 1,3-butylene glycol can constitute a 1,3-butylene glycol product.

Crude 1,3-butylene glycol

Examples of the crude 1,3-butylene glycol include (1) a reaction crude liquid produced by reduction (hydrogenation) of an acetaldol, (2) a reaction crude liquid produced by hydrolysis of 1,3-butylene oxide, (3) a reaction crude liquid produced by selective hydrocracking of erythritol, (4) a reaction crude liquid produced by selective hydration to butadiene, (5) a reaction crude liquid produced by hydrogen addition to n-butanal-3-one, (6) a reaction crude liquid produced by hydrogen addition to 1-butanol-3-one, (7) a reaction crude liquid produced by hydrogen addition to 3-hydroxy-1-butanoic acid, (8) a reaction crude liquid produced by hydrogen addition to β-butyrolactone, and (9) a reaction crude liquid produced by hydrogen addition to diketene. In the present disclosure, the crude 1,3-butylene glycol may be one type or a mixture of two or more types of the above (1) to (9). The crude 1,3-butylene glycol is preferably (1) the reaction crude liquid produced by reduction (in particular, liquid phase reduction) of an acetaldol.

Hereinafter, a case where the reaction crude liquid produced by reduction (hydrogenation) of an acetaldol is used as the crude 1,3-butylene glycol will be mainly described. Note that the reduction (hydrogenation) of an acetaldol is sometimes referred to as "hydrogenation".

The acetaldol used as a raw material in the hydrogenation is not particularly limited as long as it is a compound that becomes 1,3-butylene glycol by hydrogen reduction. Examples of the raw material acetaldols include acetaldol; its cyclic dimer paraldol; aldoxane as a cyclic trimer of acetaldehyde; and mixtures of these.

The method of producing the acetaldol (e.g., acetaldol or paraldol) is not particularly limited, but the acetaldol may be, for example, those produced by an aldol condensation reaction of acetaldehyde in the presence of a basic catalyst or those produced by pyrolysis or the like of aldoxane. Note that the production of the acetaldol is sometimes referred to as "acetaldol production" or "acetaldehyde polymerization".

A reaction crude liquid produced by the reaction described above and containing an acetaldol may be neutralized with an acid and used in the production of 1,3-butylene glycol. Such a reaction crude liquid may contain, in addition to an acetaldol, acetaldehyde (AD), crotonaldehyde (CR), another aldehyde component; a low boiling point substance; a high boiling point substance, such as an aldehyde dimer or trimer; water; a salt; and the like. In the present specification, a compound with a lower boiling point than that of 1,3-butylene glycol may be referred to as a "low boiling point substance" or "low boiling substance", and a compound with a higher boiling point than that of 1,3-butylene glycol may be referred to as a "high boiling point substance" or "high boiling substance".

The reaction crude liquid containing an acetaldol may be subjected to a pretreatment, such as dealcoholization distillation, dehydration distillation, desalting, alkaline treatment and dealkalization treatment, or impurity removal, as necessary, and a product produced by removing by-products, such as unreacted acetaldehyde and crotonaldehyde, may be used. Examples of the pretreatment method include distillation, adsorption, ion exchange, conversion to a high boiling point substance by heating, and decomposition. For the distillation, a distillation method of various types, such as reduced pressure, normal pressure, increased pressure, azeotropic, extraction, or reaction, can be used. In particular, it is preferred that the reaction crude liquid containing an acetaldol is subjected to simple evaporation, distillation, or hydrogen addition to remove aldehydes such as acetaldehyde and crotonaldehyde, followed by the hydrogenation.

The content of the acetaldol in the raw material for hydrogenation is not particularly limited but is, for example, preferably 30 wt.% or greater (e.g., from 30 to 99 wt.%), more preferably 40 wt.% or greater (for example, from 40 to 98 wt.%), 50 wt.% or greater (for example, from 50 to 97 wt.%) or 60 wt.% or greater (for example, from 60 to 95 wt.%), and even more preferably from 65 to 90 wt.%, particularly preferably from 70 to 90 wt.%, and most preferably from 75 to 90 wt.%. With the content of the acetaldol within the above ranges, impurities contained in the reaction crude liquid containing 1,3-butylene glycol (crude 1,3-butylene glycol) tend to be reduced.

The raw material for hydrogenation may or may not contain water but preferably contains water from the viewpoint of the purity of 1,3-butylene glycol product. The water content in the raw material for hydrogenation is not particularly limited but is, for example, preferably 2 wt.% or greater, more preferably 5 wt.% or greater, even more preferably 10 wt.% or greater, and particularly preferably 15 wt.% or greater. The upper limit may be, for example, 90 wt.%, 80 wt.%, 70 wt.%, 60 wt.%, 50 wt.%, 40 wt.%, 30 wt.% or 20 wt.%. With the water content within the above ranges, the acetal of 1,3-butylene glycol and acetaldol contained in the resulting crude 1,3-butylene glycol is decreased, and thus this tends to increase the purity of the 1,3-butylene glycol product finally produced. This is because the raw material for hydrogenation contains water to a certain extent, and the acetal is hydrolyzed into 1,3-butylene glycol accordingly as well as coexisting acetaldol is reduced into 1,3-butylene glycol.

Examples of the hydrogenation catalyst include Raney nickel. The hydrogenation catalyst can also be used in

a suspended state, and can also be filled in a reaction vessel and used. The amount of the hydrogenation catalyst to be used is not particularly limited but is, for example, preferably from 1 to 30 parts by weight, more preferably from 4 to 25 parts by weight, even more preferably from 8 to 20 parts by weight, and particularly preferably from 12 to 18 parts by weight relative to 100 parts by weight of the raw material for hydrogenation. The amount of hydrogen to be used in the reduction reaction is not particularly limited but is, for example, preferably from 0.5 to 40 parts by weight, more preferably from 1 to 30 parts by weight, even more preferably from 4 to 20 parts by weight, and particularly preferably from 8 to 12 parts by weight relative to 100 parts by weight of the raw material for hydrogenation. A pressure (total pressure; gauge pressure) in a reaction system in the reduction reaction is not particularly limited but is, for example, preferably from 9 to 70 MPa and more preferably from 10 to 40 MPa. A hydrogen pressure (partial pressure of hydrogen) in the reaction system is not particularly limited, but is, for example, from 7 to 60 MPa, and preferably from 10 to 30 MPa. Note that, from the perspective of reducing the amount of the reducing materials such as acetaldehyde and crotonaldehyde, it is better to increase the hydrogen pressure in the reaction system, and the hydrogen pressure is preferably 10 MPa or greater, and may be 100 MPa. A reaction temperature in the reduction reaction is not particularly limited but is, for example, from 40 to 150°C, preferably from 50 to 140°C, and more preferably from 60 to 130°C. A reaction time (residence time) in the reduction reaction is not particularly limited but is, for example, from 10 to 500 minutes, preferably from 20 to 400 minutes, more preferably from 30 to 300 minutes, even more preferably from 50 to 280 minutes, and particularly preferably from 80 to 250 minutes. The present reaction can be carried out in any of a batch, semi-batch, or continuous manner.

For example, the thus-produced crude 1,3-butylene glycol contains a low boiling point substance (low boiling point compound) having an unsaturated bond, such as acetaldehyde (AD), butylaldehyde, crotonaldehyde (CR), acetone, and methyl vinyl ketone; a condensate of these; a condensate of 1,3-butylene glycol and the above low boiling point substance (e.g., an acetal of 1,3-butylene glycol and acetaldol); an alcohol such as ethanol, isopropyl alcohol, or butanol; water (for example, solvent), a salt produced by neutralization or the like, a catalyst (when used in suspension) or the like. These impurities are removed in the purification, and a 1,3-butylene glycol product (purified 1,3-butylene glycol) can be produced.

Purification of crude 1,3-butylene glycol

The production method 1 according to the present disclosure includes, at least, performing dehydration including removing water by distillation, removing a high boiling point component including removing a high boiling point component by distillation (high boiling point component removal distillation), and performing product distillation to yield purified 1,3-butylene glycol. In the production method 2 of the present disclosure, at least dehydration for removing water by distillation and high boiling point component removal for removing a high boiling point component by distillation (high boiling point component removal distillation).

In the production method of the present disclosure, the order of the performing dehydration and the removing a high boiling point component does not matter. In the production method 1, both the performing dehydration and the removing a high boiling point component are provided prior to the performing product distillation. The production method according to the present disclosure may include, in addition to these steps, desalting, alkaline reaction (alkaline treatment), and dealkalization. Additionally, prior to the dehydration, catalyst separation, neutralization by alkali, dealcoholization (low boiling point component removal), and the like can be provided. These steps may be performed in the order described above, but the order of the steps may be changed as appropriate except that the dealkalization is provided after the alkaline reaction. For example, the dealcoholization (low boiling point component removal), the desalting, the alkaline reaction, and the dealkalization can be provided at appropriate locations, but are usually provided after the hydrogenation. Note that, among the above-described steps, the catalyst separation, the neutralization by alkali, the dealcoholization (low boiling point component removal), the desalting, the alkaline reaction, and the dealkalization may be provided as necessary, and do not necessarily have to be provided.

FIG. 1 is a flowchart illustrating an example of a production method (purification method) for producing a 1,3-butylene glycol product of the present disclosure. A is a dehydration column and is related to the dehydration. B is a desalting column and is related to the desalting. C is a distillation column for removing a high boiling point component (high boiling point component removal column) and is related to the high boiling point component removal distillation (high boiling point component removal). D is an alkaline reactor and is related to the alkaline reaction. E is a dealkalization column and is related to the dealkalization. F is a product distillation column (product column) and is related to the product distillation. A-1, B-1, C-1, E-1, and F-1 are condensers. A-2, C-2, and F-2 are reboilers. Hereinafter, an example of an embodiment of the method for producing 1,3-butylene glycol will be described using the present flow sheet.

Crude 1,3-butylene glycol (corresponding to "X-1") produced by hydrogen reduction of a raw material for hydrogenation is fed to the dehydration column A. Note that the crude 1,3-butylene glycol (corresponding to "X-1") may be fed to the dehydration column A after undergoing the dealcoholization (distillation by a dealcoholization column) for removing an alcohol such as ethanol and a low boiling point substance.

In the production method of the present disclosure, in the dehydration column A used in the dehydration, for example, a charged liquid containing 1,3-butylene glycol and water is subjected to distillation, and a liquid having a concentrated low boiling point component containing water is distilled from above the charging plate (preferably, the top of the column) (corresponding to "X-2" in FIG. 1). Further, a crude 1,3-butylene glycol stream containing 1,3-butylene glycol can be produced from below the charging plate (preferably, the bottom of the column).

The dehydration column A and any other distillation column for separating 1,3-butylene glycol can be, for example, perforated-plate columns, bubble columns, and the like, but are more preferably packed columns with a low pressure loss, filled with Sulzer Packing, Melapack (trade names of Sumitomo Heavy Industries, Ltd.). This is because 1,3-butylene glycol and trace impurities would be thermally decomposed at a high temperature (e.g., 150°C or greater) and form a low boiling point substance, which is a coloring component, and thus the distillation temperature is to be lowered. In addition, this is also because a long thermal history (residence time) for 1,3-butylene glycol would also have a similar effect. Thus, the reboiler employed is preferably one with a short residence time of the process side fluid, for example, a thin-film evaporator, such as a natural downward flow thin-film evaporator or a forced-stirring thin-film evaporator.

A theoretical number of plates of the dehydration column A is, for example, from 1 to 100 plates, preferably from 2 to 80 plates, from 3 to 80 plates, from 4 to 60 plates, from 5 to 40 plates, from 6 to 30 plates or from 7 to 20 plates, and more preferably from 8 to 15 plates. A feed position for the charged liquid is, for example, from 10 to 90%, preferably from 20 to 80%, and more preferably from 30 to 70%, and even more preferably from 40 to 60% of a height of the column downward from the top of the column. In the distillation in the dehydration column A, the pressure (absolute pressure) of the top of the column is, for example, 101 kPa or less, preferably from 0.1 to 90 kPa, more preferably from 0.5 to 70 kPa, and even more preferably from 1 to 50 kPa, from 2 to 30 kPa or from 3 to 20 kPa, and particularly preferably from 4 to 10 kPa. Note that the distillation in the dehydration column A may be performed under increased pressure, in which case the pressure (gauge pressure) at the top of the column may be, for example, 0.2 MPaG or less, or 0.1 MPaG or less.

A concentration of 1,3-butylene glycol in the charged liquid into the dehydration column A is, for example, 9 wt.% or greater, preferably 10 wt.% or greater, more preferably 15 wt.% or greater, even more preferably 20 wt.% or greater, 25 wt.% or greater, 30 wt.% or greater, 35 wt.% or greater, 40 wt.% or greater, 45 wt.% or greater, 50 wt.% or greater, 55 wt.% or greater, or 60 wt.% or greater, and particularly preferably 70% or greater. An upper limit of the concentration of 1,3-butylene glycol in the charged liquid into the dehydration column A is, for example, 90 wt.%, 85 wt.%, or 80 wt.%. However, in consideration of the hydrogen addition reaction and the like in the step prior to the dehydration, the concentration of water in the charged liquid into the dehydration column A is preferably greater in some cases. Taken together, the concentration of 1,3-butylene glycol in the charged liquid into the dehydration column A may be, for example, 1 wt.% or greater, 5 wt.% or greater, 10 wt.% or greater, 15 wt.% or greater, 20 wt.% or greater, 25 wt.% or greater, 30 wt.% or greater, 35 wt.% or greater, 40 wt.% or greater, 50 wt.% or greater, 60 wt.% or greater, 70 wt.% or greater, 80 wt.% or greater, or 90 wt.% or greater. Furthermore, the concentration of 1,3-butylene glycol in charged liquid into the dehydration column A may be, for example, 99 wt.% or less, 95 wt.% or greater, 90 wt.% or less, 85 wt.% or less, 80 wt.% or less, 75 wt.% or less, 70 wt.% or less, 65 wt.% or less, 60 wt.% or less, 55 wt.% or less, 50 wt.% or less, or 45 wt.% or less. The concentration of 1,3-butylene glycol in the charged liquid into the dehydration column A can be, for example, in the range described above by adjusting the reaction conditions in the hydrogenation (for example, the concentration of an acetaldol used as a raw material) and the distillation conditions of the dealcoholization column (low boiling point component removal column) provided before the dehydration column as needed.

The concentration (wt.%) of 1,3-butylene glycol is a value determined according to the following formula by determining an area proportion (GC area %) of the peak of 1,3-butylene glycol relative to a total peak area in the gas chromatographic analysis under the following conditions. Note that the concentration (wt.%) of water in the charged liquid into the dehydration column A is a value measured by the method which will be described below (Karl Fischer method).

Concentration (wt.%) of 1,3-butylene glycol in charged liquid into dehydration column A
= [1 - (concentration (wt.%) of water in charged liquid into dehydration column A)/100] × (GC area % of 1,3-butylene glycol described above)

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

In the production method of the present disclosure, a content of acetaldehyde in the charged liquid into the dehydration column A is, for example, 1000 ppm or less, preferably 900 ppm or less, more preferably 800 ppm or less, 700 ppm or less, 600 ppm or less, or 500 ppm or less, even more preferably 400 ppm or less, 300 ppm or less, 200 ppm or less, 155 ppm or less, or 140 ppm or less, and may be 100 ppm or less, 90 ppm or less, 80 ppm or less, 70 ppm or less, 60 ppm or less, 50 ppm or less, 40 ppm or less, or 30 ppm or less, 20 ppm or less, 10 ppm or less, 5 ppm or less, 3 ppm or less, 2 ppm or less, or 1 ppm or less.

The content of crotonaldehyde in charged liquid into the dehydration column A may be, for example, 400 ppm or less, preferably 300 ppm or less, more preferably 200 ppm or less, even more preferably 150 ppm or less, 130 ppm or less, 117 ppm or less, or 100 ppm or less, and may be 90 ppm or less, 80 ppm or less, 70 ppm or less, 60 ppm or less, 50 ppm or less, 40 ppm or less, 30 ppm or less, 20 ppm or less, 10 ppm or less, 5 ppm or less, 3 ppm or less, 2 ppm or less, or 1 ppm or less.

The acetaldehyde content and the crotonaldehyde content in the charged liquid into the dehydration column A can be reduced, for example, by providing a dealcoholization column (low boiling point component removal column) upstream of the dehydration column A, and adjusting the distillation conditions of the dealcoholization column (low boiling point component removal column). For example, increasing the reflux ratio and the number of plates, and the distillation ratio of the dealcoholization column (low boiling point component removal column) can reduce the acetaldehyde content and the crotonaldehyde content of the charged liquid into the dehydration column A. Furthermore, the contents can be adjusted according to the conditions for the hydrogen addition reaction in the hydrogenation, and when hydrogen addition is completely performed, the concentrations of acetaldehyde and crotonaldehyde can be reduced to below the detection limit, but there are disadvantages of a high reaction pressure, an increase in size of the reaction tank and the like.

Note that the acetaldehyde content and the crotonaldehyde content of the charged liquid into the dehydration column A can be quantified by GC-MS analysis (gas mass spectrometry).

In the production method of the present disclosure, the content of water in the charged liquid into the dehydration column A is, for example, 90 wt.% or less, 85 wt.% or less, 80 wt.% or less, 70 wt.% or less, 60 wt.% or less, 50 wt.% or less, or 40 wt.% or less, and preferably 35 wt.% or less, more preferably 30 wt.% or less, and even more preferably 25wt.% or less. A lower limit of the water content of the charged liquid into the dehydration column A is, for example, 10 wt.% or 15 wt.%. Note that, when the hydrogen addition reaction in the hydrogenation is taken into consideration, the greater the water concentration and the smaller the viscosity, the greater the solubility and dispersity of hydrogen in the liquid, which is advantageous for the hydrogen addition reaction. The water content of the charged liquid into the dehydration column A can be reduced, for example, by providing a dealcoholization column (low boiling point component removal column) upstream of the dehydration column A, and adjusting the distillation conditions of the dealcoholization column (low boiling point component removal column). For example, increasing the reflux ratio and the number of plates, and the distillation ratio of the dealcoholization column (low boiling point component removal column) can reduce the water content of the charged liquid into the dehydration column A. Note that the water content of the charged liquid into the dehydration column A can be quantified by the Karl Fischer water content measurement instrument.

In the production method of the present disclosure, the content of the low boiling point component (excluding water) in the charged liquid into the dehydration column A is, for example, 20% or less, preferably 10% or less, more preferably 8% or less, even more preferably 5% or less, and particularly preferably 3% or less or 2% or less, and may be 1% or less, 0.5% or less, or 0.1% or less. The content of the low boiling point component (also referred to as "low boiling point substance" or "low boiling substance") excluding water in the charged liquid into the dehydration column A is a total area proportion (area %) of peaks of shorter in retention time than the peak of 1,3-butylene glycol relative to the total peak area in the gas chromatographic analysis under the above conditions. The content of the low boiling point component (excluding water) in the charged liquid into the dehydration column A can be reduced, for example, by providing a dealcoholization column (low boiling point component removal column) upstream of the dehydration column A, and adjusting the distillation conditions of the dealcoholization column (low boiling point component removal column). For example, increasing the reflux ratio and the number of plates, and the distillation ratio of the dealcoholization column (low boiling point component removal column) can reduce the content of the low boiling point component (excluding water) in the charged liquid into the dehydration column A. Furthermore, the concentration of the low boiling point component (excluding water) in the charged liquid into the dehydration column A can be reduced, for example, by reaction conditions (e.g., reaction temperature) in the hydrogenation.

The content of the high boiling point component in the charged liquid into the dehydration column A is, for example, 20% or less, preferably 10% or less, more preferably 7% or less, 4% or less, 3% or less, or 2% or greater, even more preferably 1% or less, 0.5% or less, 0.4% or less, 0.3% or less, 0.2% or less, 0.1% or less, or 0.05% or less, and particularly preferably 0.01% or less. The content of the high boiling point component in the charged liquid into the dehydration column A can be adjusted, for example, by reaction conditions (e.g., reaction temperature) in the hydrogenation. The content of the high boiling point component in the charged liquid into the dehydration column A is a total area proportion (area %) of peaks longer in retention time than the peak of 1,3 BG to the total peak area in the gas chromatographic analysis under the above conditions. (area %).

In the production method of the present disclosure, the reflux ratio [dehydration column reflux amount/dehydration column distilled amount (discharge amount to outside of distillation column)] in the dehydration column A is, for example, greater than 0.3, preferably 0.4 or greater, 0.5 or greater, 0.6 or greater, or 0.7 or greater, 0.8 or greater, 0.9 or greater, 1 or greater, 1.1 or greater, 1.2 or greater, 1.3 or greater, 1.4 or greater, 1.5 or greater, 1.6 or greater, 1.7 or greater, 1.8 or greater, 1.9 or greater, 2 or greater, 3 or greater, 4 or greater, 5 or greater, 6 or greater, 7 or greater, 8 or greater, 9 or greater, 10 or greater, 15 or greater, 20 or greater or 25 or greater, and more preferably 30 or greater (for example, 40 or greater) from the perspective of reducing the content of the low boiling point substance (including water) in the crude 1,3-butylene glycol stream containing 1,3-butylene glycol taken out from below the charging plate of the dehydration column A (preferably, the bottom of the column). In particular, in the production method 2 of the present disclosure, the reflux ratio in the dehydration column A is preferably 10 or greater, more preferably 20 or greater, even more preferably 30 or greater, and particularly preferably 50 or greater. An upper limit of the reflux ratio is, for example, 100, preferably 50 from the point of energy cost. In a case where the theoretical number of plates of the dehydration column A is sufficiently large, sufficient separation can be performed, for example, if the reflux ratio is 0.03 or greater. Note that, in the reflux to the dehydration column A, a condensate of dehydration column top vapor is ordinarily refluxed to the dehydration column. But some or all of the refluxes may be replaced with the charge of the water-containing liquid (e.g., pure water) into the dehydration column. In this case, the "dehydration column reflux amount" refers to a sum of the amount of reflux of the condensate of the dehydration column top vapor to the dehydration column, and the amount of the water-containing liquid (e.g., pure water) charged into the dehydration column.

In the production method of the present disclosure, the distillation ratio in the dehydration column A can be appropriately set in accordance with the concentration of water in the charged liquid into the dehydration column A. Desirably, the distillation ratio is a sufficient distillation ratio for the total amount of water in the charged liquid to be distilled. For example, in a case where the concentration of water in the charged liquid into the dehydration column A is X wt.%, the distillation ratio in the dehydration column A is preferably X wt.% or greater. Therefore, the distillation ratio in the dehydration column A is, for example, 95 wt.% or less, 90 wt.% or less, 85 wt.% or less, 80 wt.% or less, 75 wt.% or less, 70 wt.% or less, 65 wt.% or less, 60 wt.% or less, 55 wt.% or less, 50 wt.% or less, 45 wt.% or less, 40 wt.% or less, 35 wt.% or less, 30 wt.% or less, 25 wt.% or less, 20 wt.% or less, 15 wt.% or less, 10 wt.% or less, or 5 wt.% or less. The distillation ratio refers to a proportion (wt.%) of an amount of liquid extracted from above the charging plate of the dehydration column A (e.g., the top of the column) to the outside of the distillation column with respect to a charged amount into the dehydration column A.

In the production method of the present disclosure, the 1,3 BG recovery ratio in the dehydration column A is, for example, 99.3% or greater. Note that, in the present specification, the 1,3 BG recovery ratio in the dehydration column A is a value (%) determined by the following formula.

$$\{1\text{-[concentration (wt.\%) of 1,3 BG in distillate} \times \text{(distilled amount (part) - recycled amount (part))]/(concentration (wt.\%) of 1,3 BG in charged liquid} \times \text{charged amount (part))}\} \times 100$$

Note that the low boiling point substance and the high boiling point substance may be hydrolyzed by water to produce 1,3 BG, while the high boiling point substance may be formed by polymerization of 1,3 BG. Further, trace impurities may be formed or disappear. Thus, the mass balance in the dehydration column may not always be retained. This applies to the dealcoholization column (low boiling point component removal column), the high boiling point component removal column, the product column, and other distillation columns.

Next, the crude 1,3-butylene glycol stream containing 1,3-butylene glycol taken out from below the charging plate of the dehydration column A (preferably, the bottom of the column) is fed to the desalting column B. In the desalting column B, the crude 1,3-butylene glycol stream after the desalting is produced from the top of the column, and a salt, a high boiling point substance, or the like is discharged from the bottom of the column. The bottom ratio (%) of the desalting column B [(desalting column bottom amount (part)/desalting column charge amount (part) $\times$ 100] is, for example, from 0.1 to 40 wt.%, preferably from 1 to 35 wt.%, more preferably from 2 to 30 wt.%, even more preferably from 3 to 25 wt.%, and particularly preferably from 5 to 20 wt.%, and may be from 7 to 15 wt.%. At least a portion of the bottom in the desalting column may be recycled to the step prior to the desalting.

The crude 1,3-butylene glycol stream after the desalting described above is fed to the high boiling point component removal column C. In the high boiling point component removal column C, the high boiling point component (high boiling point substance) is discharged from below the charging plate (preferably, from the bottom of the column). On the other hand, the crude 1,3-butylene glycol stream after high boiling point substance removal (1,3-butylene glycol with improved purity) is produced from above the charging plate.

The high boiling point component removal column C can be, for example, a perforated-plate column, a bubble

column, or the like, but is more preferably a packed column with a low pressure loss, filled with Sulzer Packing, Melapack (trade names of Sumitomo Heavy Industries, Ltd.). This is because 1,3-butylene glycol and trace impurities would be thermally decomposed at a high temperature (e.g., 150°C or greater) and form a low boiling point substance, which is a coloring component, and thus the distillation temperature is to be lowered. In addition, this is also because a long thermal history (residence time) for 1,3-butylene glycol would also have a similar effect. Thus, the reboiler employed is preferably one with a short residence time of the process side fluid, for example, a thin-film evaporator, such as a natural downward flow thin-film evaporator or a forced-stirring thin-film evaporator.

A theoretical number of plates of the high boiling point component removal column C is, for example, from 1 to 100 plates, preferably from 2 to 90 plates, more preferably from 3 to 80 plates, more preferably from 4 to 70 plates, from 5 to 60 plates, from 8 to 50 plates, or from 10 to 40 plates, and particularly preferably from 15 to 30 plates. A feed position for the charged liquid is, for example, from 10 to 90%, preferably from 20 to 80%, and more preferably from 30 to 70 plates, and even more preferably from 40 to 60% of a height of the column downward from the top of the high boiling point component removal column. In the distillation in the high boiling point component removal column C, a pressure (absolute pressure) at the top of the column is, for example, from 0.01 to 50 kPa, preferably from 0.1 to 30 kPa, more preferably from 0.3 to 20 kPa, and even more preferably from 0.5 to 10 kPa.

In the production method 1 of the present disclosure, a concentration of 1,3 BG in the charged liquid into the high boiling point component removal column C is, for example, 95% or greater, preferably 96% or greater (for example, 96.7% or greater), more preferably 97% or greater, even more preferably 98% or greater, and particularly preferably 99% or greater. In the production method 2 of the present disclosure, the concentration of 1,3 BG in the charged liquid into the high boiling point component removal column C is 96.7% or greater, preferably 97% or greater, more preferably 98% or greater, and even more preferably 99% or greater. The concentration of 1,3 BG into the charged liquid into the high boiling point component removal column C can be improved by adjusting the distillation conditions of the dehydration column A and the desalting column B. For example, increasing the reflux ratio of the dehydration column A or increasing the bottom ratio of the desalting column B can increase the concentration of 1,3 BG in the charged liquid into the high boiling point component removal column C. Note that the concentration of the 1,3 BG described above is an area proportion (area %) of a 1, 3 BG peak relative to a total peak area in a gas chromatographic analysis (GC analysis) under the following conditions.

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

A content of the high boiling point component in the charged liquid into the high boiling point component removal column C is, for example, 4% or less, preferably 3% or less, more preferably 2% or less, even more preferably 1% or less, 0.5% or less, 0.4% or less, 0.3% or less, 0.2% or less, 0.1% or less, or 0.05% or less, and particularly preferably 0.01% or less. In particular, in the production method 2 of the present disclosure, a content of the high boiling point component in the charged liquid into the high boiling point component removal column C is preferably 3% or less, more preferably 2% or less, even more preferably 1.5% or less, 1% or less, 0.5% or less, 0.4% or less, 0.3% or less, 0.2% or less, 0.1% or less, or 0.05% or less, and particularly preferably 0.01% or less. The content of the high boiling point component in the charged liquid into the high boiling point component removal column C can be reduced by adjusting the distillation conditions of the desalting column B. For example, increasing the bottom ratio of the desalting column B can reduce the content of the high boiling point component in the charged liquid into the high boiling point component removal column C. Note that the content of the high boiling point component in the charged liquid into the high boiling point component removal column C is a total area proportion (area %) of peaks longer in retention time than the 1,3 BG peaks relative to the total peak area in the gas chromatographic analysis under the above conditions.

In the production method of the present disclosure, the content of acetaldehyde in charged liquid into the high boiling point component removal column C is, for example, 500 ppm or less, preferably 205 ppm or less (e.g., 200 ppm or less), more preferably 100 ppm or less, even more preferably 90 ppm or less, 80 ppm or less, 70 ppm or less, 60 ppm or less, 50 ppm or less, 40 ppm or less, 30 ppm or less, 20 ppm, or less or 10 ppm or less, and particularly preferably 5 ppm or less, and may be less than 2 ppm, or less than 1 ppm. The content of crotonaldehyde in the charged liquid into the high boiling point component removal column C is, for example, 200 ppm or less, preferably 110 ppm or less, more preferably 100 ppm or less, even more preferably 80 ppm or less, 70 ppm or less, 60 ppm or less, 50 ppm or less,

40 ppm or less, 30 ppm or less, 20 ppm or less, 10 ppm or less, 5 ppm or less, or 3 ppm or less, and particularly preferably 2 ppm or less, and may be less than 1 ppm. The acetaldehyde content and the crotonaldehyde content in the charged liquid into the high boiling point component removal column C can be reduced, for example, by providing a dealcoholization column (low boiling point component removal column) and a dehydration column upstream of the high boiling point component removal column C, and adjusting the distillation conditions of the dealcoholization column (low boiling point component removal column) and the dehydration column. For example, increasing the reflux ratio and the number of plates, and the distillation ratio of the dealcoholization column (low boiling point component removal column) and the dehydration column can reduce the acetaldehyde content and the crotonaldehyde content of the charged liquid into the high boiling point component removal column C. Note that the acetaldehyde content and the crotonaldehyde content of the charged liquid into the high boiling point component removal column C can be quantified by GC-MS analysis (gas mass spectrometry).

In the production method of the present disclosure, the content of water in charged liquid into the high boiling point component removal column C is, for example, 3 wt.% or less, preferably 2 wt.% or less, more preferably 1.2 wt.% or less, more preferably 1.1 wt.% or less, 1.0 wt.% or less, 0.95 wt.% or less, 0.9 wt.% or less, 0.8 wt.% or less, 0.7 wt.% or less, 0.6 wt.% or less, 0.5 wt.% or less, 0.4 wt.% or less, 0.3 wt.% or less, or 0.2 wt.% or less, and particularly preferably 0.1 wt.% or less. The content of water in the charged liquid into the high boiling point component removal column C can be reduced by adjusting the distillation conditions of the dehydration column A. For example, increasing the reflux ratio and the number of plates, and the distillation ratio of the dehydration column A can reduce the concentration of water in the charged liquid into the high boiling point component removal column C. Note that the water content of the charged liquid into the high boiling point component removal column CF can be quantified by the Karl Fischer water content measurement instrument. In the production method 2 of the present disclosure, the content of water in the charged liquid into the high boiling point component removal column C is 3 wt.% or less, preferably 2 wt.% or less, 1.2 wt.% or less, 0.4 wt.% or less, 0.3 wt.% or less, or 0.2 wt.% or less, and particularly preferably 0.1 wt.% or less, 0.05 wt.% or less, or 0.03 wt.% or less.

In the production method of the present disclosure, the content of the low boiling point component (excluding water) in the charged liquid into the high boiling point component removal column C is, for example, 1.8% or less, preferably 1.6% or less, more preferably 1.4% or less, more preferably 1.2% or less, 1.1% or less, 1% or less, 0.9% or less, 0.8% or less, 0.7% or less, 0.6% or less, 0.5% or less, 0.4% or less, 0.3% or less, or 0.2% or less, and particularly preferably 0.1% or less. The content of the low boiling point component (also referred to as "low boiling point substance" or "low boiling substance") excluding water in the charged liquid into the high boiling point component removal column C is a total area proportion (area %) of peaks of shorter in retention time than the peak of 1,3-butylene glycol relative to the total peak area in the gas chromatographic analysis under the above conditions. The content of the low boiling point component (excluding water) in the charged liquid into the high boiling point component removal column C can be reduced, for example, by providing a dealcoholization column (low boiling point component removal column) upstream of the high boiling point component removal column C, and adjusting the distillation conditions of the dealcoholization column (low boiling point component removal column). For example, increasing the reflux ratio and the number of plates, and the distillation ratio of the dealcoholization column (low boiling point component removal column) can reduce the content of the low boiling point component (excluding water) in the charged liquid into the high boiling point component removal column C.

In the production method of the present disclosure, the reflux ratio [(high boiling point component removal column reflux amount)/(high boiling point component removal column distilled amount (discharge amount to outside of distillation column))] in the high boiling point component removal column C is 0.03 or greater, preferably 0.05 or greater, more preferably 0.1 or greater, even more preferably 0.2 or greater, 0.3 or greater, 0.4 or greater, 0.5 or greater, 0.6 or greater, 0.7 or greater, 0.8 or greater, 0.9 or greater, 1 or greater, 1.2 or greater, 1.5 or greater, 2 or greater, 3 or greater, 4 or greater, 5 or greater, or 10 or greater, and particularly preferably 20 or greater, from the perspective of reducing the dry point of the 1,3-butylene glycol product. In particular, in the production method 2 of the present disclosure, the reflux ratio in the high boiling point component removal column C is preferably 0.1 or greater, more preferably 0.2 or greater, 0.3 or greater, 0.4 or greater, 0.5 or greater, 0.6 or greater, 0.7 or greater, 0.8 or greater, 0.9 or greater, 1 or greater, 1.2 or greater, 1.5 or greater, 2 or greater, 3 or greater, 4 or greater, 5 or greater, or 10 or greater, and particularly preferably 20 or greater. An upper limit of the reflux ratio is, for example, 100, preferably 50 from the point of energy cost. In a case where the theoretical number of plates of the high boiling point component removal column C is large, sufficient separation can be performed, even when the reflux ratio in the high boiling point component removal column C is about 1 or less.

In the production method of the present disclosure, the reflux ratio in the high boiling point component removal column C is within the range described above, and thus highly pure 1,3 BG having a very low content of the high boiling point component and a low dry point can be produced with a high recovery ratio.

In the production method of the present disclosure, the bottom ratio of the high boiling point component removal column C is, for example, less than 30 wt.%. Note that it is not limited to the value when the bottom of the high boiling

point component removal column is distilled in a further distillation column and a high boiling point substance is removed therefrom, and 1,3 BG is yielded as the product. When an extracted amount of the material containing a high boiling point substance to the outside of the system is reduced to less than 30 wt.% with respect to the charged amount into the high boiling point component removal column C, it is possible to obtain 1,3 BG in a high yield. Note that the bottom ratio refers to a proportion (wt.%) of an amount of liquid extracted from below the charging plate (for example, bottom of the column) of the high boiling point component removal column C with respect to a charged amount into the high boiling point component removal column C (also including an amount of the liquid recycled, when the liquid is recycled to the previous steps which will be described later). Note that when the liquid is recycled to the previous steps described below, the recovery ratio of 1,3 BG is improved as the discharge ratio to the outside is less.

The bottom ratio of the high boiling point component removal column C can also be preferably 25 wt.% or less, more preferably 20 wt.% or less, even more preferably 15 wt.% or less, 10 wt.% or less, 7 wt.% or less, 5 wt.% or less, 4 wt.% or less, 3 wt.% or less, or 2 wt.% or less, and can be 1 wt.% or less, from the perspective of improving the recovery ratio of 1,3 BG. In addition, the bottom ratio of the high boiling point component removal column is, for example, 0.01 wt.% or greater, preferably 0.1 wt.% or greater, 0.5 wt.% or greater, or 1 wt.% or greater, more preferably 2 wt.% or greater, 3 wt.% or greater, 4 wt.% or greater, 5 wt.% or greater, 6 wt.% or greater, 7 wt.% or greater, 8 wt.% or greater, 9 wt.% or greater, 10 wt.% or greater, or 15 wt.% or greater, and particularly preferably 20 wt.% or greater, from the perspective of reducing the dry point of the 1,3-butylene glycol product.

At least a portion of the liquid (hereinafter, sometimes referred to as "bottom") in which the high boiling point component is concentrated, the high boiling point component having been extracted from below the charging plate of the high boiling point component removal column C, may be recycled to step prior to the high boiling point component removal (dashed arrow illustrated in the lower part of the high boiling point component removal column C in FIG. 1). The recovery ratio of 1,3 BG can be improved by recycling at least a portion of the bottom to the step prior to the high boiling point component removal. Note that, in the present specification, the recovery ratio of 1,3 BG in the high boiling point component removal column C is a value (%) determined by the following formula.

$$\{1 - [\text{GC area \% of 1,3 BG in bottom} \times (\text{bottom amount (part)} - \text{amount (part) of bottom recycled}]/(\text{GC area \% of 1,3 BG in charged liquid} \times \text{charged amount (part)}\} \times 100$$

Note that the low boiling point substance and the high boiling point substance may be hydrolyzed by water to produce 1,3 BG, while the high boiling point substance may be formed by polymerization of 1,3 BG. Further, trace impurities may be formed or disappear. Thus, the mass balance in the high boiling point component removal column may not always be retained.

The recovery ratio of 1,3 BG in the high boiling point component removal column C is, for example, greater than 80%, preferably 85% or greater, more preferably 90% or greater, even more preferably 95% or greater, and particularly preferably 99% or greater.

Examples of the step prior to the high boiling point component removal include acetaldehyde polymerization (aldol condensation of acetaldehyde), reaction (hydrogenation), dealcoholization (low boiling point component removal), dehydration, and desalting. The bottom is preferably recycled to the acetaldehyde polymerization (aldol condensation of acetaldehyde), among these steps, since 1,3 BG is produced by hydrolysis of the high boiling point substance. In addition, the hydrogen addition reduction may produce 1,3 BG, and the bottom may be recycled to the hydrogen addition from that viewpoint.

The amount of the bottom recycled to the step prior to the high boiling point component removal can be appropriately selected within a range of the amount of the bottom. The amount of the bottom recycled to the step prior to the high boiling point component removal is, for example, less than 30 wt.%, preferably 25 wt.% or less with respect to the charged amount into the high boiling point component removal column C. Note that the amount of the bottom recycled may be 20 wt.% or less, 15 wt.% or less, 10 wt.% or less, 7 wt.% or less, 5 wt.% or less, 4 wt.% or less, 3 wt.% or less, 2 wt.% or less, or 1 wt.% or less with respect to the charged amount into the high boiling point component removal column C. In addition, from the perspective of improving the recovery ratio of 1,3 BG in the high boiling point component removal column and the yield throughout the 1,3 BG production process, the amount of the bottom recycled to the step prior to the high boiling point component removal is, for example, 0.01 wt.% or greater, preferably 0.1 wt.% or greater, more preferably 2 wt.% or greater, 3 wt.% or greater, 4 wt.% or greater, 5 wt.% or greater, 7 wt.% or greater, or 10 wt.% or greater, and particularly preferably 20 wt.% or greater relative to the amount of charge into the high boiling point component removal column C. Note that, in a case where the amount of the bottom is minimized as much as possible, 1,3 BG can be recovered in a high yield, even without recycle to the previous steps.

The crude 1,3-butylene glycol stream taken out from above the charging plate of the high boiling point component

removal column C can be a 1,3-butylene glycol product as it is in the production method 2 of the present disclosure. Alternatively, the crude 1,3-butylene glycol stream taken out from above the charging plate of the high boiling point component removal column C is subjected to alkaline treatment in the alkaline reactor D described below, and evaporated (or distilled) with the dealkalization column E, and the distillate at the top of the dealkalization column E can be a 1,3-butylene glycol product.

According to the production method 2 of the present disclosure, the content of acetaldehyde and the content of crotonaldehyde in the charged liquid into the high boiling point component removal column are within the specific ranges, and the reflux ratio of the high boiling point component removal column is within the specific range. Therefore, it is possible to industrially efficiently yield high-purity 1,3-butylene glycol that is colorless and odorless (or almost colorless and odorless), unlikely to cause or increase coloration and odor over time, and, besides, unlikely to cause an acid concentration increase over time even in a state containing water.

In the production method 1 of the present disclosure, the crude 1,3-butylene glycol stream taken out from above the charging plate of the high boiling point component removal column C is fed, for example, to the alkaline reactor (e.g., a flow-through tubular reactor) D and is treated with a base (treated with alkali). The base treatment can decompose by-products contained in the crude 1,3-butylene glycol. The base is added in the alkaline reactor D or its upstream piping or the like. The base is added in an amount of, for example, from 0.05 to 10 wt.%, preferably from 0.1 to 1.0 wt.% relative to the crude 1,3-butylene glycol stream subjected to the alkaline treatment. With the added amount of the base exceeding 10 wt.%, the base would precipitate in the distillation column, piping, or the like, and this may cause blockage. In addition, the decomposition reaction of a high boiling point compound would occur, and by-products may be formed on the contrary. With the added amount of the base of less than 0.05 wt.%, the effect of decomposing by-products is reduced.

The base added in the alkaline reactor D or its upstream piping is not particularly limited but is, for example, preferably an alkali metal compound. Examples of the alkali metal compound include sodium hydroxide, potassium hydroxide, sodium (bi)carbonate, and potassium (bi)carbonate. A basic ion exchange resin can also be used as the base. The base is preferably sodium hydroxide or potassium hydroxide from the perspective of reducing the by-products contained in the final 1,3-butylene glycol product. The base may be added as is in the solid form but is preferably added in an aqueous solution to facilitate operation and contact with a solution to be treated. One of the bases described above may be used alone, or two or more may be used simultaneously.

The reaction temperature in the alkaline reactor D is not particularly limited but is, for example, preferably from 90 to 140°C and more preferably from 110 to 130°C. The reaction at a reaction temperature lower than 90°C would require long reaction residence time and thus require a reactor with a large volume and make the process uneconomical. The reaction at a reaction temperature exceeding 140°C would increase coloration in the final 1,3-butylene glycol product. The reaction residence time is, for example, preferably from 5 to 120 minutes and more preferably from 10 to 30 minutes. A reaction residence time shorter than 5 minutes may cause an insufficient reaction and deteriorate the quality of the final 1,3-butylene glycol product. A reaction residence time exceeding 120 minutes would require a large reactor and increase the cost of equipment, and thus would be disadvantageous from the economic point of view.

After exiting the alkaline reactor D, the reaction crude liquid stream is fed to the dealkalization column (e.g., thin film evaporator) E as necessary, and the base and the like are removed from the bottom of the column by evaporation. On the other hand, from the top of the dealkalization column E, a crude 1,3-butylene glycol stream after the removal of a base (which is used as the 1,3-butylene glycol product, in the production method 2 of the present disclosure) is produced. The evaporator used for the dealkalization column E is suitably a natural downward flow thin-film evaporator or a forced-stirring thin-film evaporator with a short residence time for the purpose of reducing the thermal history to the process fluid. A demister may be installed in a space above the charging position of the dealkalization column (e.g., thin film evaporator) E, and droplets of a base or the like may be removed. This makes it possible to prevent the base and the like from being mixed into the 1,3-butylene glycol product.

Evaporation is performed in the evaporator used for the dealkalization column E, for example, under a reduced pressure at the top of the column of 20 kPa or less (absolute pressure), preferably from 0.5 to 10 kPa (absolute pressure). The temperature of the evaporator is, for example, preferably from 90 to 120°C. The crude 1,3-butylene glycol stream containing a low boiling point substance distilled from the top of the column is fed to the product distillation column (product column) F. Note that, as described above, in the production method 2 of the present disclosure, the distillate (corresponding to E-1) from the top of the dealkalization column E can be a 1,3-butylene glycol product.

Note that the alkaline reactor D and the dealkalization column E may be installed between the desalting column B and the high boiling point component removal column C, between the dehydration column A and desalting column B (in this case, the desalting column may also serve as a dealkalization column), or before the dehydration column A. In addition, without the alkaline reactor D or the dealkalization column E being provided, the alkaline treatment can be performed by charging the base into a high boiling point component removal column charging line or into a dehydration column charging line, or adding the base to the reaction solution after the hydrogenation [and then charging the base into the dealcoholization column (low boiling point component removal column)].

In the production method 1 of the present disclosure, in the product column F for use in the product distillation,

a charged liquid having a concentration of 1,3-butylene glycol of, for example, 97.6 area % or greater determined by GC analysis is distilled, a liquid with a concentrated low boiling point component is distilled from above the charging plate (corresponding to "X-6" in FIG. 1), and 1,3-butylene glycol is extracted from below the charging plate (corresponding to "Y" in FIG. 1). The extracted 1,3-butylene glycol can be a 1,3-butylene glycol product.

The product column F can be, for example, a perforated-plate column, a bubble column, or the like, but is more preferably a packed column with a low pressure loss, filled with Sulzer Packing, Melapack (trade names of Sumitomo Heavy Industries, Ltd.). This is because 1,3-butylene glycol and trace impurities would be thermally decomposed at a high temperature (e.g., 150°C or greater) and form a low boiling point substance, which is a coloring component, and thus the distillation temperature is to be lowered. In addition, this is also because a long thermal history (residence time) for 1,3-butylene glycol would also have a similar effect. Thus, the reboiler employed is preferably one with a short residence time of the process side fluid, for example, a thin-film evaporator, such as a natural downward flow thin-film evaporator or a forced-stirring thin-film evaporator.

A theoretical number of plates of the product column F is, for example, from 1 to 100 plates, preferably from 2 to 90 plates, from 3 to 80 plates, from 4 to 70 plates, from 5 to 60 plates, from 8 to 50 plates, or from 10 to 40 plates, and more preferably from 15 to 30 plates. A feed position for the charged liquid is, for example, from 10 to 90%, preferably from 20 to 80%, and more preferably from 30 to 70%, and even more preferably from 40 to 60% of a height of the column downward from the top of the column. In the distillation in the product distillation column F, a pressure (absolute pressure) at the top of the column is, for example, from 20 kPa or less, preferably from 0.1 to 10 kPa, more preferably from 0.3 to 8 kPa, and even more preferably from 0.5 to 5 kPa.

In FIG. 1, in charging to the product column F, the column top vapor from the dealkalization column E is condensed in the condenser E-1, and the resulting condensed liquid is fed, but the column top vapor from the dealkalization column E may be directly fed to the product column F.

The concentration of 1,3-butylene glycol in the charged liquid (1,3-butylene glycol charged liquid) into the product column F is 97.6% or greater, preferably 97.8% or greater, more preferably 98% or greater, even more preferably 98.2% or greater (e.g., 98.4% or greater, 98.6% or greater, or 98.8% or greater), and particularly preferably 99% or greater (e.g., 99.1% or greater, 9.2% or greater, 99.3% or greater, 99.4% or greater, 99.5% or greater, 99.6% or greater, 99.7% or greater, 99.8% or greater, or 99.9% or greater).

The concentration of 1,3-butylene glycol in the charged liquid into the product column F can be improved, for example, by adjusting the distillation conditions of the dehydration column A; providing a dealcoholization column (low boiling point component removal column) before the dehydration column A, and adjusting the distillation conditions thereof; or adjusting the distillation conditions of the high boiling point component removal column C. For example, it is possible to increase the purity of 1,3-butylene glycol in charged liquid into the product column F by increasing the reflux ratio of the dealcoholization column (low boiling point component removal column), the dehydration column A, and/or the high boiling point component removal column C or increasing the number of plates.

Note that the concentration of 1,3-butylene glycol in the charged liquid into the product column F is an area proportion (area %) of the peak of 1,3-butylene glycol relative to the total peak area in the gas chromatographic analysis of the following conditions.

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

In the production method 1 of the present disclosure, the content of acetaldehyde in charged liquid into the product column F is 500 ppm or less, preferably 205 ppm or less (e.g., 200 ppm or less), more preferably 150 ppm or less, even more preferably 120 ppm or less, 100 ppm or less, 90 ppm or less, 80 ppm or less, 70 ppm or less, 60 ppm or less, 50 ppm or less, 40 ppm or less, 30 ppm or less, 20 ppm or less, or 10 ppm or less, and particularly preferably 5 ppm or less, and may be less than 2 ppm. The content of crotonaldehyde in the charged liquid into the product column F is 200 ppm or less, preferably 150 ppm or less, more preferably 130 ppm or less, even more preferably 110 ppm or less, 100 ppm or less, 80 ppm or less, 70 ppm or less, 60 ppm or less, 50 ppm or less, 40 ppm or less, 30 ppm or less, 20 ppm or less, 10 ppm or less, 5 ppm or less, or 3 ppm or less, and particularly preferably 2 ppm or less, and may be less than 1 ppm. The acetaldehyde content and the crotonaldehyde content in the charged liquid into the product column F can be reduced, for example, by providing a dealcoholization column (low boiling point component removal column)

and a dehydration column upstream of the product column F, and adjusting the distillation conditions of the dealcoholization column (low boiling point component removal column) and the dehydration column. For example, increasing the reflux ratio and the number of plates, and the distillation ratio of the dealcoholization column (low boiling point component removal column) and the dehydration column can reduce the acetaldehyde content and the crotonaldehyde content of the charged liquid into the product column F. In addition, the acetaldehyde content and the crotonaldehyde content in the charged liquid into the product column F can be reduced by increasing the reaction temperature, increasing the residence time, or increasing the added amount of the base, in the alkaline reaction. Note that the acetaldehyde content and the crotonaldehyde content of the charged liquid into the product column F can be quantified by GC-MS analysis (gas mass spectrometry).

In the production method 1 of the present disclosure, a content of water in charged liquid into the product column F is 0.7 wt.% or less, preferably 0.6 wt.% or less, 0.5 wt.% or less, 0.4 wt.% or less, 0.3 wt.% or less, or 0.2 wt.% or less, and particularly preferably 0.1 wt.% or less. The content of water in the charged liquid into the product column F can be reduced by adjusting the distillation conditions of the dehydration column A. For example, increasing the reflux ratio and the number of plates, and the distillation ratio of the dehydration column A can reduce the concentration of water in the charged liquid into the product column F. Note that the water content of the charged liquid into the product column F can be quantified by the Karl Fischer water content measurement instrument.

The content of the low boiling point component (excluding water) in the charged liquid into the product column F is, for example, 1.8% or less, preferably 1.6% or less, more preferably 1.4% or less, more preferably 1.2% or less, 1.1% or less, 1% or less, 0.9% or less, 0.8% or less, 0.7% or less, 0.6% or less, 0.5% or less, 0.4% or less, 0.3% or less, or 0.2% or less, and particularly preferably 0.1% or less. The content of the low boiling point component (also referred to as "low boiling point substance") excluding water in the charged liquid into the product column F is a total area proportion (area %) of peaks of shorter in retention time than the peak of 1,3-butylene glycol relative to the total peak area in the gas chromatographic analysis under the above conditions. The content of the low boiling point component (excluding water) in the charged liquid into the product column F can be reduced, for example, by providing a dealcoholization column (low boiling point component removal column) upstream of the product column F, and adjusting the distillation conditions of the dealcoholization column (low boiling point component removal column). For example, increasing the reflux ratio and the number of plates, and the distillation ratio of the dealcoholization column (low boiling point component removal column) can reduce the content of the low boiling point component (excluding water) in the charged liquid into the product column F.

The content of the high boiling point component (excluding water) in the charged liquid into the product column F is, for example, 1.8% or less, preferably 1.6% or less, more preferably 1.4% or less, more preferably 1.2% or less, 1.1% or less, 1% or less, 0.9% or less, 0.8% or less, 0.7% or less, 0.6% or less, 0.5% or less, 0.4% or less, 0.3% or less, or 0.2% or less, and particularly preferably 0.1% or less. The content of the high boiling point component (also referred to as "high boiling point substance" or "high boiling substance") excluding water in the charged liquid into the product column F is a total area proportion (area %) of peaks of longer in retention time than the peak of 1,3-butylene glycol relative to the total peak area in the gas chromatographic analysis under the above conditions. The content of the high boiling point component (excluding water) in the charged liquid into the product column F can be reduced, for example, by adjusting the distillation conditions of the high boiling point component removal column. For example, increasing the reflux ratio, the number of plates, or the bottom ratio of the high boiling point component removal column can reduce the content of the high boiling point component (excluding water) in the charged liquid into the product column F.

In the production method 1 of the present disclosure, the reflux ratio [(product column reflux amount)/(product column distilled amount (discharge amount to outside of distillation column))] in the product column F is 0.3 or greater, preferably 0.4 or greater, more preferably 0.5 or greater, 1 or greater, 2 or greater, 3 or greater, 4 or greater, 5 or greater, 6 or greater, 7 or greater, 8 or greater, 9 or greater, 10 or greater, 20 or greater, or 50 or greater, and particularly preferably 400 or greater (for example, 500 or greater), from the perspective of increasing the initial boiling point of the 1,3-butylene glycol product. An upper limit of the reflux ratio of the product column F is, for example, 700 or 1000 from the perspective of energy cost.

In the production method 1 of the present disclosure, the distillation ratio of the product column F is, for example, less than 30 wt.%, 29 wt.% or less, more preferably 28 wt.% or less, even more preferably 27 wt.% or less, 26 wt.% or less, 25 wt.% or less, 24 wt.% or less, 23 wt.% or less, 22 wt.% or less, 21 wt.% or less, 20 wt.% or less, 19 wt.% or less, 18 wt.% or less, 17 wt.% or less, 16 wt.% or less, 15 wt.% or less, 12 wt.% or less, 10 wt.% or less, 8 wt.% or less, 5 wt.% or less, 3 wt.% or less, 2 wt.% or less, 1 wt.% or less, 0.8 wt.% or less, or 0.6 wt.% or less, and particularly preferably 0.4 wt.% or less, from the perspective of improving the recovery ratio of 1,3-butylene glycol. Note that the distillation ratio refers to a proportion (wt.%) of an amount of liquid extracted from above the charging plate of the product column F (for example, the top of the column) to the outside of the distillation column (when recycled to the previous step which will be described below, including also the amount of liquid recycled) with respect to a charged amount into the product column F.

At least a portion of the liquid (hereinafter, sometimes referred to as "distillate") in which the low boiling component is concentrated, which is extracted from above the charging plate of the product column F, may be recycled to the step prior to the product distillation (dashed arrow illustrated on the right side of the product column F in FIG. 1). The recovery ratio of 1,3-butylene glycol can be improved by recycling at least a portion of the distillate to the step prior to the product distillation.

Examples of the step prior to the product distillation include dehydration and dealcoholization (low boiling point component removal). Note that the dealcoholization (low boiling point component removal) is preferably provided before the dehydration.

The amount of the distillate recycled to the step prior to the product distillation can be appropriately selected within the range of the amount of distillate. The amount of the distillate recycle to the step prior to the product distillation is less than 30 wt.%, for example, with respect to the charged amount into the product column F. Also, from the perspective of improving the 1,3 BG recovery ratio in the product column and the yield throughout the process, the amount of the distillate recycled to the step prior to the product distillation is, for example, 0.01 wt.% or greater, preferably 0.05 wt.% or greater, more preferably 0.1 wt.% or greater, 0.5 wt.% or greater, 1 wt.% or greater, 1.5 wt.% or greater, 2 wt.% or greater, 3 wt.% or greater, 4 wt.% or greater, 5 wt.% or greater, 7 wt.% or greater, or 10 wt.% or greater, and particularly preferably 20 wt.% or greater with respect to the charged amount into the product column F.

In the production method 1 of the present disclosure, the content of acetaldehyde and the content of crotonaldehyde in the charged liquid into product column F are within the specific ranges, and the reflux ratio of the product column F is within the specific range. Therefore, it is possible to industrially efficiently produce high-purity 1,3-butylene glycol that is colorless and odorless (or almost colorless and odorless), unlikely to cause or increase coloration and odor over time, and, besides, unlikely to cause an acid concentration increase over time even in a state containing water.

The recovery ratio of 1,3 BG in the product column F is, for example, greater than 80%, preferably 85% or greater, more preferably 90% or greater, even more preferably 95% or greater, and particularly preferably 99% or greater.

Note that, in the present specification, the recovery ratio of 1,3 BG in the product column F is a value (%) determined by the following formula.

$$\{1 - [\text{GC area \% of 1,3 BG in distillate} \times (\text{distilled amount (part)} - \text{amount (part) of distillate recycled}]/(\text{GC area \% of 1,3 BG in charged liquid} \times \text{charged amount (part)}\} \times 100$$

Note that, as described above, the low boiling point substance and the high boiling point substance may be hydrolyzed by water to produce 1,3 BG, while the high boiling point substance may be formed by polymerization of 1,3 BG. Thus, the mass balance in the product column may not always be retained.

Each aspect disclosed in the present specification can be combined with any other feature disclosed herein. Note that each of the configurations, combinations thereof, and the like in each of the embodiments are an example, and various additions, omissions, and other changes may be made as appropriate without departing from the spirit of the present disclosure. The present disclosure is not limited by the embodiments and is limited only by the claims.

## Examples

Hereinafter, the present disclosure will be specifically described with reference to examples. "Parts" used in the examples means "parts by weight" unless otherwise specified. Gas chromatographic analysis (GC analysis), initial boiling point measurement, and water content measurement were performed according to methods which will be described below.

### Example 1

The method of producing 1,3-butylene glycol will be described using FIG. 1.

Relative to 100 parts of an acetaldol solution containing 30 wt.% of water (mixed solution of 69 parts of acetaldol and 29 parts of water, containing a total of 2 parts of low boiling and high boiling impurities, Na salt: less than 0.1 parts) as a raw material, 10 parts of hydrogen were charged in a reactor for liquid-phase hydrogen reduction, and 15 parts of Raney nickel were added as a catalyst. The reactor was kept at 120°C and 10 MPa (gauge pressure), and liquid-phase hydrogen reduction was performed. After the catalyst was separated, the liquid after the reaction was neutralized with sodium hydroxide, and crude 1,3-butylene glycol (1) containing water was produced.

Note that the acetaldol solution containing 30 wt.% of water used as the raw material was produced by stirring acetaldehyde and water in the presence of 100 ppm by weight NaOH at 30°C at a residence time of 10 hours and

dimerizing the acetaldehyde [acetaldehyde polymerization (aldol condensation of acetaldehyde)].

The crude 1,3-butylene glycol (1) (corresponding to "X-1" in FIG. 1) was charged in the dehydration column A. The concentration of 1,3-butylene glycol in the charged liquid into the dehydration column A was 56 wt.%, the water concentration was 40 wt.%, the content of acetaldehyde (AD) was 130 ppm, the content of crotonaldehyde (CR) was 89 ppm, the total area ratio of impurity peaks shorter in retention time (RT) than 1,3-butylene glycol in GC analysis which will be described later was 3%, and the total area ratio of impurity peaks longer in retention time than 1,3-butylene glycol was 1%. In the dehydration column A, the charged liquid containing 1,3-butylene glycol was distilled under conditions of a pressure at the top of 10 kPa (absolute pressure) and a reflux ratio of 1, water was extracted from the top of the column, and 43 parts (distilled amount) relative to 100 parts of the charged liquid amount was discharged and removed to the outside of the system (corresponding to "X-2" in FIG. 1). From the bottom of the column, crude 1,3-butylene glycol (2) having a 1,3-butylene glycol concentration of 96.9 GC area %, a water content of 0.9 wt.%, a total area ratio of impurity peaks shorter in retention time than 1,3-butylene glycol in the GC analysis which will be described later of 0.8%, a total area ratio of peaks longer in retention time than peak of 1,3-butylene glycols of 2.3%, an acetaldehyde content of 18 ppm, and a crotonaldehyde content of 17 ppm was produced.

The crude 1,3-butylene glycol (2) was then charged in the desalting column B. In the desalting column B, a salt, a high boiling point substance, and a portion of 1,3-butylene glycol were discharged as the evaporation residue from the bottom of the column (corresponding to "X-3" in FIG. 1). The discharge amount of the evaporation residue was 5 parts relative to 100 parts of the charged liquid amount. On the other hand, from the top of the column, crude 1,3-butylene glycol (3) containing 1,3-butylene glycol, a low boiling point substance, and a portion of a high boiling point substance was produced.

The crude 1,3-butylene glycol (3) was then charged in the high boiling point component removal column C. In the high boiling point component removal column C, distillation was performed under the conditions of a pressure at the top of the column of 5 kPa (absolute pressure) and a reflux ratio of 0.05, and a high boiling point substance and a portion of 1,3-butylene glycol were discharged from the bottom of the column (corresponding to "X-4" in FIG. 1). The discharge amount from the bottom of the column was 20 parts relative to 100 parts of the charged liquid amount. On the other hand, 80 parts of crude 1,3-butylene glycol (4) containing a low boiling point substance was produced, as a distillate, from the top of the column.

The crude 1,3-butylene glycol (4) was then charged in the alkaline reactor D. At this time, a 20 wt.% sodium hydroxide aqueous solution was added to give a concentration of sodium hydroxide of 0.1 wt.% relative to the charged liquid. The reaction temperature was maintained at 120°C in the alkaline reactor D, and a reaction was performed at a residence time of 20 minutes.

A reaction crude liquid exiting the alkaline reactor D was then charged in the dealkalization column E. In the dealkalization column E, sodium hydroxide, a high boiling point substance, and a portion of 1,3-butylene glycol were discharged from the bottom of the column (corresponding to "X-5" in FIG. 1). The discharge amount from the bottom of the column was 10 parts relative to 100 parts of the charged liquid amount. On the other hand, from the top of the column 90 parts of crude 1,3-butylene glycol (5) containing 1,3-butylene glycol and a low boiling point substance were produced. The crude 1,3-butylene glycol (5) containing 1,3-butylene glycol and a low boiling point substance was measured for water content, and subjected to GC analysis and GC-MS analysis. As a result, the water concentration was 1 wt.%, the area ratio of 1,3-butylene glycol was 99%, the total area ratio of impurity peaks shorter in retention time than 1,3-butylene glycol was 0.4%, the total area ratio of impurity peaks longer in retention time than 1,3-butylene glycol was 0.6%, the content of acetaldehyde was 20 ppm, and the content of crotonaldehyde was 9 ppm.

The crude 1,3-butylene glycol (5) was then charged in the product column F. In the product column F, 10 parts of the low boiling point substance and a portion of 1,3-butylene glycol relative to 100 parts of the charged liquid amount were distilled off from the top of the column (corresponding to "X-6" in FIG. 1), and the entire amount was discharged to the outside of the system. The operation was performed at a reflux ratio (reflux amount/distilled amount) of 0.5 at that time, and 90 parts of a 1,3-butylene glycol product was produced from the bottom of the column (distilled amount: 10 parts) (corresponding to "Y" in FIG. 1).

The produced 1,3-butylene glycol product was measured for initial boiling point and water content, and subjected to GC analysis and GC-MS analysis. As a result, the 1,3-butylene glycol product had an initial boiling point of 203.3°C, a dry point of 209°C, a water concentration of 0.2 wt.%, an area ratio of 1,3-butylene glycol of 99.2%, a total area ratio of impurity peaks shorter in retention time than 1,3-butylene glycol of 0.08%, a total area ratio of impurity peaks longer in retention time than 1,3-butylene glycol of 0.7%, an acetaldehyde content of 1.5 ppm, and a crotonaldehyde content of 0.9 ppm. The potassium permanganate test value was 35 minutes. The 1,3-butylene glycol recovery ratio in the product column F was 90%.

Example 2

The same operation as in Example 1 was performed except that the reflux ratio of the dehydration column A

was changed to 50. A 1,3-butylene glycol product was produced from the bottom of the product column F. Note that, due to the changes in conditions employed in the dehydration column A, the dehydration column bottom composition changed, and the charged liquid compositions in the high boiling point component removal column C and the product column F changed. As a result, the product qualities changed.

The produced 1,3-butylene glycol product was measured for initial boiling point and water content, and subjected to GC analysis and GC-MS analysis. As a result, the 1,3-butylene glycol product had an initial boiling point of 206.7°C, a dry point of 208.9°C, a water concentration of 0.1 wt.%, an area ratio of 1,3-butylene glycol of 99.3%, a total area ratio of impurity peaks shorter in retention time than 1,3-butylene glycol of 0.05%, a total area ratio of impurity peaks longer in retention time than 1,3-butylene glycol of 0.7%, an acetaldehyde content of 0.7 ppm, and a crotonaldehyde content of 0.7 ppm. The potassium permanganate test value was 45 minutes. The 1,3-butylene glycol recovery ratio in the product column F was 90%.

Examples 3 to 27

Under the conditions shown in Table 1 and Table 2, the dehydration column A, the high boiling point component removal column C, and the product column F were operated. In Examples 4 to 22 and 24 to 27, the entire amount of the distillate from the product column F was recycled into hydrogen reduction reactor. In Example 23, the product column F was not used, and a distillate at the top of the dealkalization column E (demister was installed in a space above the charging position) was a 1,3-butylene glycol product. At this time, the concentration of the sodium hydroxide aqueous solution in the alkaline reactor D was set to 1.5 times, and the added amount of the sodium hydroxide aqueous solution was set to the half of the amount in Example 1. Thus, the increase in water content due to alkaline treatment was avoided as much as possible. Note that, when the alkali concentration of the sodium hydroxide aqueous solution is too high and crystals precipitate. Therefore, heating is preferably performed to 40°C or greater. In Table 2, the column "Bottom from product column F" of Example 23 shows the composition and physical properties of the distillate at the top of the dealkalization column E. Note that, in Example 16, 8 parts of 10 parts of the bottom from the high boiling point component removal column was recycled into the hydrogenation, and 2 parts thereof was discharged to the outside of the system. In addition, in Example 25, the pressure of the hydrogen addition reaction was reduced to 7 MPaG (gauge pressure). Therefore, the acetaldehyde content and crotonaldehyde content of the charged liquid into the dehydration column are high. In Example 26, the reflux ratio of the dehydration column was reduced to 0.3, and the purity of 1,3-butylene glycol in the charged liquid into the product column was reduced, and the reflux ratio of the product column was increased to 20. In Example 27, the pressure of the hydrogen addition reaction was increased to 40 MPaG (gauge pressure) (the remaining conditions are the same as in Example 18).

Comparative Example 1

Eighty (80) parts of a 1,3-butylene glycol product was produced from the bottom of the product column F by the same method as in Example 1 except that the reflux ratio of the dehydration column A was changed to 0.5, that the distilled amount was changed to 42 parts, that the reflux ratio of the high boiling point component removal column C was changed to 0.02, that the reflux ratio of the product column F was changed to 0.05, and that the distilled amount was changed to 20 parts. The produced 1,3-butylene glycol product had an initial boiling point of 193.2°C, a dry point of 210.3°C, a water concentration of 0.6 wt.%, an area ratio of 1,3-butylene glycol of 98.3%, a total area ratio of impurity peaks shorter in retention time than 1,3-butylene glycol of 0.2%, a total area ratio of impurity peaks longer in retention time than 1,3-butylene glycol of 1.5%, an acetaldehyde content of 5 ppm, and a crotonaldehyde content of 4 ppm. The potassium permanganate test value was 0 minutes. The 1,3-butylene glycol recovery ratio in the product column F was 80%.

Comparative Example 2

Eighty (80) parts of a 1,3-butylene glycol product was produced from the bottom of the product column F by the same method as in Example 1 except that the charged composition in the dehydration column A was changed, the reflux ratio and the distilled amount were changed to 0.5 and 32 parts, respectively, that the reflux ratio of the high boiling point component removal column C was changed to 0.02, that the reflux ratio of the product column F was changed to 0.05, and that the distilled amount was changed to 20 parts. The produced 1,3-butylene glycol product had an initial boiling point of 199.0°C, a dry point of 210.1°C, a water concentration of 0.4 wt.%, an area ratio of 1,3-butylene glycol of 98.5%, a total area ratio of impurity peaks shorter in retention time than 1,3-butylene glycol of 0.1%, a total area ratio of impurity peaks longer in retention time than 1,3-butylene glycol of 1.4%, an acetaldehyde content of 4 ppm, and a crotonaldehyde content of 2 ppm. The potassium permanganate test value was 5 minutes. The 1,3-butylene glycol recovery ratio in the product column F was 80%.

Comparative Example 3

Seventy (70) parts of a 1,3-butylene glycol product was produced from the bottom of the product column F by the same method as in Example 1 except that the charged composition in the dehydration column A was changed, the reflux ratio and the distilled amount were changed to 0.5 and 32 parts, respectively, that the reflux ratio of the high boiling point component removal column C was changed to 0.02, that the reflux ratio of the product column F was changed to 0.05, and that the distilled amount was changed to 30 parts. The produced 1,3-butylene glycol product had an initial boiling point of 203.0°C, a dry point of 210.2°C, a water concentration of 0.2 wt.%, an area ratio of 1,3-butylene glycol of 98.4%, a total area ratio of impurity peaks shorter in retention time than 1,3-butylene glycol of 0.1%, a total area ratio of impurity peaks longer in retention time than 1,3-butylene glycol of 1.5%, an acetaldehyde content of 2 ppm, and a crotonaldehyde content of 1.3 ppm. The potassium permanganate test value was 30 minutes. The 1,3-butylene glycol recovery ratio in the product column F was 70%.

Comparative Example 4

Eighty (80) parts of a 1,3-butylene glycol product was produced from the bottom of the product column F by the same method as in Example 1 except that the charged composition in the dehydration column A was changed, the distilled amount was changed to 23 parts, that the reflux ratio of the high boiling point component removal column C was changed to 0.02, that the reflux ratio of the product column F was changed to 0.1, and that the distilled amount was changed to 20 parts. The produced 1,3-butylene glycol product had an initial boiling point of 203.1°C, a dry point of 209.5°C, a water concentration of 0.2 wt.%, an area ratio of 1,3-butylene glycol of 98.8%, a total area ratio of impurity peaks shorter in retention time than 1,3-butylene glycol of 0.1%, a total area ratio of impurity peaks longer in retention time than 1,3-butylene glycol of 1.1%, an acetaldehyde content of 2 ppm, and a crotonaldehyde content of 1.3 ppm. The potassium permanganate test value was 30 minutes. The 1,3-butylene glycol recovery ratio in the product column F was 80%.

Gas chromatographic analysis

A gas chromatographic analysis of the target 1,3-butylene glycol product was performed under the conditions below. A chromatogram of the gas chromatographic analysis of the 1,3-butylene glycol product in Example 1 is shown in FIG. 2. A chromatogram of the gas chromatographic analysis of the 1,3-butylene glycol product in Example 12 is shown in FIG. 3. In addition, a chromatogram of the gas chromatographic analysis of the 1,3-butylene glycol product in Comparative Example 2 is shown in FIG. 4.

The conditions for the gas chromatographic analysis are as follows:

Analytical Instrument: Shimadzu GC 2010
Analytical Column: column with dimethylpolysiloxane as a stationary phase (a length of 30 m, an inner diameter of 0.25 mm, and a film thickness of 1.0 μm) (Agilent J&W GC column - DB-1, available from Agilent Technologies Japan, Ltd.)
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction and Temperature: split sample introduction, 250°C
Gas Flow Rate of Split and Carrier Gas: 23 mL/min, helium
Column Gas Flow Rate and Carrier Gas: 1 mL/min, helium
Detector and Temperature: a flame ionization detector (FID), 280°C.
Injection Sample: 0.2 μL of a 80 wt.% 1,3-butylene glycol product aqueous solution

Measurement of initial boiling point and dry point

Measurement was made according to the test method specified in the normal pressure distillation test method of JIS K2254 "Petroleum products - distillation test method".

Measurement of water content

Measurement was made using a Karl Fischer water content measurement instrument.

GC-MS analysis

Analytical Instrument: Agilent 6890A-GC/5973A-MSD
Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Ion source temperature: EI 230°C, CI 250°C
Q Pole temperature: 150°C
Sample: subjected to analysis as it was

Potassium permanganate test

In the present specification, the potassium permanganate test value (PMT) is a value measured in accordance with the visual colorimetric procedure of JIS K1351 (1993).

Coloration-over-time test 1 (coloration test 1)

The target 1,3-butylene glycol product was placed in a wide-mouth bottle, the bottle was stoppered tightly and kept in a constant temperature oven set at 180°C for 3 hours. A Hazen color number (APHA) of the 1,3-butylene glycol product after the 1,3-butylene glycol product had been kept at 180°C for 3 hours was measured using a color difference meter ("ZE6000" available from Nippon Denshoku Industries Co., Ltd.) and a quartz cell with an optical path length of 10 mm. The Hazen color number (APHA) of the 1,3-butylene glycol product prior to the test was similarly measured. Furthermore, each impurity content of the 1,3-butylene glycol product before and after the coloration-over-time test 1 was measured by GC-MS analysis. Also, the initial boiling point, dry point, potassium permanganate test value, odor and acid content of the 1,3-butylene glycol product before and after the coloration-over-time test 1 were measured.

Coloration-over-time test 2 (coloration test 2)

The target 1,3-butylene glycol product was placed in a wide-mouth bottle, and the bottle was stoppered tightly and kept in a constant temperature oven set at 100°C for 75 days. A Hazen color number (APHA) of the 1,3-butylene glycol product after the 1,3-butylene glycol product had been kept at 100°C for 75 days was measured using a color difference meter ("ZE6000" available from Nippon Denshoku Industries Co., Ltd.) and a quartz cell with an optical path length of 10 mm.

Water added heating test (acid concentration analysis)

The target 1,3-butylene glycol product adjusted to a 90 wt.% aqueous solution and then kept at 100°C for 1 week was used as a sample for an acid concentration analysis performed according to the technique below. In addition, the 1,3-butylene glycol product prior to the test was also subjected to acid concentration analysis by the following technique.

Acid concentration analysis

The sample was measured using an automatic potentiometric titrator (AT-510 available from Kyoto Electronics Manufacturing Co., Ltd.). First, 50 g of the sample was diluted with 50 g of distilled water and titrated with 0.01 N aqueous sodium hydroxide solution from a burette with stirring until the titration was automatically stopped at the endpoint. Then, the acid concentration (acid content) in terms of acetic acid was calculated based on the following equation.

$$\text{Acid concentration (wt.\%)} = \text{titration volume (mL)} \times F \times A \times (100/\text{sample amount (g)})$$

F: 1.0 (factor of the 0.01 N aqueous sodium hydroxide solution)
A: 0.0006 (grams of acetic acid corresponding to 1 mL of the aqueous sodium hydroxide solution)

Odor test

The target 1,3-butylene glycol product (100 mL) was placed in a wide-mouth reagent bottle (internal volume: 100 mL), and the bottle was stoppered tightly and allowed to stand at room temperature for a while (about 120 minutes). Then, the stopper was opened, the content was transferred to a 300-mL wide-mouth beaker, and 100 mL of pure water was added into the beaker to make a total of 200 mL. The wide-mouth beaker was shaken manually to stir the content, and the odor was then immediately smelled and scored according to the following evaluation. The same odor test was performed also on the sample after the coloration-over-time test 1, and the sample after the water added heating test.

1: Odor is not sensed
2: Slightly odorous
3: Odor is clearly sensed

The coloration-over-time tests 1 and 2 and the water added heating test are accelerated tests assuming long-term storage of 1,3 BG products.

Considerations of results

The results of the comparative examples and the examples are shown in Table 1, Table 2, and Table 3.

[Table 1]

Table 1

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|---|
| Charge into dehydration column A | Part | 100 | 100 | ← | 100 | 100 | ← | ← | 100 | ← | ← | ← | ← | ← | ← |
| | 1,3 BG GC area % | 56 | 66 | ← | 76 | 56 | ← | ← | 66 | ← | ← | ← | ← | ← | ← |
| | Water wt. % | 40 | 30 | ← | 20 | 40 | ← | ← | 30 | ← | ← | ← | ← | ← | ← |
| | AD ppm | 130 | 145 | ← | 155 | 130 | ← | ← | 145 | ← | ← | ← | ← | ← | ← |
| | CR ppm | 89 | 110 | ← | 117 | 89 | ← | ← | 110 | ← | ← | ← | ← | ← | ← |
| | Low boiling substance GC area % | 3 | 3 | ← | 3 | 3 | ← | ← | 3 | ← | ← | ← | ← | ← | ← |
| | High boiling substance GC area % | 1 | 1 | ← | 1 | 1 | ← | ← | 1 | ← | ← | ← | ← | ← | ← |
| Reflux ratio of dehydration column A | | 0.5 | 0.5 | ← | 1 | 1 | 50 | 50 | 2 | 10 | 20 | 20 | 20 | 50 | 100 |
| Distillate from dehydration column A | Part | 42 | 32 | ← | 23 | 43 | 43 | 43 | 33 | 33 | 33 | 33 | 33 | 33 | 33 |
| 1,3 BG recovery ratio | % | 99.3 | 99.8 | ← | 99.8 | 99.6 | 99.9 or greater | 99.6 | 99.9 | 99.9 or greater | 99.9 or greater | 99.9 or greater | 99.9 or greater | 99.9 or greater | 99.9 or greater |

(continued)

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|---|
| Charge into high boiling point | Part | 100 | ← | ← | ← | ← | ← | ← | ← | ← | ← | ← | ← | ← | ← |
| | 1,3 BG GC area % | 96.6 | 97.4 | ← | 97.9 | 97.4 | 97.9 | ← | 98.2 | 98.3 | 98.3 | 98.3 | 98.3 | 98.3 | 98.4 |
| | Water wt. % | 1.2 | 1.1 | ← | 0.7 | 0.9 | 0.2 | ← | 0.5 | 0.1 | 0.08 | 0.08 | 0.08 | 0.03 | 0.02 |
| component removal column C | AD ppm | 63 | 48 | ← | 39 | 20 | 9 | 9 | 38 | 18 | 13 | 13 | 13 | 5 | 4 |
| | CR ppm | 45 | 37 | ← | 23 | 19 | 12 | 9 | 30 | 12 | 11 | 11 | 11 | 5 | 3 |
| | Low boiling substanceGC area % | 1.6 | 1.1 | ← | 0.8 | 0.8 | 0.3 | ← | 0.3 | 0.2 | 0.2 | 0.2 | 0.2 | 0.2 | 0.1 |
| | High boiling substanceGC area % | 1.8 | 1.5 | ← | 1.3 | 1.8 | 1.8 | ← | 1.5 | 1.5 | 1.5 | 1.5 | 1.5 | 1.5 | 1.5 |
| High boiling point component removal column C | Reflux ratio | 0.02 | 0.02 | 0.02 | 0.02 | 0.05 | 0.05 | 0.05 | 0.05 | 0.05 | 0.05 | 0.05 | 0.05 | 0.05 | 0.05 |
| | Distillation extraction part | 80 | 80 | 80 | 80 | 80 | 80 | 80 | 80 | 80 | 80 | 80 | 80 | 80 | 80 |
| | Bottom extraction part | 20 | 20 | 20 | 20 | 20 | 20 | 20 | 20 | 20 | 20 | 20 | 20 | 20 | 20 |
| | Recycle | Absent | Absent | Absent | Absent | Absent | Absent | Absent | Absent | Absent | Absent | Absent | Absent | Absent | Absent |
| | 1,3 BG recovery ratio % | 81 | 81 | 81 | 81 | 82 | 81 | 81 | 81 | 81 | 81 | 81 | 81 | 81 | 81 |

(continued)

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|---|
| Charge into product column F | Part | 100 | 100 | ← | 100 | 100 | ← | 100 | 100 | ← | ← | ← | ← | ← | ← |
| | 1,3 BG GC area % | 97.8 | 98.3 | ← | 98.6 | 99.0 | 99.1 | 99.2 | 99.2 | 99.3 | 99.3 | 99.3 | 99.3 | 99.3 | 99.4 |
| | Water wt. % | 1.3 | 1.2 | ← | 0.8 | 1 | 0.3 | 0.3 | 0.6 | 0.3 | 0.2 | 0.2 | 0.2 | 0.1 | 0.08 |
| | AD ppm | 45 | 39 | ← | 38 | 20 | 9 | 9 | 29 | 16 | 10 | 11 | 9 | 3 | 2 |
| | CR ppm | 20 | 12 | ← | 13 | 9 | 6 | 6 | 13 | 5 | 3 | 3 | 3 | 2 | 1 |
| | Low boiling substance GC area % | 1.0 | 0.6 | ← | 0.6 | 0.4 | 0.3 | 0.3 | 0.3 | 0.2 | 0.2 | 0.2 | 0.2 | 0.2 | 0.1 |
| | High boiling substance GC area % | 1.2 | 1.1 | ← | 0.8 | 0.6 | 0.6 | 0.5 | 0.5 | 0.5 | 0.5 | 0.5 | 0.5 | 0.5 | 0.5 |
| Reflux ratio of product column F | | 0.05 | 0.05 | 0.05 | 0.1 | 0.5 | 0.5 | 10 | 0.5 | 0.5 | 2 | 100 | 500 | 2 | 2 |
| Distillation from product column F | Part | 20 | 20 | 30 | 20 | 10 | 10 | 1 | 10 | 10 | 1 | 1 | 1 | 1 | 1 |
| | Recycle | Absent | Absent | Absent | Absent | Absent | Absent | Absent | Present | Present | Present | Present | Present | Present | Present |

(continued)

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|---|---|
| Bottom (product) from product column F | Part | 80 | 80 | 70 | 80 | 90 | 90 | 99 | 90 | 90 | 99 | 99 | 99 | 99 | 99 |
| | 1,3 BG GC area % | 98.3 | 98.5 | 98.4 | 98.8 | 99.2 | 99.3 | 99.5 | 99.4 | 99.4 | 99.4 | 99.5 | 99.5 | 99.4 | 99.5 |
| | Water wt. % | 0.6 | 0.4 | 0.2 | 0.2 | 0.2 | 0.1 | 0.1 | 0.2 | 0.1 | 0.1 | 0.003 | 0.002 | 0.05 | 0.04 |
| | AD ppm | 5 | 4 | 2 | 2 | 1.5 | 0.7 | 1 | 2 | 1.1 | 1 | 0.2 | less than 0.2 | 0.3 | less than 0.2 |
| | CR ppm | 4 | 2 | 1.3 | 1.3 | 0.9 | 0.7 | 0.7 | 1 | 0.7 | 0.6 | 0.1 | less than 0.1 | 0.2 | less than 0.1 |
| | Low boiling substance GC area % | 0.2 | 0.1 | 0.1 | 0.1 | 0.08 | 0.05 | 0.05 | 0.04 | 0.03 | 0.07 | 0.004 | 0.003 | 0.06 | 0.03 |
| | High boiling substance GC area % | 1.5 | 1.4 | 1.5 | 1.1 | 0.7 | 0.7 | 0.5 | 0.6 | 0.6 | 0.5 | 0.5 | 0.5 | 0.5 | 0.5 |
| | Initial boiling point °C | 193.2 | 199.0 | 203.0 | 203.1 | 203.3 | 206.7 | 206.7 | 203.8 | 206.7 | 206.8 | 208.4 | 208.4 | 207.5 | 207.2 |
| | Dry point °C | 210.3 | 210.1 | 210.2 | 209.5 | 209 | 208.9 | 208.8 | 208.9 | 208.9 | 208.8 | 208.8 | 208.8 | 208.8 | 208.8 |
| | PMT min | 0 | 5 | 30 | 30 | 35 | 45 | 40 | 30 | 40 | 40 | 55 | 60 | 50 | 55 |
| | 1,3 BG recovery ratio % | 81 | 80 | 70 | 80 | 90 | 90 | 99 or greater | 99 or greater | 99 or greater | 99 or greater | 99 or greater | 99 or greater | 99 or greater | 99 or greater |

[Table 2]

Table 2

| | | Exam-pl e 11 | Exam-ple 12 | Exam-ple 13 | Exam-ple 14 | Exam-ple 15 | Exam-ple 16 | Exam-ple 17 | Exam-ple 18 | Exam-ple 19 | Exam-ple 20 | Exam-ple 21 | Exam-ple 22 | Exam-ple 23 | Exam-ple 24 | Exam-ple 25 | Exam-ple 26 | Exam-ple 27 |
|---|---|---|---|---|---|---|---|---|---|---|---|---|---|---|---|---|---|---|
| Charge into dehy-dration column A | Part | 100 | ← | ← | 100 | ← | ← | ← | ← | ← | ← | ← | 100 | 100 | 100 | 100 | 100 | 100 |
| | 1,3 BG GC area % | 76 | ← | ← | 66 | ← | ← | ← | ← | ← | ← | ← | 56 | 76 | 9 | 66 | 67 | 69 |
| | Water wt. % | 20 | ← | ← | 30 | ← | ← | ← | ← | ← | ← | ← | 40 | 20 | 90 | 30 | 30 | 30 |
| | AD ppm | 155 | ← | ← | 145 | ← | ← | ← | ← | ← | ← | ← | 130 | 155 | 85 | 970 | 145 | 3 |
| | CR ppm | 117 | ← | ← | 110 | ← | ← | ← | ← | ← | ← | ← | 89 | 117 | 71 | 390 | 110 | 1 |
| | Low boil-ing sub-stance GC area % | 3 | ← | ← | 3 | ← | ← | ← | ← | ← | ← | ← | 3 | 3 | 0.4 | 3 | 3 | 1 |
| | High boil-ing sub-stance GC area % | 1 | ← | ← | 1 | ← | 1.2 | ← | ← | ← | ← | ← | 1 | 1 | 0.1 | 1 | 1 | 0.5 |
| Reflux ra-tio of dehy-dration column A | | 2 | 10 | 100 | 10 | 10 | 10 | 10 | 10 | 10 | 10 | 10 | 0.5 | 100 | 20 | 2 | 0.3 | 10 |
| Distillate from dehydration column A | Part | 23 | 23 | 23 | 33 | 33 | 33 | 33 | 33 | 33 | 33 | 33 | 42 | 23 | 91 | 33 | 32 | 32 |
| 1,3 BG re-covery ra-tio | % | 99.8 | 99.9 or greater | 99.9 or greater | 99.8 | 99.8 | 99.8 | 99.8 | 99.8 | 99.8 | 99.8 | 99.8 | 99.3 | 99.9 or greater | 99.0 | 99.9 or greater | 99.8 | 99.9 or greater |

(continued)

| | | Exam-pl e 11 | Exam-ple 12 | Exam-ple 13 | Exam-ple 14 | Exam-ple 15 | Exam-ple 16 | Exam-ple 17 | Exam-ple 18 | Exam-ple 19 | Exam-ple 20 | Exam-ple 21 | Exam-ple 22 | Exam-ple 23 | Exam-ple 24 | Exam-ple 25 | Exam-ple 26 | Exam-ple 27 |
|---|---|---|---|---|---|---|---|---|---|---|---|---|---|---|---|---|---|---|
| Charge into high boiling point component removal column C | Part | 100 | ← | ← | ← | ← | ← | ← | ← | ← | ← | ← | 100 | ← | 100 | 100 | 100 | ← |
| | 1,3 BG GC area % | 98.4 | 98.5 | 98.6 | 98.3 | ← | 98.1 | 98.3 | ← | ← | ← | ← | 96.6 | 98.6 | 97.5 | 98.2 | 96.7 | 99.1 |
| | Water wt. % | 0.1 | 0.08 | 0.01 | 0.1 | ← | 0.1 | 0.1 | ← | ← | ← | ← | 1.2 | 0.01 | 3 | 0.5 | 1.3 | 0.1 |
| | AD ppm | 29 | 15 | 2 | 18 | ← | 18 | 18 | ← | ← | ← | ← | 63 | 2 | 9 | 221 | 61 | 0.3 |
| | CR ppm | 21 | 8 | 1 | 12 | ← | 12 | 12 | ← | ← | ← | ← | 45 | 1 | 4 | 128 | 48 | 0.2 |
| | Low boiling substance GC area % | 0.3 | 0.2 | 0.1 | 0.2 | ← | 0.2 | 0.2 | ← | ← | ← | ← | 1.6 | 0.1 | 1.1 | 0.3 | 1.8 | 0.1 |
| | High boiling substance GC area % | 1.3 | 1.3 | 1.3 | 1.5 | ← | 1.7 | 1.5 | ← | ← | ← | ← | 1.8 | 1.3 | 1.4 | 1.5 | 1.5 | 0.8 |

(continued)

| | | Exampl e 11 | Example 12 | Example 13 | Example 14 | Example 15 | Example 16 | Example 17 | Example 18 | Example 19 | Example 20 | Example 21 | Example 22 | Example 23 | Example 24 | Example 25 | Example 26 | Example 27 |
|---|---|---|---|---|---|---|---|---|---|---|---|---|---|---|---|---|---|---|
| High boiling point component removal column C | Reflux ratio | 0.05 | 0.05 | 0.05 | 0.1 | 0.5 | 0.5 | 0.5 | 1 | 2 | 5 | 20 | 0.1 | 1 | 1 | 0.05 | 0.05 | 1 |
| | Distillation extraction part | 80 | 80 | 80 | 90 | 90 | 90 | 95 | 95 | 95 | 95 | 95 | 90 | 90 | 90 | 80 | 80 | 95 |
| | Bottom extraction part | 20 | 20 | 20 | 10 | 10 | 10 | 5 | 5 | 5 | 5 | 5 | 10 | 10 | 10 | 20 | 20 | 5 |
| | Recycle | Absent | Absent | Absent | Absent | Absent | Present | Absent | Absent | Absent | Absent | Absent | Absent | Absent | Absent | Absent | Absent | Absent |
| | 1,3 BG recovery ratio % | 81 | 81 | 81 | 91 | 91 | 99.9 or greater | 96 | 96 | 96 | 96 | 96 | 92 | 91 | 91 | 81 | 81 | 96 |
| Charge into product column F | Part | 100 | ← | ← | 100 | 100 | 100 | 100 | 100 | 100 | 100 | 100 | 100 | - | 100 | 100 | 100 | 100 |
| | 1,3 BG GC area % | 99.3 | 99.4 | 99.5 | 99.4 | 99.6 | 99.5 | 99.5 | 99.7 | 99.7 | 99.7 | 99.7 | 98.7 | - | 98.7 | 99.2 | 97.6 | 99.7 |
| | Water wt. % | 0.3 | 0.1 | 0.09 | 0.3 | 0.3 | 0.3 | 0.3 | 0.3 | 0.3 | 0.3 | 0.3 | 1.2 | - | 3 | 0.7 | 1.4 | 0.3 |
| | AD ppm | 22 | 12 | 1 | 10 | 9 | 10 | 10 | 9 | 8 | 8 | 9 | 43 | | 7 | 205 | 54 | 0.3 |
| | CR ppm | 14 | 5 | 0.7 | 5 | 6 | 6 | 5 | 5 | 5 | 4 | 5 | 17 | - | 3 | 110 | 38 | 0.2 |

(continued)

| | | Example 11 | Example 12 | Example 13 | Example 14 | Example 15 | Example 16 | Example 17 | Example 18 | Example 19 | Example 20 | Example 21 | Example 22 | Example 23 | Example 24 | Example 25 | Example 26 | Example 27 |
|---|---|---|---|---|---|---|---|---|---|---|---|---|---|---|---|---|---|---|
| | Low boiling substance GC area % | 0.3 | 0.2 | 0.1 | 0.2 | 0.2 | 0.2 | 0.2 | 0.2 | 0.2 | 0.2 | 0.2 | 0.9 | - | 0.8 | 0.3 | 1.8 | 0.1 |
| | High boiling substance GC area % | 0.4 | 0.4 | 0.4 | 0.4 | 0.2 | 0.3 | 0.3 | 0.1 | 0.09 | 0.07 | 0.06 | 0.4 | - | 0.5 | 0.5 | 0.6 | 0.2 |
| Reflux ratio of product column F | | 1 | 2 | 2 | 1 | 1 | 1 | 1 | 1 | 1 | 1 | 1 | 5 | | 10 | 10 | 20 | 1 |
| Distillate from product column F | Part | 10 | 1 | 1 | 10 | 10 | 10 | 10 | 10 | 10 | 10 | 10 | 10 | - | 10 | 10 | 10 | 10 |
| | Recycle | Present | Present | Present | Present | Present | Present | Present | Present | Present | Present | Present | Present | | Present | Present | Present | Present |

(continued)

| | | Exam-pl e 11 | Exam-ple 12 | Exam-ple 13 | Exam-ple 14 | Exam-ple 15 | Exam-ple 16 | Exam-ple 17 | Exam-ple 18 | Exam-ple 19 | Exam-ple 20 | Exam-ple 21 | Exam-ple 22 | Exam-ple 23 | Exam-ple 24 | Exam-ple 25 | Exam-ple 26 | Exam-ple 27 |
|---|---|---|---|---|---|---|---|---|---|---|---|---|---|---|---|---|---|---|
| Bottom (product) from product column F | Part | 90 | 99 | 99 | 90 | 90 | 90 | 90 | 90 | 90 | 90 | 90 | 90 | - | 90 | 90 | 90 | 90 |
| | 1,3 BG GC area % | 99.4 | 99.4 | 99.5 | 99.5 | 99.8 | 99.6 | 99.6 | 99.9 | 99.9 | 99.9 | 99.9 | 99.4 | 99.5 | 99.4 | 99.4 | 99.3 | 99.8 |
| | Water wt. % | 0.06 | 0.05 | 0.04 | 0.05 | 0.05 | 0.05 | 0.05 | 0.05 | 0.05 | 0.05 | 0.05 | 0.1 | 0.09 | 0.1 | 0.02 | 0.01 | 0.05 |
| | AD ppm | 1.6 | 1 | less than 0.2 | 0.9 | 0.8 | 0.8 | 0.9 | 0.8 | 0.9 | 0.9 | 0.8 | 1.1 | 1.5 | 0.3 | 1.2 | 0.5 | less than 0.2 |
| | CR ppm | 1 | 0.8 | less than 0.1 | 0.7 | 0.6 | 0.6 | 0.5 | 0.5 | 0.4 | 0.4 | 0.3 | 0.4 | 1 | 0.2 | 1.1 | 0.4 | less than 0.1 |
| | Low boiling substance GC area % | 0.09 | 0.07 | 0.05 | 0.02 | 0.02 | 0.02 | 0.02 | 0.02 | 0.02 | 0.02 | 0.02 | 0.1 | 0.1 | 0.02 | 0.004 | 0.03 | 0.01 |
| | High boiling substance GC area % | 0.5 | 0.5 | 0.5 | 0.5 | 0.2 | 0.4 | 0.4 | 0.1 | 0.09 | 0.08 | 0.07 | 0.5 | 0.4 | 0.6 | 0.6 | 0.7 | 0.2 |
| | Initial boiling point °C | 207.3 | 207.5 | 207.2 | 207.5 | 207.5 | 207.5 | 207.5 | 207.5 | 207.5 | 207.6 | 207.6 | 206.8 | 206.9 | 206.7 | 208.0 | 207.8 | 207.6 |
| | Dry point °C | 208.9 | 208.9 | 208.9 | 208.8 | 208.6 | 208.8 | 208.8 | 208.6 | 208.5 | 208.5 | 208.4 | 208.8 | 208.8 | 208.9 | 208.9 | 208.9 | 208.6 |
| | PMT min | 35 | 40 | 60 | 45 | 45 | 45 | 45 | 45 | 45 | 45 | 45 | 45 | 35 | 50 | 35 | 45 | 65 |
| | 1,3 BG recovery ratio % | 90 | 99 or greater | 99 or greater | 99.9 or greater | 99.9 or greater | 99.9 or greater | 99.9 or greater | 99.9 or greater | 99.9 or greater | 99.9 or greater | 99.9 or greater | 99.9 or greater | - | 99.9 or greater | 99 or greater | 99 or greater | 99.9 or greater |

[Table 3]

Table 3

| | Comparative Example 1 | Comparative Example 2 | Example 1 | Example 7 | Example 18 | Example 27 |
|---|---|---|---|---|---|---|
| Pre-test impurity content (ppm) | | | | | | |
| Acetaldehyde | 5 | 4 | 1.6 | 0.2 | 0.8 | less than 0.2 |
| Crotonaldehyde | 4 | 2 | 1 | 0.1 | 0.5 | less than 0.1 |
| Methyl vinyl ketone | 11 | 10 | 6 | less than 0.2 | 0.9 | less than 0.2 |
| Acetone | 10 | 9 | 5 | less than 0.2 | 0.8 | less than 0.2 |
| Formaldehyde | 4 | 3 | 1 | less than 0.2 | 0.8 | less than 0.2 |
| Butylaldehyde | 9 | 8 | 5 | less than 0.2 | 0.7 | less than 0.2 |
| Acetaldol | 10 | 10 | 6 | less than 0.2 | 0.5 | less than 0.2 |
| 1-Hydroxy-3-butanone | 9 | 9 | 6 | less than 0.2 | 1 | less than 0.2 |
| 2-Butanol | 1 | 1 | less than 0.2 | less than 0.2 | 0.3 | less than 0.2 |
| Compound represented by Formula (1) | 5 | 4 | 2 | less than 0.2 | 0.8 | less than 0.2 |
| Compound represented by Formula (2) | 3 | 3 | 1 | less than 0.2 | less than 0.2 | less than 0.2 |
| Compound represented by Formula (3) | 8 | 7 | 4 | less than 0.2 | 4 | less than 0.2 |
| Compound represented by Formula (4) | 7 | 5 | 3 | less than 0.2 | 3 | less than 0.2 |
| Compound represented by Formula (5) | 9 | 8 | 6 | less than 0.2 | less than 0.2 | less than 0.2 |
| Compound represented by Formula (6) | 8 | 7 | 5 | 0.5 | less than 0.2 | less than 0.2 |
| Compound represented by Formula (7) | 11 | 10 | 7 | 0.5 | 0.8 | less than 0.2 |
| Compound represented by Formula (8) | 3 | 3 | 1 | less than 0.2 | less than 0.2 | less than 0.2 |
| Compound represented by Formula (9) | 9 | 8 | 4 | 0.3 | less than 0.2 | less than 0.2 |
| Compound represented by Formula (10) | 2 | 1 | 1 | 0.3 | less than 0.2 | less than 0.2 |
| Pre-test initial boiling point °C | 193.2 | 199 | 203.3 | 208.4 | 207.5 | 207.6 |

(continued)

| | Comparative Example 1 | Comparative Example 2 | Example 1 | Example 7 | Example 18 | Example 27 |
|---|---|---|---|---|---|---|
| Pre-test dry point °C | 210.3 | 210.1 | 209 | 208.8 | 208.6 | 208.6 |
| Pre-test PMT min | 0 | 5 | 35 | 55 | 45 | 65 |
| Pre-test APHA | 7 | 7 | 4 | 1 | 2 | 1 |
| Pre-test acid content (ppm) | 11 | 11 | 6 | 2 | 3 | 1 |
| Pre-test odor | 2 | 2 | 1 | 1 | 1 | 1 |
| Impurity content (ppm) after coloration test 1 | | | | | | |
| Acetaldehyde | 7 | 5 | 1 | 0.2 | 0.6 | less than 0.2 |
| Crotonaldehyde | 3 | 1 | 0.5 | 0.1 | 0.3 | less than 0.1 |
| Methyl vinyl ketone | 9 | 8 | 4 | less than 0.2 | 0.8 | less than 0.2 |
| Acetone | 8 | 6 | 2 | less than 0.2 | 0.5 | less than 0.2 |
| Formaldehyde | 4 | 2 | 0.5 | less than 0.2 | 0.6 | less than 0.2 |
| Butylaldehyde | 8 | 4 | 3 | less than 0.2 | 0.5 | less than 0.2 |
| Acetaldol | 2 | 1 | 0.5 | less than 0.2 | 0.4 | less than 0.2 |
| 1-Hydroxy-3-butanone | 1 | 1 | 0.5 | less than 0.2 | 0.4 | less than 0.2 |
| 2-Butanol | 2 | 2 | less than 0.2 | less than 0.2 | 0.2 | less than 0.2 |
| Compound represented by Formula (1) | 10 | 9 | 6 | less than 0.2 | 3 | less than 0.2 |
| Compound represented by Formula (2) | 8 | 8 | 5 | less than 0.2 | 3 | less than 0.2 |
| Compound represented by Formula (3) | 10 | 9 | 4 | less than 0.2 | 2 | less than 0.2 |
| Compound represented by Formula (4) | 11 | 8 | 5 | 0.3 | 4 | less than 0.2 |
| Compound represented by Formula (5) | 12 | 11 | 7 | 0.2 | 4 | less than 0.2 |
| Compound represented by Formula (6) | 10 | 9 | 6 | 0.5 | 3 | less than 0.2 |
| Compound represented by Formula (7) | 14 | 12 | 8 | 0.7 | 3 | less than 0.2 |
| Compound represented by Formula (8) | 9 | 10 | 6 | less than 0.2 | 5 | less than 0.2 |
| Compound represented by Formula (9) | 11 | 10 | 5 | 0.4 | 4 | less than 0.2 |

(continued)

| | Comparative Example 1 | Comparative Example 2 | Example 1 | Example 7 | Example 18 | Example 27 |
|---|---|---|---|---|---|---|
| Compound represented by Formula (10) | 12 | 11 | 7 | 0.8 | 4 | less than 0.2 |
| Initial boiling point °C after coloration test 1 | 193 | 198.8 | 203.2 | 208.4 | 207.4 | 207.6 |
| Dry point °C after coloration test 1 | 210.5 | 210.3 | 209 | 208.8 | 208.6 | 208.6 |
| PMT min after coloration test 1 | 0 | 0 | 30 | 50 | 40 | 65 |
| Odor after coloration test 1 | 3 | 3 | 2 | 1 | 1 | 1 |
| APHA after coloration test 1 | 85 | 79 | 25 | 10 | 13 | 7 |
| Acid content (ppm) after coloration test 1 | 17 | 16 | 8 | 3 | 3 | 1 |
| APHA after coloration test 2 | 51 | 43 | 12 | 3 | 5 | 2 |
| Acid content (ppm) after water added heating test | 27 | 23 | 9 | 2 | 3 | 1 |
| Odor after water added heating test | 3 | 3 | 2 | 1 | 1 | 1 |

Since many impurities are produced in the 1,3 BG production process, the impurities shown in Table 3 are generally contained in the 1,3 BG product, though varying depending on the conditions for the aldol condensation, the conditions for the hydrogen addition reaction, and the subsequent purification conditions. For example, acetaldol, crotonaldehyde, various carbonyl compounds, and alcohols contained in the 1,3 BG product react with oxygen during long-term storage to produce peroxides. In particular, aldehydes produce peroxides relatively more easily than ketones, alcohols and hydrocarbons. Peroxides produced from impurities react with the product 1,3 BG and promote oxidation from the top (1,3 BG) of the tree (see the impurity formation pathway diagram). Note that acetaldol is mainly produced by dimerization of acetaldehyde. Therefore, reducing the amounts of a series of impurities in the reaction system or the purification system makes it possible to suppress the concentration of peroxides produced from impurities during long-term storage, and thus suppress the formation reaction of many impurities via the additional impurities formed from 1-hydroxy-3-butanone, acetaldol, and both compounds thereof by oxidation of 1,3 BG. For example, among the impurities shown in Table 3, the substances formed by reacting with oxygen during long-term storage in the presence of oxygen are 1-hydroxy-3-butanone, acetaldol, 2-butanol, the compound represented by Formula (8), the compound represented by Formula (9), the compound represented by Formula (10), and the like, and other impurities are formed in the presence of 1-hydroxy-3-butanone or acetaldol, 1,3 BG, and other impurities, as well as a small amount of water.

It can be seen, from the results of the comparative examples and the examples, that greater contents (initial contents) of acetaldehyde, crotonaldehyde, methyl vinyl ketone, acetone, formaldehyde, butylaldehyde, acetaldol, and 1-hydroxy-3-butanone in the 1,3 BG products tend to increase the contents of impurities represented by Formulas (1) to Formula (10) more. Also, in Comparative Examples 1 and 2, in which the initial contents of acetaldehyde, crotonaldehyde, methyl vinyl ketone, acetone, formaldehyde, butylaldehyde, acetaldol, and 1-hydroxy-3-butanone were large, and impurities represented by Formulas (1) to (10) increased after a heating test under specific conditions assuming long-term storage, APHA, odor, initial boiling point, dry point, PMT, and acid content of the 1,3 BG products were severely deteriorated. On the other hand, it can be seen that, in Examples 1, 7, 18, and 27, in which the initial contents of acetaldehyde, crotonaldehyde, methyl vinyl ketone, acetone, formaldehyde, butylaldehyde, acetaldol, and 1-hydroxy-3-butanone were low and, after the heating test, the impurities represented by Formulas (1) to (10) did not increase so much or did not increase at all, the quality of the 1,3 BG products was hardly or not deteriorated even after the heating test. In particular, it can be seen that, in the order of Example 1 → Example 18 → Example 7 → Example 27, the contents of the impurities represented by Formulas (1) to (10) after the heating test decreased as a whole, and, at the same time, that the quality stability of the 1,3 BG products was also improved. The above results indicate that there is a correlation between the contents of the impurities represented by Formulas (1) to (10) after the heating test of the 1,3 BG product and the deterioration over time in quality of the 1,3 BG product. Also, there is a correlation between the contents (initial

contents) of acetaldehyde, crotonaldehyde, methyl vinyl ketone, acetone, formaldehyde, butylaldehyde, acetaldol, and 1-hydroxy-3-butanone in the 1,3 BG product, and the contents of the impurities represented by Formulas (1) to Formula (10) after the heating test of the 1,3 BG product.

As a summary of the above, configurations and variations of the present disclosure are described below.

[1] A 1,3-butylene glycol product containing 1,3-butylene glycol, in which, after the 1,3-butylene glycol product has been kept at 180°C for 3 hours in air atmosphere, at least one of contents of compounds represented by the following Formula (A) or (B) is less than 8 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm):

[Chemical Formula 6]

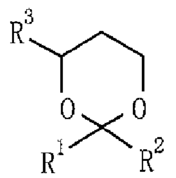

(A)

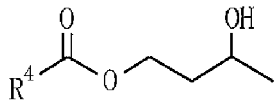

(B)

where $R^1$ to $R^4$ are the same as or different from each other, and each of $R^1$ to $R^4$ is a hydrogen atom, an alkyl group with from 1 to 4 carbon atoms which may be substituted with a hydroxy group, or an alkenyl group with 2 to 4 carbon atoms which may be substituted with a hydroxy group.

[2] The 1,3-butylene glycol product according to [1], in which, after the 1,3-butylene glycol product has been kept at 180°C for 3 hours in air atmosphere, each of contents of two compounds (or three compounds, four compounds, five compounds, six compounds, seven compounds, eight compounds, nine compounds, or ten compounds), of the contents of the compounds represented by Formula (A) or (B), is less than 8 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[3] The 1,3-butylene glycol product according to [1], in which, after the 1,3-butylene glycol product has been kept at 180°C for 3 hours in air atmosphere, contents of at least four (four, five, six, seven, eight, nine, or ten) compounds, of the contents of the compounds represented by Formula (A) or (B), are less than 8 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[4] The 1,3-butylene glycol product according to any one of [1] to [3], in which, after the 1,3-butylene glycol product has been kept at 180°C for 3 hours in air atmosphere, a sum of the contents of the compounds represented by Formula (A) [or a sum of contents of compounds represented by the following Formulas (1) to (7)] is less than 66 ppm (or 60 ppm or less, 50 ppm or less, 40 ppm or less, 30 ppm or less, 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[5] The 1,3-butylene glycol product according to any one of [1] to [4], in which, after the 1,3-butylene glycol product has been kept at 180°C for 3 hours in air atmosphere, a sum of the contents of the compounds represented by Formula (B) [or a sum of contents of compounds represented by the following Formulas (8) to (10)] is less than 31 ppm (or 25 ppm or less, 20 ppm or less, 18 ppm or less, 16 ppm or less, 14 ppm or less, 13 ppm or less, 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[6] The 1,3-butylene glycol product according to any one of [1] to [5], in which the compound represented by Formula (A) is any of the compounds represented by the following Formulas (1) to (7), and the compound represented by Formula (B) is any of the compounds represented by the following Formulas (8) to (10).

[Chem. 7]

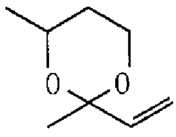
(1)

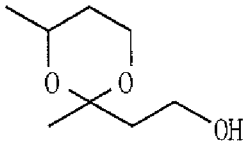
(2)

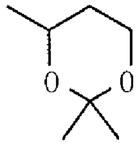
(3)

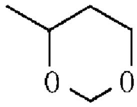
(4)

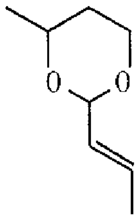
(5)

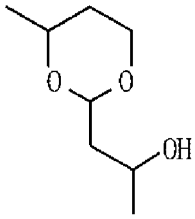
(6)

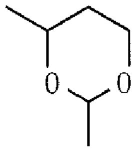
(7)

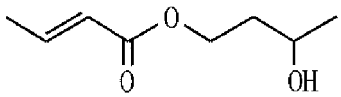
(8)

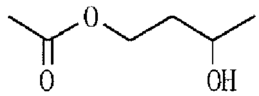
(9)

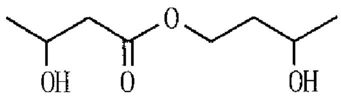
(10)

[7] The 1,3-butylene glycol product according to [6], in which, after the 1,3-butylene glycol product has been kept at 180°C for 3 hours in air atmosphere, a total content of the compounds represented by Formulas (1) to (4) is less than 34 ppm (or, 30 ppm or less, 25 ppm or less, 20 ppm or less, 18 ppm or less, 16 ppm or less, 15 ppm or less, 14 ppm or less, 13 ppm or less, 12 ppm or less, 11 pm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[8] The 1,3-butylene glycol product according to [6] or [7], in which, after the 1,3-butylene glycol product has been kept at 180°C for 3 hours in air atmosphere, a total content of the compounds represented by Formulas (5) to (10) is less than 63 ppm (or, 60 ppm or less, 50 ppm or less, 40 ppm or less, 30 ppm or less, 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[9] The 1,3-butylene glycol product according to any one of [6] to [8], in which, after the 1,3-butylene glycol product has been kept at 180°C for 3 hours in air atmosphere, a total content of the compounds represented by Formulas (1) to (10) is less than 97 ppm (or 80 ppm or less, 70 ppm or less, 60 ppm or less, 50 ppm or less, 40 ppm or less, 30 ppm or less, 20 ppm or less, 18 ppm or less, 16 ppm or less, 14 ppm or less, 12 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[10] The 1,3-butylene glycol product according to any one of [1] to [9], in which, after the 1,3-butylene glycol product has been kept at 180°C for 3 hours in air atmosphere, a content of methyl vinyl ketone is less than 8 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm) and/or a content of 1-hydroxy-3-butanone is less than 1 ppm (0.5 ppm or less), and/or a content of acetone is less than 6 ppm (or 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), and/or a content of formaldehyde is less than 2 ppm (or 1 ppm or less, 0.5 ppm or less, 0.4 ppm or less, 0.3 ppm or less, or

less than 0.2 ppm), and/or a content of crotonaldehyde is less than 1 ppm (or 0.5 ppm or less), and/or a content of acetaldol is less than 1 ppm (or 0.5 ppm or less, 0.4 ppm or less, 0.3 ppm or less, or less than 0.2 ppm), and/or a content of acetaldehyde is less than 5 ppm (or 4 ppm or less, 3 ppm or less, 2 ppm or less, 1 ppm or less, 0.5 ppm or less, 0.4 ppm or less, 0.3 ppm or less, or less than 0.2 ppm), and/or a content of butylaldehyde is less than 4 ppm (or 3 ppm or less, 2 ppm or less, 1 ppm or less, 0.5 ppm or less, 0.4 ppm or less, 0.3 ppm or less, or less than 0.2 ppm).

[11] The 1,3-butylene glycol product according to any one of [1] to [10], in which, after the 1,3-butylene glycol product has been kept at 180°C for 3 hours in air atmosphere, a total content of methyl vinyl ketone, 1-hydroxy-3-butanone, acetone, formaldehyde, crotonaldehyde, acetaldol and acetaldehyde is less than 24 ppm (or 20 ppm or less, 18 ppm or less, 16 ppm or less, 14 ppm or less, 12 ppm or less, 11 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[12] The 1,3-butylene glycol product according to any one of [1] to [11], in which, after the 1,3-butylene glycol product has been kept at 180°C for 3 hours in air atmosphere, a total content of methyl vinyl ketone, acetone, and butylaldehyde is less than 18 ppm (or 16 ppm or less, 15 ppm or less, 14 ppm or less, 13 ppm or less, 12 ppm or less, 11 ppm or less, 10 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[13] The 1,3-butylene glycol product according to any one of [1] to [12], in which, after the 1,3-butylene glycol product has been kept at 180°C for 3 hours in air atmosphere, an APHA is less than 79 (or 65 or less, 60 or less, 55 or less, 50 or less, 45 or less, 40 or less, 35 or less, 30 or less, 25 or less, 20 or less, 18 or less, 15 or less, 14 or less, 13 or less, 12 or less, 11 or less, 10 or less, 9 or less, 8 or less, or 7 or less).

[14] The 1,3-butylene glycol product according to any one of [1] to [13], in which, after the 1,3-butylene glycol product has been kept at 180°C for 3 hours in air atmosphere, an initial boiling point is higher than 203°C (or 204°C or higher, 205°C or higher, 206°C or higher, 207°C or higher, or 208°C or higher), and/or a dry point is 209°C or lower.

[15] The 1,3-butylene glycol product according to any one of [1] to [14], in which, after the 1,3-butylene glycol product has been kept at 180°C for 3 hours in air atmosphere, a potassium permanganate test value is 30 minutes or longer (or longer than 30 minutes, 32 minutes or longer, 35 minutes or longer, 40 minutes or longer, 50 minutes or longer, or 60 minutes or longer).

[16] The 1,3-butylene glycol product according to any one of [1] to [15], in which, after the 1,3-butylene glycol product has been kept at 180°C for 3 hours in air atmosphere, an acid concentration (in terms of acetic acid) is less than 16 ppm (or 14 ppm or less, 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[17] The 1,3-butylene glycol product according to any one of [1] to [16], in which, in a state where the 1,3-butylene glycol product has not undergone a test in which 1,3-butylene glycol product is kept at 180°C for 3 hours in air atmosphere, a total content of 1-hydroxy-3-butanone and acetaldol is less than 19 ppm (or 18 ppm or less, 16 ppm or less, 14 ppm or less, 12 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[18] The 1,3-butylene glycol product according to [1] to [17], in which, in a state where the 1,3-butylene glycol product has not undergone a test in which 1,3-butylene glycol product is kept at 180°C for 3 hours in air atmosphere, a content of methyl vinyl ketone is less than 10 ppm (or 8 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), and/or a content of 1-hydroxy-3-butanone is less than 9 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), and/or a content of acetone is less than 9 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), and/or a content of formaldehyde is less than 3 ppm (or 2 ppm or less, 1 ppm or less, 0.5 ppm or less, 0.4 ppm or less, 0.3 ppm or less, or less than 0.2 ppm), and/or a content of crotonaldehyde is less than 2 ppm (or 1.5 ppm or less, 1 ppm or less, 0.5 ppm or less, 0.4 ppm or less, 0.3 ppm or less, or less than 0.2 ppm), and/or a content of acetaldol is less than 10 ppm (or 8 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm), and/or a content of acetaldehyde is or less 4 ppm (or 3 ppm or less, 2 ppm or less, 1 ppm or less, 0.5 ppm or less, 0.4 ppm or less, 0.3 ppm or less, or less than 0.2 ppm) and/or a content of butylaldehyde is less than 8 ppm (or 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[19] The 1,3-butylene glycol product according to any one of [1] to [18], in which, in a state where the 1,3-butylene glycol product has not undergone a test in which 1,3-butylene glycol product is kept at 180°C for 3 hours in air

atmosphere, a total content of methyl vinyl ketone, 1-hydroxy-3-butanone, acetone, formaldehyde, crotonaldehyde, acetaldol and acetaldehyde is less than 47 ppm (or 40 ppm or less, 30 ppm or less, 25 ppm or less, 20 ppm or less, 18 ppm or less, 16 ppm or less, 14 ppm or less, 13 ppm or less, 12 ppm or less, 11 pm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[20] The 1,3-butylene glycol product according to any one of [1] to [19], in which, in a state where the 1,3-butylene glycol product has not undergone a test in which 1,3-butylene glycol product is kept at 180°C for 3 hours in air atmosphere, an acid concentration (in terms of acetic acid) is less than 11 ppm (or 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[21] The 1,3-butylene glycol product according to any one of [1] to [20], in which, in a state where the 1,3-butylene glycol product has not undergone a test in which 1,3-butylene glycol product is kept at 180°C for 3 hours in air atmosphere, an acid concentration (in terms of acetic acid) after a 90 wt.% aqueous solution of the 1,3-butylene glycol product is kept at 100°C for 1 week in air atmosphere is less than 23 ppm (20 ppm or less, 19 ppm or less, 18 ppm or less, 17 ppm or less, 16 ppm or less, 15 ppm or less, 14 ppm or less, 13 ppm or less, 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.5 ppm or less, 0.4 ppm or less, 0.3 ppm or less, or less than 0.2 ppm).

[22] The 1,3-butylene glycol product according to any one of [1] to [21], in which, in a state where the 1,3-butylene glycol product has not undergone a test in which 1,3-butylene glycol product is kept at 180°C for 3 hours in air atmosphere, for the acid concentration (in terms of acetic acid) of the 90 wt.% aqueous solution of the 1,3-butylene glycol product, a ratio of the acid concentration after keeping at 100°C for 1 week to the acid concentration before keeping [(acid concentration after keeping at 100°C for 1 week)/(acid concentration before keeping) × 100 (%)] is 200% or less (or 150% or less or 120% or less).

[23] The 1,3-butylene glycol product according to any one of [1] to [22], in which, in a state where the 1,3-butylene glycol product has not undergone a test in which 1,3-butylene glycol product is kept at 180°C for 3 hours in air atmosphere, an APHA is 6 or less (or 5 or less, 4 or less, 3 or less, or 2 or less).

[24] The 1,3-butylene glycol product according to any one of [1] to [23], in which, the 1,3-butylene glycol product has been kept at 100°C for 75 days in air atmosphere, an APHA is 42 or less (35 or less, 30 or less, 25 or less, 20 or less, 18 or less, 16 or less, 15 or less, 14 or less, 13 or less, 12 or less, 11 or less, 10 or less, 9 or less, 8 or less, 7 or less, 6 or less, 5 or less, 4 or less, 3 or less, or 2 or less).

[25] The 1,3-butylene glycol product according to any one of [1] to [24], in which, for the 1,3-butylene glycol product, a ratio of the APHA after keeping at 100°C for 75 days to the APHA before keeping [(APHA after keeping at 100°C for 75 days)/(APHA before keeping)] is 10 or less (or 8 or less, 7 or less, 6 or less, 5 or less, 4 or less, or 3 or less).

[26] The 1,3-butylene glycol product according to any one of [1] to [25], in which, in a state where the 1,3-butylene glycol product has not undergone a test in which 1,3-butylene glycol product is kept at 180°C for 3 hours in air atmosphere, an initial boiling point is higher than 203°C (or 204°C or higher, 205°C or higher, 206°C or higher, 207°C or higher, or 208°C or higher), and/or a dry point of 209°C or lower.

[27] The 1,3-butylene glycol product according to any one of [1] to [26], in which, in a state where the 1,3-butylene glycol product has not undergone a test in which 1,3-butylene glycol product is kept at 180°C for 3 hours in air atmosphere, a potassium permanganate test value is 30 minutes or longer (or longer than 30 minutes, 32 minutes or longer, 35 minutes or longer, 40 minutes or longer, 50 minutes or longer, or 60 minutes or longer).

[28] The 1,3-butylene glycol product according to any one of [1] to [27], in which a content of 1,3-butylene glycol (GC area ratio under the following GC analysis conditions) is 98.6% or greater (or 98.7% or greater, 98.8% or greater, 98.9% or greater, 99.0% or greater, 99.1% or greater, 99.2% or greater, 99.3% or greater, 99.4% or greater, 99.5% or greater, 99.6% or greater, 99.7% or greater, or 99.8% or greater), in which the conditions for the gas chromatographic analysis (GC) are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample introduction Temperature: 250°C
Carrier gas: Helium
Column Gas Flow Rate: 1 mL/min
Detector and Detection Temperature: a flame ionization detector (FID), 280°C.

[29] The 1,3-butylene glycol product according to any one of [1] to [28], in which a total area ratio of peaks shorter

in retention time than the peak of 1,3-butylene glycol is 0.28% or less (or 0.25% or less, 0.23% or less, 0.2% or less, 0.17% or less, 0.15% or less, 0.12% or less, 0.1% or less, 0.07% or less, 0.04% or less, 0.03% or less, 0.02% or less, 0.01% or less, 0.007% or less, 0.005% or less, or 0.002% or less).
[30] The 1,3-butylene glycol product according to any one of [1] to [29], in which, in the GC analysis, a total area ratio of peaks longer in retention time than the peak of 1,3-butylene glycol is 1% or less (or 0.9% or less, 0.8% or less, 0.7% or less, 0.6% or less, 0.5% or less, 0.4% or less, 0.3% or less, 0.2% or less, or 0.1% or less).
[31] The 1,3-butylene glycol product according to any one of [1] to [30], in which a content of water is less than 0.4 wt.% (or 0.3 wt.% or less, 0.2 wt.% or less, 0.1 wt.% or less, 0.07 wt.% or less, 0.05 wt.% or less, 0.03 wt.% or less, 0.02 wt.% or less, 0.01 wt.% or less, or 0.005 wt.% or less).
[32] A moisturizer containing the 1,3-butylene glycol product described in any one of [1] to [31].
[33] The moisturizer according to [32], in which a content of the 1,3-butylene glycol product described in any one of [1] to [31] is 10 wt.% or greater (or 30 wt.% or greater, 50 wt.% or greater, 80 wt.% or greater, or 90 wt.% or greater).
[34] A cosmetic product containing the moisturizer described in [32] or [33].
[35] The cosmetic product according to [34], in which a content of the 1,3-butylene glycol product described in any one of [1] to [31] is from 0.01 to 40 wt.% (or from 0.1 to 30 wt.%, from 0.2 to 20 wt.%, from 0.5 to 15 wt.%, or from 1 to 10 wt.%).
[36] The cosmetic product according to [34] or [35], which is a skin cosmetic product, a hair cosmetic, a sunscreen cosmetic product or a make-up cosmetic product.
[37] A method for producing 1,3-butylene glycol to yield a 1,3-butylene glycol product described in any one of [1] to [31] from a reaction crude liquid containing 1,3-butylene glycol,

the method including: performing dehydration including removing water by distillation, removing a high boiling point component including removing a high boiling point component by distillation, and performing product distillation to yield purified 1,3-butylene glycol (1,3-butylene glycol product),
wherein, in a product column for use in the performing product distillation, a 1,3-butylene glycol charged liquid is subjected to distillation to yield the 1,3-butylene glycol product, the 1,3-butylene glycol charged liquid having a content of acetaldehyde of 500 ppm or less (or 205 ppm or less, 200 ppm or less, 150 ppm or less, 120 ppm or less, 100 ppm or less, 90 ppm or less, 80 ppm or less, 70 ppm or less, 60 ppm or less, 50 ppm or less, 40 ppm or less, 30 ppm or less, 20 ppm or less, 10 ppm or less, 5 ppm or less, or less than 2 ppm), a content of crotonaldehyde of 200 ppm or less (or 150 ppm or less, 130 ppm or less, 110 ppm or less, 100 ppm or less, 80 ppm or less, 70 ppm or less, 60 ppm or less, 50 ppm or less, 40 ppm or less, 30 ppm or less, 20 ppm or less, 10 ppm or less, 5 ppm or less, 3 ppm or less, 2 ppm or less, or less than 1 ppm), a content of water of 0.7 wt% or less (or 0.6 wt.% or less, 0.5 wt.% or less, 0.4 wt.% or less, 0.3 wt.% or less, 0.2 wt.% or less, or 0.1 wt.% or less), and a concentration of 1,3-butylene glycol determined by gas chromatographic analysis under the following conditions of 97.6 area % or greater (or 97.8 area % or greater, 98 area % or greater, 98.2 area % or greater, 98.4 area % or greater, 98.6 area % or greater, 98.8 area % or greater, 99 area % or greater, 99.1 area % or greater, 99.2 area % or greater, 99.3 area % or greater, 99.4 area % or greater, 99.5 area % or greater, 99.6 area % or greater, 99.7 area % or greater, 99.8 area % or greater, or 99.9 area % or greater), and the distillation is performed under a condition that a reflux ratio is 0.3 or greater (or 0.4 or greater, 0.5 or greater, 1 or greater, 2 or greater, 3 or greater, 4 or greater, 5 or greater, 6 or greater, 7 or greater, 8 or greater, 9 or greater, 10 or greater, 20 or greater, 50 or greater, 400 or greater, or 500 or greater).
The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample introduction Temperature: 250°C
Carrier gas: Helium
Column Gas Flow Rate: 1 mL/min
Detector and Detection Temperature: a flame ionization detector (FID), 280°C.

[38] A method for producing 1,3-butylene glycol to yield a 1,3-butylene glycol product described in any one of [1] to [31] from a reaction crude liquid containing 1,3-butylene glycol, the method including:

performing dehydration including removing water by distillation and removing a high boiling point component including removing a high boiling point component by distillation,
wherein, in a high boiling point component removal column for use in the removing a high boiling point component,

a charged liquid is subjected to distillation to yield the 1,3-butylene glycol product containing 1,3-butylene glycol, the charged liquid having a content of acetaldehyde of 500 ppm or less (or 205 ppm or less, 200 ppm or less, 100 ppm or less, 90 ppm or less, 80 ppm or less, 70 ppm or less, 60 ppm or less, 50 ppm or less, 40 ppm or less, 30 ppm or less, 20 ppm or less, 10 ppm or less, 5 ppm or less, less than 2 ppm, or less than 1 ppm), a content of crotonaldehyde of 200 ppm or less (or 110 ppm or less, 100 ppm or less, 80 ppm or less, 70 ppm or less, 60 ppm or less, 50 ppm or less, 40 ppm or less, 30 ppm or less, 20 ppm or less, 10 ppm or less, 5 ppm or less, 3 ppm or less, 2 ppm or less, or less than 1 ppm), a content of water of 3 wt.% or less (or 2 wt% or less, 1.2 wt% or less, 0.4 wt% or less, 0.3 wt% or less, 0.2 wt% or less, 0.1 wt% or less, 0.05 wt% or less, or 0.03 wt% or less), and a concentration of 1,3-butylene glycol determined by gas chromatographic analysis under the following conditions of 96.7 area % or greater (or 97% or greater, 98% or greater, or 99% or greater), and the distillation is performed under a condition that a reflux ratio is 0.03 or greater (or 0.1 or greater, 0.2 or greater, 0.3 or greater, 0.4 or greater, 0.5 or greater, 0.6 or greater, 0.7 or greater, 0.8 or greater, 0.9 or greater, 1 or greater, 1.2 or greater, 1.5 or greater, 2 or greater, 3 or greater, 4 or greater, 5 or greater, 10 or greater, or 20 or greater).

The conditions for the gas chromatographic analysis are as follows:

Analytical Column: a column with dimethylpolysiloxane as a stationary phase, having a length of 30 m, an inner diameter of 0.25 mm, and a film thickness of 1.0 μm
Heating conditions: heating from 80°C to 120°C at 5°C/min, then heating again to 160°C at 2°C/min and maintaining for 2 minutes, and further heating to 230°C at 10°C/min and maintaining at 230°C for 18 minutes
Sample Introduction Temperature: 250°C
Carrier Gas: helium
Column Gas Flow Rate: 1 mL/min
Detector and Detection Temperature: a flame ionization detector (FID), 280°C.

Industrial Applicability

The 1,3-butylene glycol product according to the present disclosure has high purity and is colorless and odorless (or almost colorless and odorless), unlikely to cause or increase coloration and odor over time, and/or unlikely to cause an acid concentration increase over time even in a state containing water. This 1,3-butylene glycol product can be used as a raw material for a moisturizer and a cosmetic product that have excellent moisturizing performance and can retain high quality for a long period of time.

Reference Signs List

A: Dehydration column
B: Desalting column
C: High boiling point component removal column
D: Alkaline reactor
E: Dealkalization column
F: Product distillation column (product column)
A-1, B-1, C-1, E-1, F-1: Condenser
A-2, C-2, F-2: Reboiler
X-1: Crude 1,3-butylene glycol
X-2: Water (discharged water)
X-3: Salt, high boiling point substance, and portion of 1,3-butylene glycol
X-4: High boiling point substance and portion of 1,3-butylene glycol
X-5: Sodium hydroxide, high boiling point substance, and portion of 1,3-butylene glycol
X-6: Low boiling point substance and portion of 1,3-butylene glycol
Y: 1,3-Butylene glycol product

## Claims

1. A 1,3-butylene glycol product comprising 1,3-butylene glycol, wherein, after the 1,3-butylene glycol product has been kept at 180°C for 3 hours in air atmosphere, at least one of contents of compounds represented by Formula

(A) or (B) is less than 8 ppm:

[Chemical Formula 1]

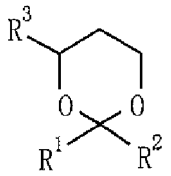

(A)

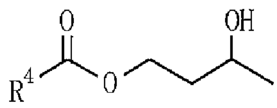

(B)

where $R^1$ to $R^4$ are the same as or different from each other, and each of $R^1$ to $R^4$ is a hydrogen atom, an alkyl group which has from 1 to 4 carbon atoms and may be substituted with a hydroxy group, or an alkenyl group which has from 2 to 4 carbon atoms and may be substituted with a hydroxy group.

**2.** The 1,3-butylene glycol product according to claim 1, wherein, after the 1,3-butylene glycol product has been kept at 180°C for 3 hours in air atmosphere, a sum of the contents of the compounds represented by Formula (A) is less than 66 ppm.

**3.** The 1,3-butylene glycol product according to claim 1 or 2, wherein, after the 1,3-butylene glycol product has been kept at 180°C for 3 hours in air atmosphere, a sum of the contents of the compounds represented by Formula (B) is less than 31 ppm.

**4.** The 1,3-butylene glycol product according to any one of claims 1 to 3, wherein the compound represented by Formula (A) is any of the compounds represented by the Formulas (1) to (7), and the compound represented by Formula (B) is any of the compounds represented by the Formulas (8) to (10):

[Chemical Formula 2]

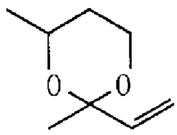

(1)

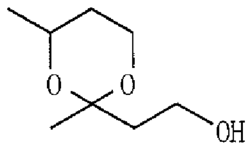

(2)

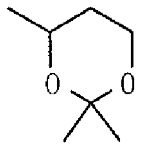

(3)

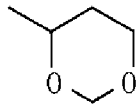

(4)

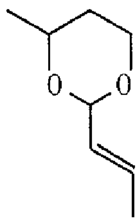

(5)

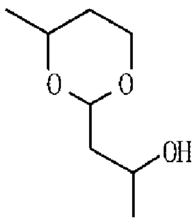

(6)

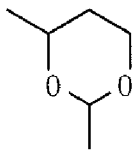

(7)

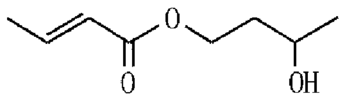

(8)

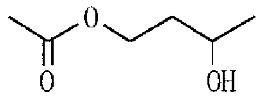

(9)

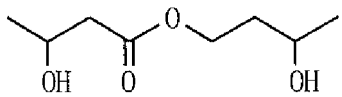

(10)

5. The 1,3-butylene glycol product according to claim 4, wherein, after the 1,3-butylene glycol product has been kept at 180°C for 3 hours in air atmosphere, a total content of the compounds represented by Formulas (1) to (4) is less than 34 ppm.

6. The 1,3-butylene glycol product according to claim 4 or 5, wherein, after the 1,3-butylene glycol product has been kept at 180°C for 3 hours in air atmosphere, a total content of the compounds represented by Formulas (5) to (10) is less than 63 ppm.

7. The 1,3-butylene glycol product according to any one of claims 4 to 6, wherein, after the 1,3-butylene glycol product has been kept at 180°C for 3 hours in air atmosphere, a total content of the compounds represented by Formulas (1) to (10) is less than 97 ppm.

8. The 1,3-butylene glycol product according to any one of claims 1 to 7, wherein, after the 1,3-butylene glycol product has been kept at 180°C for 3 hours in air atmosphere, a content of methyl vinyl ketone is less than 8 ppm, and/or a content of 1-hydroxy-3-butanone is less than 1 ppm, and/or a content of acetone is less than 6 ppm, and/or a content of formaldehyde is less than 2 ppm, and/or a content of crotonaldehyde is less than 1 ppm, and/or a content of acetaldol is less than 1 ppm, and/or a content of acetaldehyde is less than 5 ppm, and/or a content of butylaldehyde is less than 4 ppm.

9. The 1,3-butylene glycol product according to any one of claims 1 to 8, wherein, after the 1,3-butylene glycol product has been kept at 180°C for 3 hours in air atmosphere, a total content of methyl vinyl ketone, 1-hydroxy-3-butanone, acetone, formaldehyde, crotonaldehyde, acetaldol and acetaldehyde is less than 24 ppm.

10. The 1,3-butylene glycol product according to any one of claims 1 to 9, wherein, after the 1,3-butylene glycol product has been kept at 180°C for 3 hours in air atmosphere, a total content of methyl vinyl ketone, acetone, and butylaldehyde is less than 18 ppm.

**11.** The 1,3-butylene glycol product according to any one of claims 1 to 10, wherein, after the 1,3-butylene glycol product has been kept at 180°C for 3 hours in air atmosphere, an APHA is less than 79.

**12.** The 1,3-butylene glycol product according to any one of claims 1 to 11, wherein, after the 1,3-butylene glycol product has been kept at 180°C for 3 hours in air atmosphere, an initial boiling point is 203°C or higher and/or a dry point is 209°C or lower.

**13.** The 1,3-butylene glycol product according to any one of claims 1 to 12, wherein, after the 1,3-butylene glycol product has been kept at 180°C for 3 hours in air atmosphere, a potassium permanganate test value is 30 minutes or longer.

**14.** The 1,3-butylene glycol product according to any one of claims 1 to 13, wherein, after the 1,3-butylene glycol product has been kept at 180°C for 3 hours in air atmosphere, an acid concentration (in terms of acetic acid) is less than 16 ppm.

**15.** The 1,3-butylene glycol product according to any one of claims 1 to 14, wherein, in a state where the 1,3-butylene glycol product has not undergone a test in which 1,3-butylene glycol product is kept at 180°C for 3 hours in air atmosphere, a total content of 1-hydroxy-3-butanone and acetaldol is less than 19 ppm.

**16.** The 1,3-butylene glycol product according to any one of claims 1 to 15, wherein, in a state where the 1,3-butylene glycol product has not undergone a test in which 1,3-butylene glycol product is kept at 180°C for 3 hours in air atmosphere, a content of methyl vinyl ketone is less than 10 ppm, and/or a content of 1-hydroxy-3-butanone is less than 9 ppm, and/or a content of acetone is less than 9 ppm, and/or a content of formaldehyde is less than 3 ppm, and/or a content of crotonaldehyde is less than 2 ppm, and/or a content of acetaldol is less than 10 ppm, and/or a content of acetaldehyde is less than 4 ppm, and/or a content of butylaldehyde is less than 8 ppm.

**17.** The 1,3-butylene glycol product according to any one of claims 1 to 16, wherein, in a state where the 1,3-butylene glycol product has not undergone a test in which 1,3-butylene glycol product is kept at 180°C for 3 hours in air atmosphere, a total content of methyl vinyl ketone, 1-hydroxy-3-butanone, acetone, formaldehyde, crotonaldehyde, acetaldol and acetaldehyde is less than 47 ppm.

**18.** A moisturizer comprising the 1,3-butylene glycol product described in any one of claims 1 to 17.

**19.** A cosmetic product comprising the moisturizer described in claim 18.

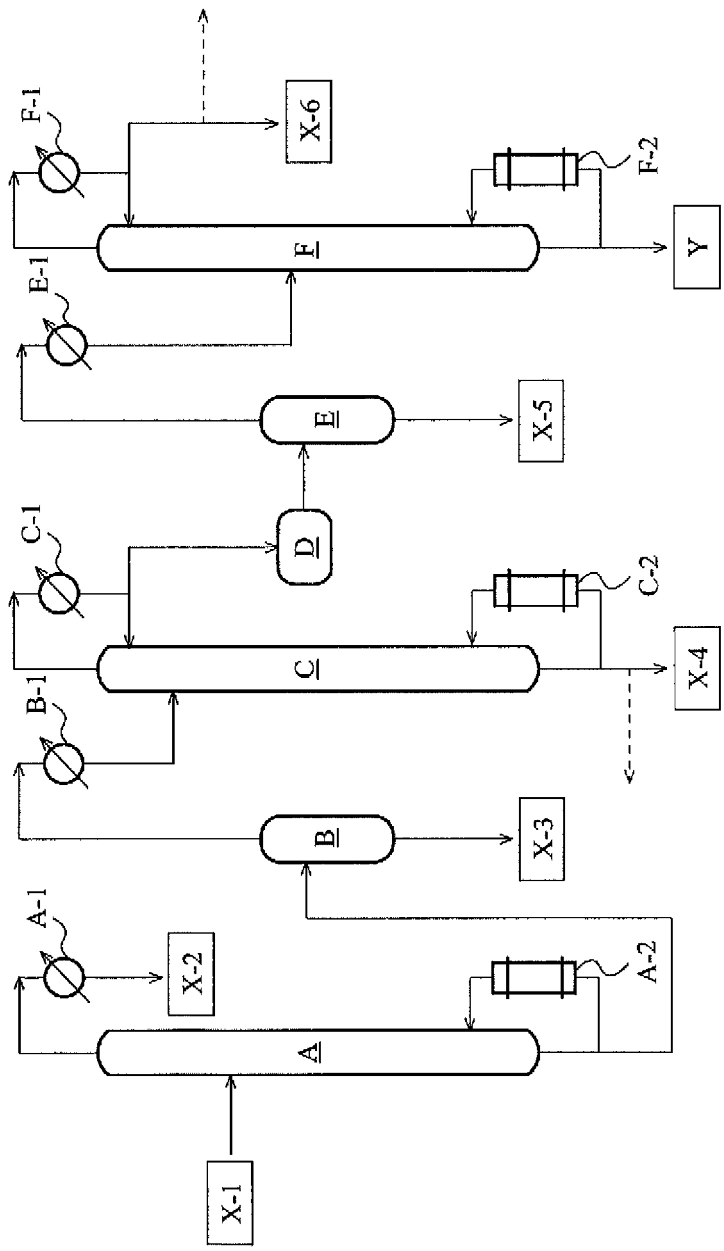


FIG. 1

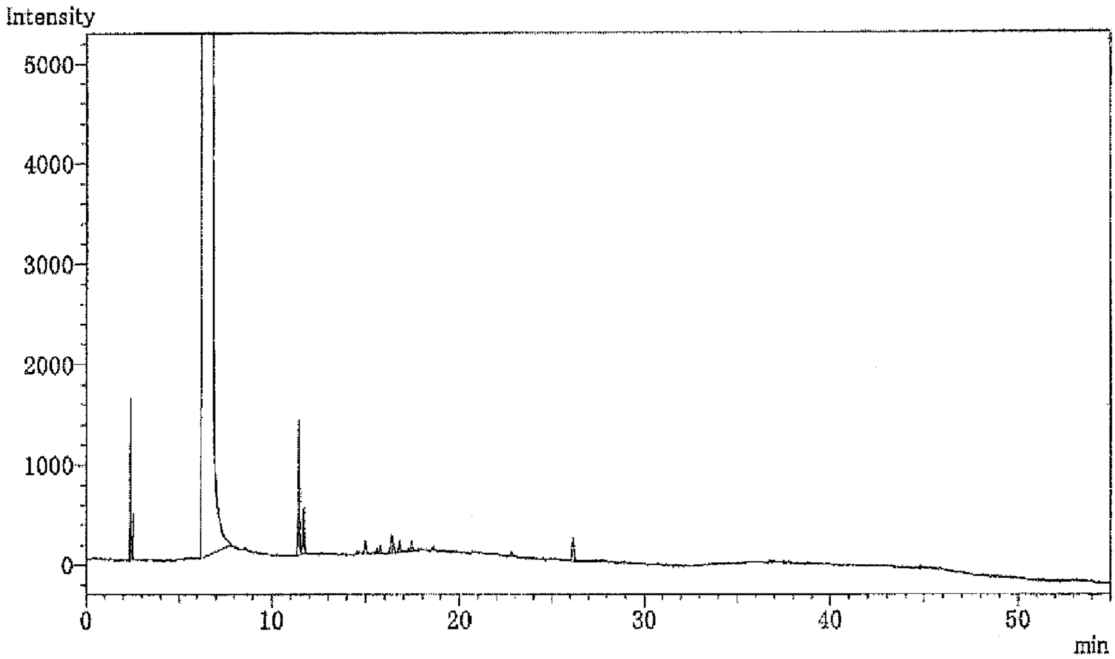


FIG. 2

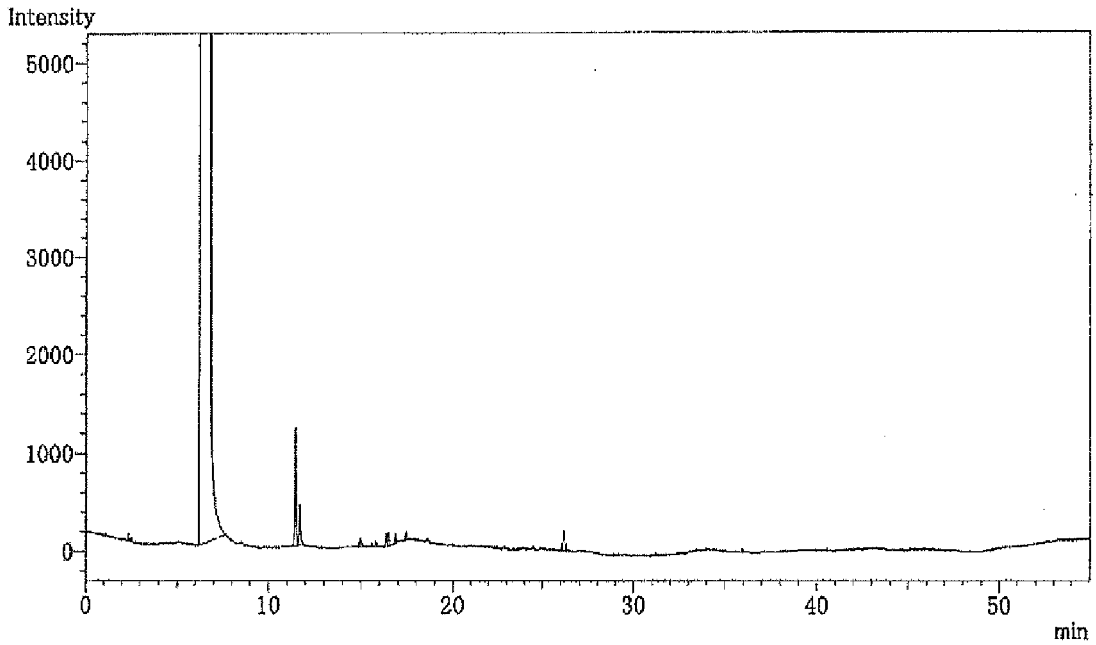


FIG. 3

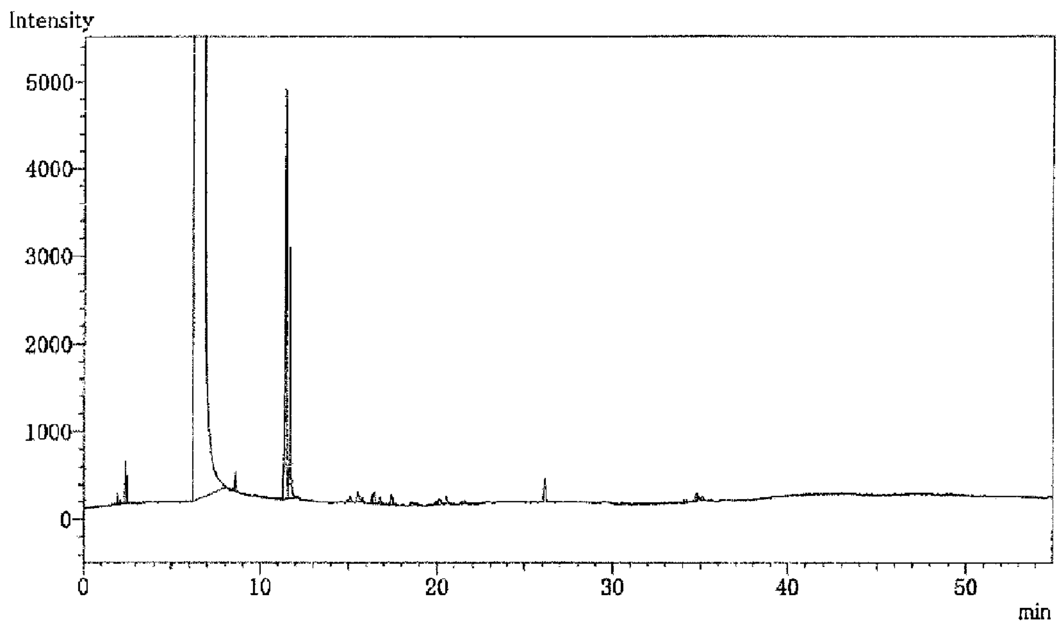


FIG. 4

## INTERNATIONAL SEARCH REPORT

International application No. PCT/JP2020/048232

A. CLASSIFICATION OF SUBJECT MATTER
C07C 31/20(2006.01)i; A61K 8/34(2006.01)i; A61Q 19/00(2006.01)i; C07C 29/80(2006.01)n
FI: C07C31/20 B; A61K8/34; A61Q19/00; C07C29/80
According to International Patent Classification (IPC) or to both national classification and IPC

B. FIELDS SEARCHED
Minimum documentation searched (classification system followed by classification symbols)
C07C29/00-31/44; A61K8/00-8/99; A61Q1/00-90/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 00/07969 A1 (DICEL CHEMICAL INDUSTRIES, LTD.) 17 February 2000 (2000-02-17) claims, page 1, line 11, page 4, lines 9-15, examples | 1-19 |
| X | WO 01/56963 A1 (DICEL CHEMICAL INDUSTRIES, LTD.) 09 August 2001 (2001-08-09) claims, page 1, line 25 to page 2, line 12, page 12, lines 12-18, examples | 1-19 |
| E, X | JP 6804601 B1 (DAICEL CORPORATION) 23 December 2020 (2020-12-23) claims, paragraphs [0002]-[0008], [0028], examples | 1-19 |
| E, X | JP 6804602 B1 (DAICEL CORPORATION) 23 December 2020 (2020-12-23) claims, paragraphs [0002]-[0008], [0026], examples | 1-19 |

☐ Further documents are listed in the continuation of Box C.　☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 March 2021 (12.03.2021) | 23 March 2021 (23.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/048232

| Patent Documents referred in the Report | Publication Date | Patent Family | Publication Date |
|---|---|---|---|
| WO 00/07969 A1 | 17 Feb. 2000 | US 6376725 B1<br>claims, column 1, lines 21-53, column 2, line 66 to column 3, line 9, examples<br>DE 69933987 T2<br>EP 1046628 A1<br>JP 4559625 B2<br>KR 10-0595399 B1 | |
| WO 01/56963 A1 | 09 Aug. 2001 | US 2003/0018224 A1<br>claims, paragraphs [0002]-[0005], [0065], examples<br>DE 10190479 T1<br>JP 2001-213822 A<br>JP 2001-213823 A<br>JP 2001-213824 A<br>JP 2001-213825 A<br>JP 2001-213828 A<br>JP 2001-288131 A<br>KR 10-2001-0102420 A | |
| JP 6804601 B1 | 23 Dec. 2020 | (Family: none) | |
| JP 6804602 B1 | 23 Dec. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019239974 A
- JP 2019239975 A
- JP 2019239976 A
- JP 2019239977 A
- JP 2019239978 A
- JP 2019239979 A
- JP 2020006660 A
- JP 2020018910 A
- JP 7258129 A
- WO 0007969 A
- JP 2001213822 A
- JP 2001213824 A
- JP 2001213825 A
- JP 2001213828 A